# EUROPEAN PATENT APPLICATION

(11) **EP 4 520 759 A1**
(43) Date of publication of application: **12.03.2025**
(21) Application number: 23799534.5
(22) Date of filing: 23.02.2023
(51) Int. Cl.: C07D 491/048, H10K 85/60, H10K 50/15, H10K 50/18, H10K 50/11

(54) **HETEROCYCLIC COMPOUND AND ORGANIC LIGHT-EMITTING ELEMENT COMPRISING SAME**

(30) Priority: 02.05.2022 KR 20220054355
(71) Applicant: LT Materials Co., Ltd., Yongin-si, Gyeonggi-do 17118 (KR)
(72) Inventor: LEE, Nam Jin, Yongin-si, Gyeonggi-do 17118 (KR); JEONG, Won Jang, Yongin-si, Gyeonggi-do 17118 (KR); KIM, Dong Jun, Yongin-si, Gyeonggi-do 17118 (KR)
(74) Representative: Goddar, Heinz J.
(86) International application number: PCT/KR2023/002592
(87) International publication number: WO 2023/214651

(57) **Abstract**

The present disclosure relates to a heterocyclic compound represented by Chemical Formula 1 and an organic light emitting device comprising the same.

## Description

### [Technical Field]

This application claims priority to and the benefits of Korean Patent Application No. 10-2022-0054355, filed with the Korean Intellectual Property Office on May 2, 2022, the entire contents of which are incorporated herein by reference.

The present disclosure relates to a heterocyclic compound and an organic light emitting device comprising the same.

### [Background Art]

An organic light emitting device is one type of self-emissive display devices, and has advantages of having a wide viewing angle and a high response speed as well as having an excellent contrast.

The organic light emitting device has a structure of disposing an organic thin film between two electrodes. When a voltage is applied to the organic light emitting device having such a structure, electrons and holes injected from the two electrodes bind and pair in the organic thin film, and then light emits as these annihilate. The organic thin film may be formed in a single layer or a multilayer as necessary.

A material of the organic thin film may have a light emitting function as necessary. For example, as a material of the organic thin film, compounds each capable of forming a light emitting layer themselves alone may be used, or compounds each capable of performing a role of a host or a dopant of a host-dopant-based light emitting layer may also be used. In addition thereto, compounds capable of performing roles of hole injection, hole transport, electron blocking, hole blocking, electron transport, electron injection and the like may also be used as a material of the organic thin film.

Development of an organic thin film material has been continuously required for enhancing performance, lifetime or efficiency of an organic light emitting device.

### Prior Art Documents

### Patent Documents

US Patent No. 4,356,429

### [Disclosure]

### [Technical Problem]

An object of the present disclosure is to provide a heterocyclic compound and an organic light emitting device comprising the same.

### [Technical Solution]

In order to the above object, one embodiment of the present disclosure provides a heterocyclic compound represented by the following Chemical Formula 1. 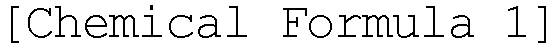 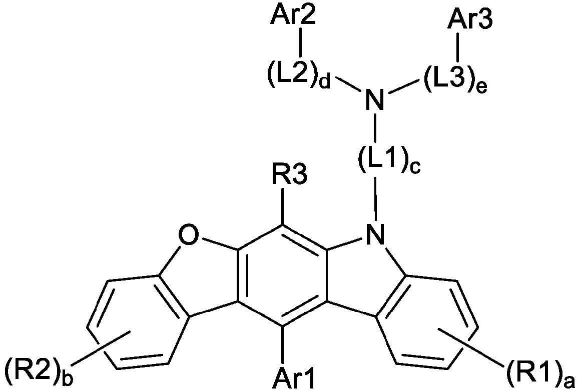 In Chemical Formula 1,
R1 to R3 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; -SiR101R102R103; and -NR101R102, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group,
a is an integer of 0 to **4,** and when a is 2 or greater, R1s are the same as or different from each other,
b is an integer of 0 to **4,** and when b is 2 or greater, R2s are the same as or different from each other,
Ar1 to Ar3 are the same as or different from each other, and each independently a substituted or unsubstituted C6 to C60 aryl group; or a substituted or unsubstituted C2 to C60 heteroaryl group,
L1 is a substituted or unsubstituted C6 to C60 arylene group; or a substituted or unsubstituted C2 to C60 heteroarylene group,
c is an integer of 1 to 5, and when c is 2 or greater, L1s are the same as or different from each other,
L2 and L3 are the same as or different from each other, and each independently a direct bond; a substituted or unsubstituted C6 to C60 arylene group; or a substituted or unsubstituted C2 to C60 heteroarylene group,
d is an integer of 0 to 5, and when d is 2 or greater, L2s are the same as or different from each other, and
e is an integer of 0 to 5, and when e is 2 or greater, L3s are the same as or different from each other.

In addition, the present disclosure provides an organic light emitting device comprising:
a first electrode;
a second electrode provided opposite to the first electrode; and
one or more organic material layers provided between the first electrode and the second electrode,
wherein at least one of the one or more organic material layers comprise the heterocyclic compound represented by Chemical Formula 1.

In addition, the present disclosure provides an organic light emitting device, wherein the organic material layer comprises a hole transport layer, and the hole transport layer comprises the heterocyclic compound.

In addition, the present disclosure provides an organic light emitting device, wherein the organic material layer comprises an electron blocking layer, and the electron blocking layer comprises the heterocyclic compound.

### [Advantageous Effects]

A compound described in the present specification can be used as an organic material layer material of an organic light emitting device. The compound is capable of performing roles of a hole injection layer material, an electron blocking layer material, a hole transport layer material, a light emitting layer material, an electron transport layer material, a hole blocking layer material, an electron injection layer material and the like in an organic light emitting device. Particularly, the compound can be used as a hole transport layer material, an electron blocking layer material or a light emitting layer material of an organic light emitting device.

Specifically, the heterocyclic compound represented by Chemical Formula 1 has high hole mobility, and has a proper HOMO (highest occupied molecular orbital) level and a high LUMO (lowest unoccupied molecular orbital) level, thereby lowering a driving voltage, and enhancing light emission efficiency and lifetime properties.

### [Description of Drawings]

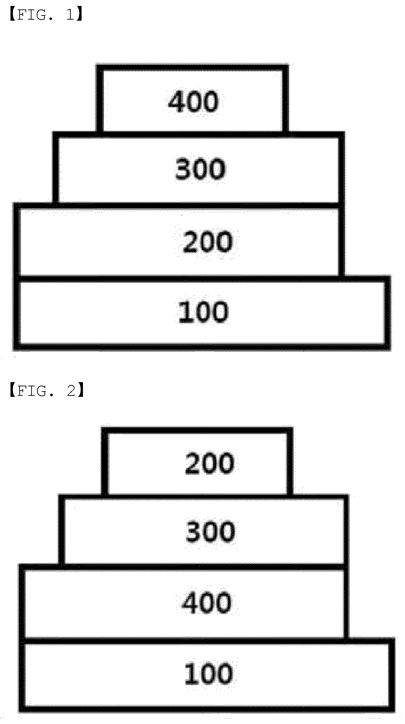

FIGS. 1 to 3 are diagrams each schematically illustrating a lamination structure of an organic light emitting device according to one embodiment of the present disclosure.

### [Mode for Disclosure]

Hereinafter, the present disclosure will be described in more detail.

In the present specification, a term "substitution" means that a hydrogen atom bonding to a carbon atom of a compound is changed to another substituent, and the position of substitution is not limited as long as it is a position at which the hydrogen atom is substituted, that is, a position at which a substituent is capable of substituting, and when two or more substituents substitute, the two or more substituents may be the same as or different from each other.

In the present specification, "substituted or unsubstituted" means being substituted with one or more substituents selected from the group consisting of deuterium; halogen; a cyano group; a C1 to C60 linear or branched alkyl group; a C2 to C60 linear or branched alkenyl group; a C2 to C60 linear or branched alkynyl group; a C3 to C60 monocyclic or polycyclic cycloalkyl group; a C2 to C60 monocyclic or polycyclic heterocycloalkyl group; a C6 to C60 monocyclic or polycyclic aryl group; a C2 to C60 monocyclic or polycyclic heteroaryl group; - SiRR'R"; -P(=O)RR'; a C1 to C20 alkylamine group; a C6 to C60 monocyclic or polycyclic arylamine group; and a C2 to C60 monocyclic or polycyclic heteroarylamine group or being unsubstituted, or being substituted with a substituent in which two or more substituents selected from among the substituents exemplified above are linked or being unsubstituted.

In the present specification, the halogen may be fluorine, chlorine, bromine or iodine.

In the present specification, the alkyl group includes a linear or branched form having 1 to 60 carbon atoms, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, , a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes a linear or branched form having 2 to 60 carbon atoms, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes a linear or branched form having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may include a methoxy group, an ethoxy group, an n-propoxy group, an isopropoxy group, an n-butoxy group, an isobutoxy group, a tert-butoxy group, a sec-butoxy group, an n-pentyloxy group, a neopentyloxy group, an isopentyloxy group, an n-hexyloxy group, a 3,3-dimethylbutyloxy group, a 2-ethylbutyloxy group, an n-octyloxy group, an n-nonyloxy group, an n-decyloxy group, a benzyloxy group, a p-methylbenzyloxy group and the like, but are not limited thereto.

In the present specification, the cycloalkyl group includes a monocyclic or polycyclic group having 3 to 60 carbon atoms, and may be further substituted with other substituents. Herein, the polycyclic group means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon atoms of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes a monocyclic or polycyclic group having 2 to 60 carbon atoms, and may be further substituted with other substituents. Herein, the polycyclic group means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes a monocyclic or polycyclic group having 6 to 60 carbon atoms, and may be further substituted with other substituents. Herein, the polycyclic group means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group may include a spiro group. The number of carbon atoms of the aryl group may be from 6 to 60, specifically from 6 to 40 and more specifically from 6 to 25. Specific examples of the aryl group may include a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the phosphine oxide group is represented by -P(=O)R101R102, and R101 and R102 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specifically, the phosphine oxide group may be substituted with an aryl group, and as the aryl group, the examples described above may be applied. Examples of the phosphine oxide group may include a diphenylphosphine oxide group, a dinaphthylphosphine oxide group and the like, but are not limited thereto.

In the present specification, the silyl group is a substituent including Si and having the Si atom directly linked as a radical, and is represented by -SiR101R102R103. R101 to R103 are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the fluorenyl group may be substituted, and adjacent substituents may bond to each other to form a ring.

When the fluorenyl group is substituted, 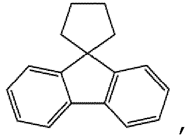 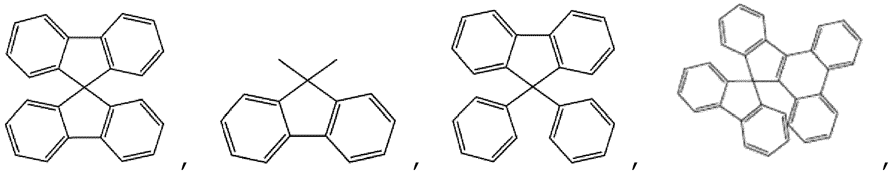 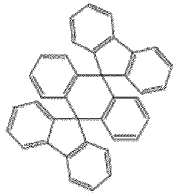 and the like may be included, however, the structure is not limited thereto.

In the present specification, the spiro group is a group including a spiro structure, and may have 15 to 60 carbon atoms. For example, the spiro group may include a structure in which a 2,3-dihydro-1H-indene group or a cyclohexane group spiro bonds to a fluorenyl group. Specifically, the spiro group may include any one of groups of the following structural formulae.

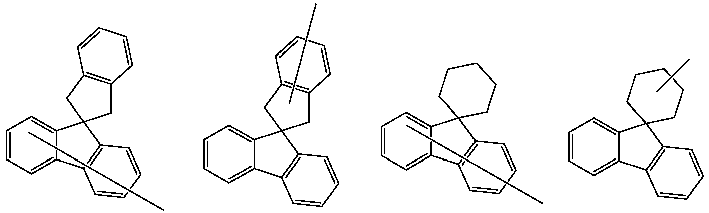

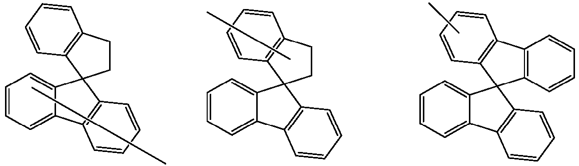

In the present specification, the heteroaryl group includes S, O, Se, N or Si as a heteroatom, includes a monocyclic or polycyclic group having 2 to 60 carbon atoms, and may be further substituted with other substituents. Herein, the polycyclic group means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may include a pyridyl group, a pyrrolyl group, a pyrimidyl group, a pyridazinyl group, a furanyl group, a thiophenyl group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a quinozolinyl group, a naphthyridyl group, an acridinyl group, a phenanthridinyl group, an imidazopyridinyl group, a diazanaphthalenyl group, a triazaindenyl group, a 2-indolyl group, an indolizinyl group, a benzothiazolyl group, a benzoxazolyl group, a benzimidazolyl group, a benzothiophenyl group, a benzofuranyl group, a dibenzothiophenyl group, a dibenzofuranyl group, a carbazolyl group, a benzocarbazolyl group, a dibenzocarbazolyl group, a phenazinyl group, a dibenzosilole group, a spirobi(dibenzosilole) group, a dihydrophenazinyl group, a phenoxazinyl group, a phenanthridyl group, a thienyl group, an indolo[2,3-a]carbazolyl group, an indolo[2,3-b]carbazolyl group, an indolinyl group, a 10,11-dihydro-dibenzo [b,f] azepinyl group, a 9,10-dihydroacridinyl group, a phenanthrazinyl group, a phenothiazinyl group, a phthalazinyl group, a naphthylidinyl group, a phenanthrolinyl group, a benzo[c] [1,2,5]thiadiazolyl group, a 5,10-dihydrodibenzo[b,e] [1,4]azasilinyl group, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and the number of carbon atoms is not particularly limited, but preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except for those that are each a divalent group. In addition, the heteroarylene group means the heteroaryl group having two bonding sites, that is, a divalent group. The descriptions on the heteroaryl group provided above may be applied thereto except for those that are each a divalent group.

In the present specification, an "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting at ortho positions in a benzene ring, and two substituents substituting at the same carbon in an aliphatic ring may be interpreted as groups "adjacent" to each other.

In the present disclosure, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present disclosure, a "case of a substituent being not indicated in a chemical formula or compound structure" may mean that positions that may come as a substituent are all hydrogen or deuterium. In other words, since deuterium is an isotope of hydrogen, some hydrogen atoms may be deuterium that is an isotope, and herein, a content of the deuterium may be from 0% to 100%.

In one embodiment of the present disclosure, in a "case of a substituent being not indicated in a chemical formula or compound structure", hydrogen and deuterium may be used interchangeably in compounds when deuterium is not explicitly excluded such as "a deuterium content being 0%", "a hydrogen content being 100%" or "substituents being all hydrogen".

In one embodiment of the present disclosure, deuterium is one of isotopes of hydrogen, is an element having deuteron formed with one proton and one neutron as a nucleus, and may be expressed as hydrogen-2, and the elemental symbol thereof may also be written as D or ²H.

In one embodiment of the present disclosure, an isotope means an atom with the same atomic number (Z) but with a different mass number (A), and may also be interpreted as an element with the same number of protons but with a different number of neutrons.

In one embodiment of the present disclosure, a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 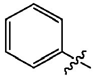 may mean that the total number of substituents that the phenyl group may have is 5 (T1 in the formula), and the number of deuterium among these is 1 (T2 in the formula). In other words, having a deuterium content of 20% in a phenyl group may be represented by the following structural formulae.

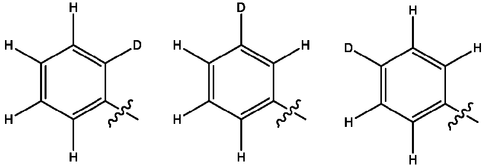

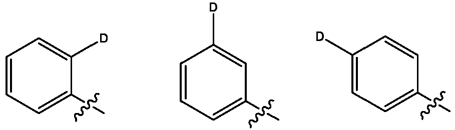

In addition, in one embodiment of the present disclosure, "a phenyl group having a deuterium content of 0%" may mean a phenyl group that does not include a deuterium atom, that is, a phenyl group that has 5 hydrogen atoms.

In the present disclosure, the C6 to C60 aromatic hydrocarbon ring means a compound including an aromatic ring formed with C6 to C60 carbons and hydrogens. Examples thereof may include phenyl, biphenyl, terphenyl, triphenylene, naphthalene, anthracene, phenalene, phenanthrene, fluorene, pyrene, chrysene, perylene, azulene and the like, but are not limited thereto, and include all aromatic hydrocarbon ring compounds known in the art and satisfying the above-mentioned number of carbon atoms.

One embodiment of the present disclosure provides a heterocyclic compound represented by the following Chemical Formula 1. 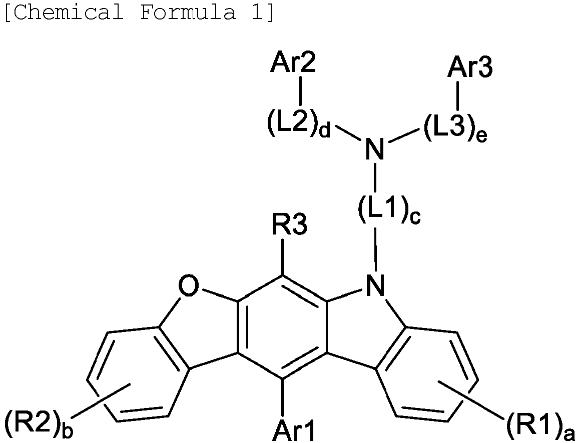 In Chemical Formula 1,
R1 to R3 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; -SiR101R102R103; and -NR101R102, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group,
a is an integer of 0 to **4,** and when a is 2 or greater, R1s are the same as or different from each other,
b is an integer of 0 to 4, and when b is 2 or greater, R2s are the same as or different from each other,
Ar1 to Ar3 are the same as or different from each other, and each independently a substituted or unsubstituted C6 to C60 aryl group; or a substituted or unsubstituted C2 to C60 heteroaryl group,
L1 is a substituted or unsubstituted C6 to C60 arylene group; or a substituted or unsubstituted C2 to C60 heteroarylene group,
c is an integer of 1 to 5, and when c is 2 or greater, L1s are the same as or different from each other,
L2 and L3 are the same as or different from each other, and each independently a direct bond; a substituted or unsubstituted C6 to C60 arylene group; or a substituted or unsubstituted C2 to C60 heteroarylene group,
d is an integer of 0 to 5, and when d is 2 or greater, L2s are the same as or different from each other, and
e is an integer of 0 to 5, and when e is 2 or greater, L3s are the same as or different from each other.

In one embodiment of the present disclosure, R1 to R3 are the same as or different from each other, and each independently hydrogen; deuterium; halogen; a cyano group; a substituted or unsubstituted C1 to C30 alkyl group; a substituted or unsubstituted C2 to C30 alkenyl group; a substituted or unsubstituted C2 to C30 alkynyl group; a substituted or unsubstituted C1 to C30 alkoxy group; a substituted or unsubstituted C3 to C30 cycloalkyl group; a substituted or unsubstituted C2 to C30 heterocycloalkyl group; a substituted or unsubstituted C6 to C30 aryl group; a substituted or unsubstituted C2 to C30 heteroaryl group; -P(=O)R101R102; -SiR101R102R103; or -NR101R102, or two or more groups adjacent to each other bond to each other to form a substituted or unsubstituted C6 to C30 aromatic hydrocarbon ring; or a substituted or unsubstituted C2 to C30 heteroring, and R101, R102 and R103 are the same as or different from each other and may be each independently a substituted or unsubstituted C1 to C30 alkyl group; a substituted or unsubstituted C6 to C30 aryl group; or a substituted or unsubstituted C2 to C30 heteroaryl group.

In another embodiment of the present disclosure, R1 to R3 are the same as or different from each other, and each independently hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; -P(=O)R101R102; -SiR101R102R103; or -NR101R102, or two or more groups adjacent to each other bond to each other to form a substituted or unsubstituted C6 to C20 aromatic hydrocarbon ring; or a substituted or unsubstituted C2 to C20 heteroring, and R101, R102 and R103 are the same as or different from each other and may be each independently a substituted or unsubstituted C1 to C20 alkyl group; a substituted or unsubstituted C6 to C20 aryl group; or a substituted or unsubstituted C2 to C20 heteroaryl group.

In another embodiment of the present disclosure, R1 to R3 are the same as or different from each other and may be each independently hydrogen; deuterium; halogen; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; -P(=O)R101R102; -SiR101R102R103; or -NR101R102, and R101, R102 and R103 are the same as or different from each other and may be each independently a substituted or unsubstituted C1 to C20 alkyl group; a substituted or unsubstituted C6 to C20 aryl group; or a substituted or unsubstituted C2 to C20 heteroaryl group.

In another embodiment of the present disclosure, R1 to R3 are the same as or different from each other, and may be each independently hydrogen; deuterium; halogen; a cyano group; a substituted or unsubstituted C1 to C20 alkyl group; a substituted or unsubstituted C6 to C20 aryl group; or a substituted or unsubstituted C2 to C20 heteroaryl group.

In another embodiment of the present disclosure, R1 to R3 are the same as or different from each other, and may be each independently hydrogen; deuterium; a substituted or unsubstituted C6 to C20 aryl group; or a substituted or unsubstituted C2 to C20 heteroaryl group.

In another embodiment of the present disclosure, R1 may be hydrogen; deuterium; a substituted or unsubstituted C6 to C30 aryl group; or a substituted or unsubstituted C2 to C30 heteroaryl group.

In another embodiment of the present disclosure, R1 may be hydrogen; deuterium; a substituted or unsubstituted phenyl group; a substituted or unsubstituted biphenyl group; a substituted or unsubstituted naphthyl group; a substituted or unsubstituted phenanthrenyl group; a substituted or unsubstituted triphenylenyl group; a substituted or unsubstituted dibenzofuranyl group; or a substituted or unsubstituted dibenzothiophenyl group.

In another embodiment of the present disclosure, R2 and R3 are the same as or different from each other, and may be each independently hydrogen; or deuterium.

In one embodiment of the present disclosure, Ar1 to Ar3 are the same as or different from each other, and may be each independently a substituted or unsubstituted C6 to C30 aryl group; or a substituted or unsubstituted C2 to C30 heteroaryl group.

In another embodiment of the present disclosure, Ar1 to Ar3 are the same as or different from each other, and may be each independently a substituted or unsubstituted C6 to C20 aryl group; or a substituted or unsubstituted C2 to C20 heteroaryl group.

In another embodiment of the present disclosure, Ar1 to Ar3 are the same as or different from each other, and may be each independently a substituted or unsubstituted phenyl group; a substituted or unsubstituted biphenyl group; a substituted or unsubstituted naphthyl group; a substituted or unsubstituted fluorenyl group; a substituted or unsubstituted phenanthrenyl group; a substituted or unsubstituted triphenylenyl group; a substituted or unsubstituted dibenzofuranyl group; or a substituted or unsubstituted dibenzothiophenyl group.

In one embodiment of the present disclosure, L1 may be a substituted or unsubstituted C6 to C30 arylene group; or a substituted or unsubstituted C2 to C30 heteroarylene group.

In another embodiment of the present disclosure, L1 may be a substituted or unsubstituted C6 to C20 arylene group; or a substituted or unsubstituted C2 to C20 heteroarylene group.

In another embodiment of the present disclosure, L1 may be a substituted or unsubstituted C6 to C20 arylene group.

In another embodiment of the present disclosure, L1 may be a substituted or unsubstituted phenylene group; or a substituted or unsubstituted biphenylene group.

In one embodiment of the present disclosure, L2 and L3 are the same as or different from each other, and may be each independently a direct bond; a substituted or unsubstituted C6 to C30 arylene group; or a substituted or unsubstituted C2 to C30 heteroarylene group.

In another embodiment of the present disclosure, L2 and L3 are the same as or different from each other, and may be each independently a direct bond; a substituted or unsubstituted C6 to C20 arylene group; or a substituted or unsubstituted C2 to C20 heteroarylene group.

In another embodiment of the present disclosure, L2 and L3 are the same as or different from each other, and may be each independently a direct bond; or a substituted or unsubstituted C6 to C20 arylene group.

In another embodiment of the present disclosure, L2 and L3 are the same as or different from each other, and may be each independently a direct bond; or a substituted or unsubstituted phenylene group.

In one embodiment of the present disclosure, the heterocyclic compound represented by Chemical Formula 1 may not include deuterium as a substituent, or may have a deuterium content of, for example, greater than 0%, 1% or greater, 10% or greater, 20% or greater, 30% or greater, 40% or greater or 50% or greater, and 100% or less, 90% or less, 80% or less, 70% or less or 60% or less with respect to the total number of hydrogen atoms and deuterium atoms.

In another embodiment of the present disclosure, the heterocyclic compound represented by Chemical Formula 1 may not include deuterium as a substituent, or may have a deuterium content of 1% to 100% with respect to the total number of hydrogen atoms and deuterium atoms.

In another embodiment of the present disclosure, the heterocyclic compound represented by Chemical Formula 1 may not include deuterium as a substituent, or may have a deuterium content of 20% to 90% with respect to the total number of hydrogen atoms and deuterium atoms.

In another embodiment of the present disclosure, the heterocyclic compound represented by Chemical Formula 1 may not include deuterium as a substituent, or may have a deuterium content of 30% to 80% with respect to the total number of hydrogen atoms and deuterium atoms.

In another embodiment of the present disclosure, the heterocyclic compound represented by Chemical Formula 1 may not include deuterium as a substituent, or may have a deuterium content of 50% to 70% with respect to the total number of hydrogen atoms and deuterium atoms.

In one embodiment of the present disclosure, the heterocyclic compound represented by Chemical Formula 1 may be represented by any one of the following compounds.

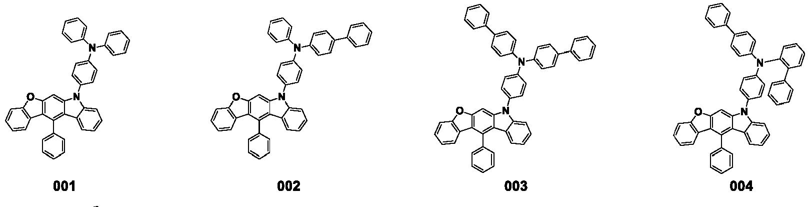

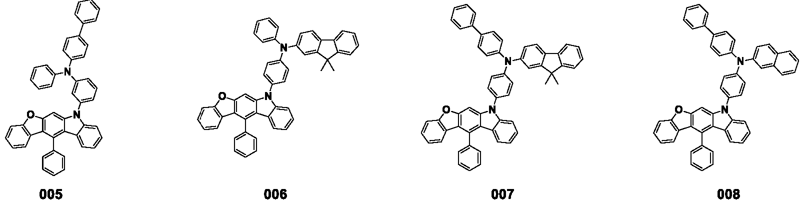

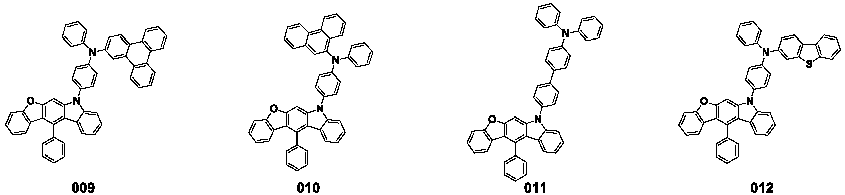

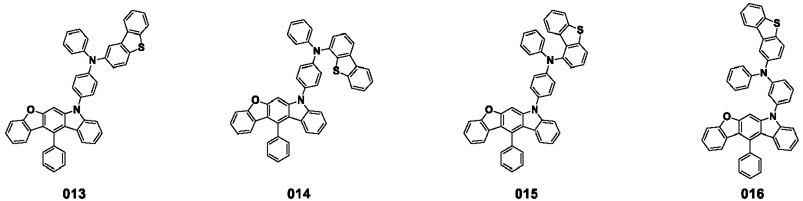

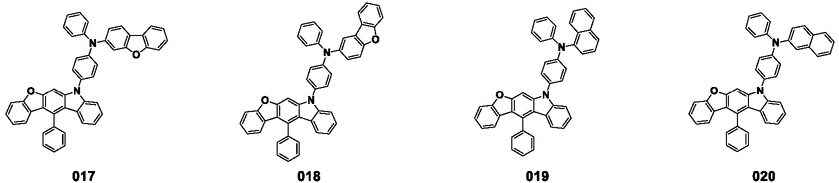

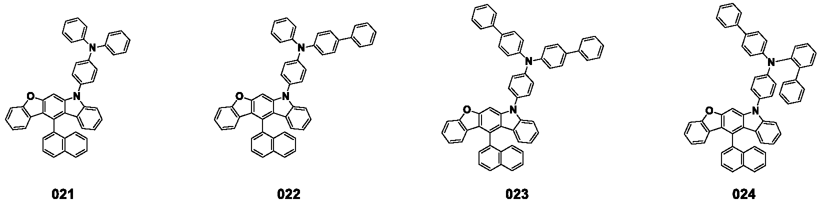

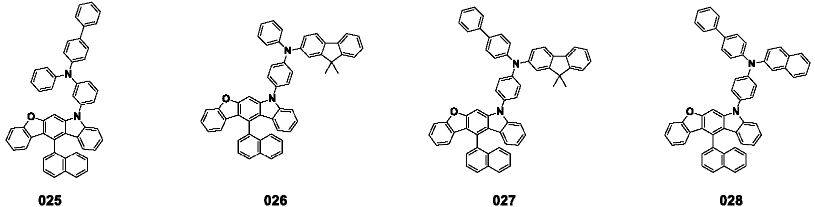

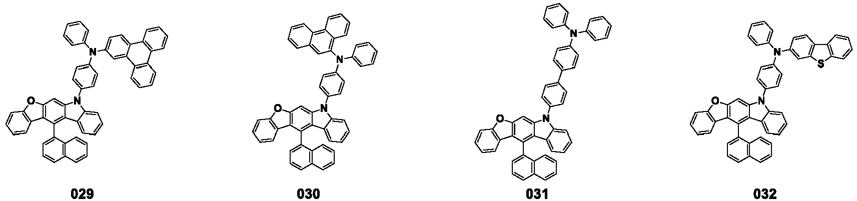

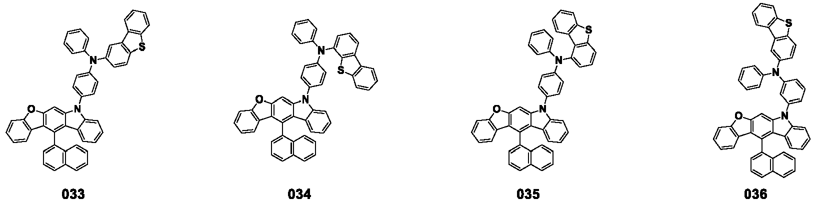

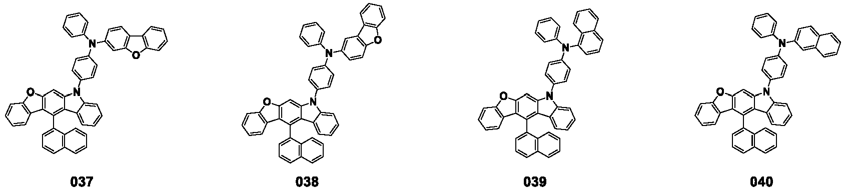

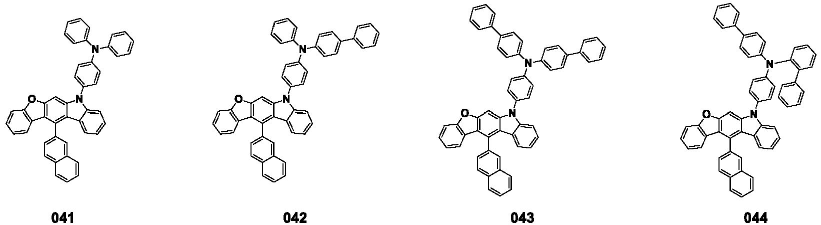

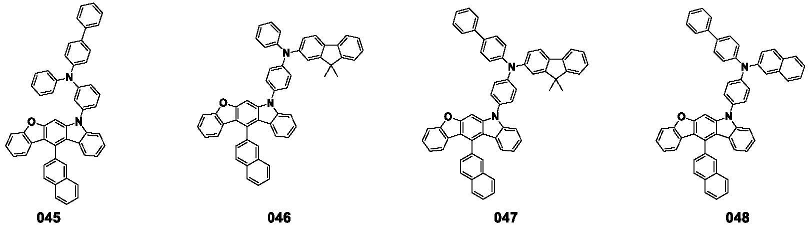

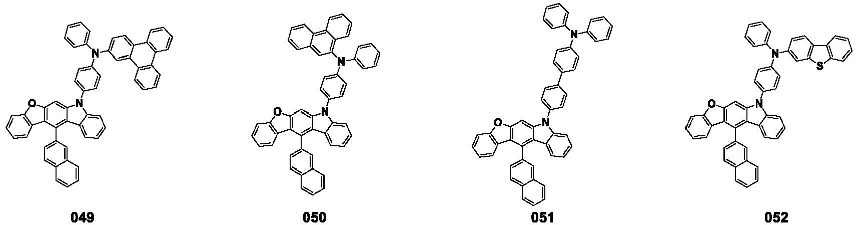

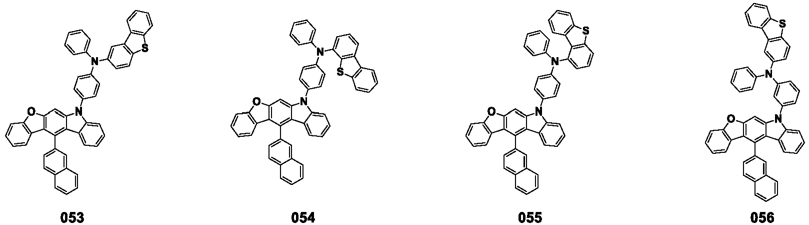

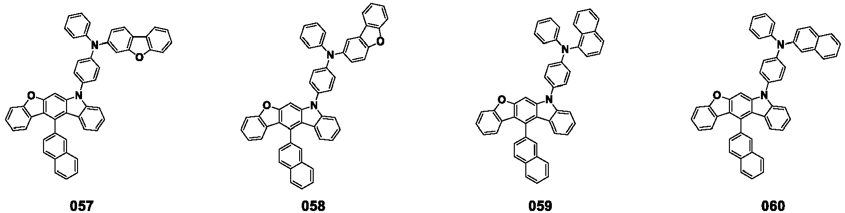

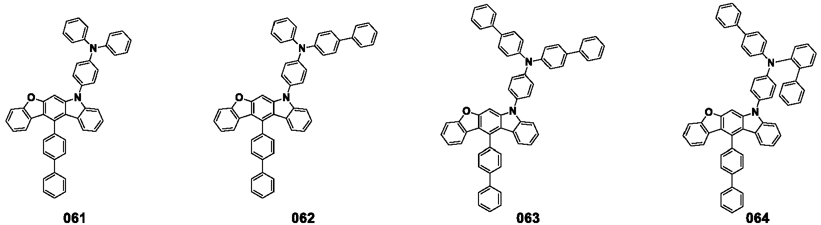

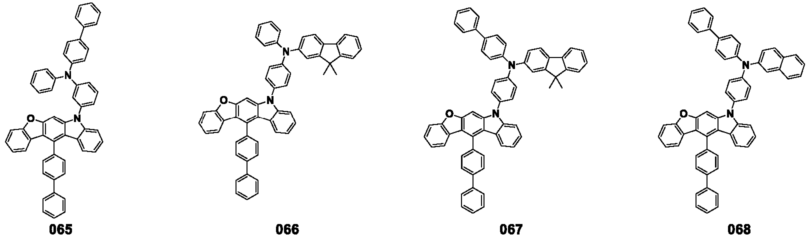

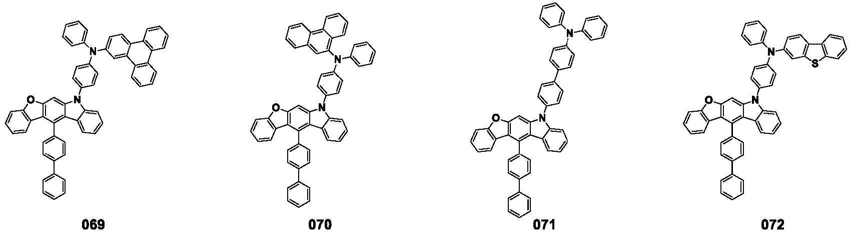

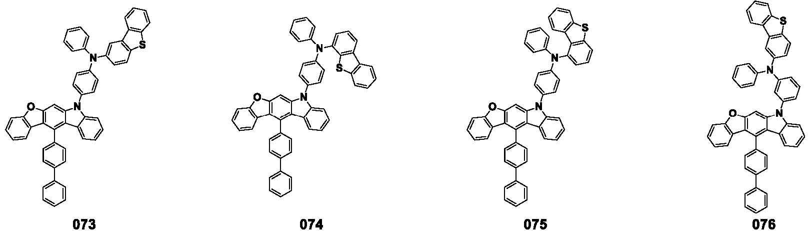

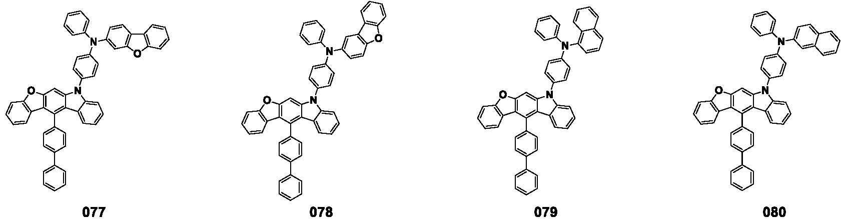

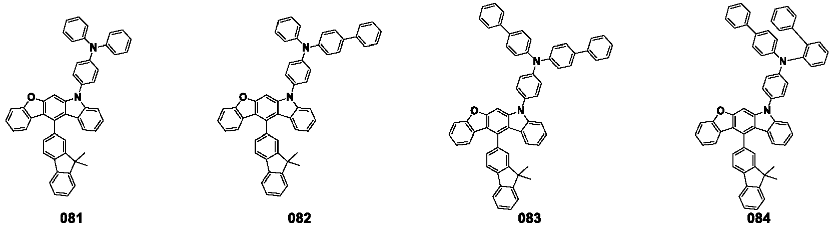

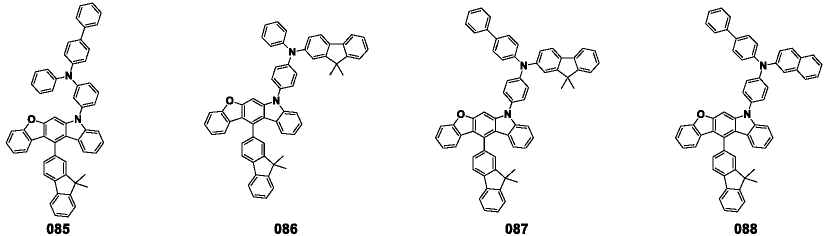

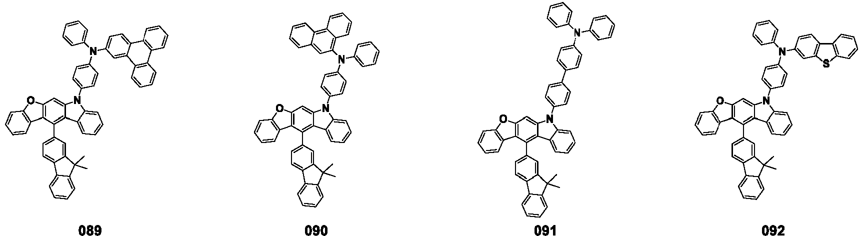

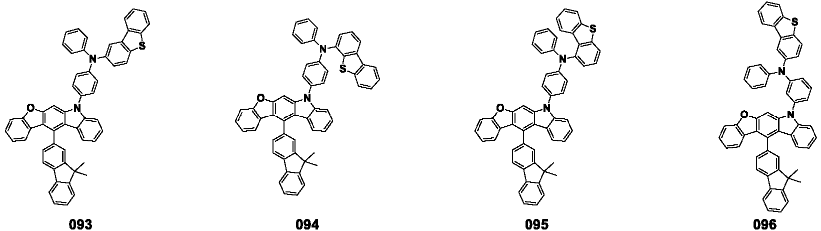

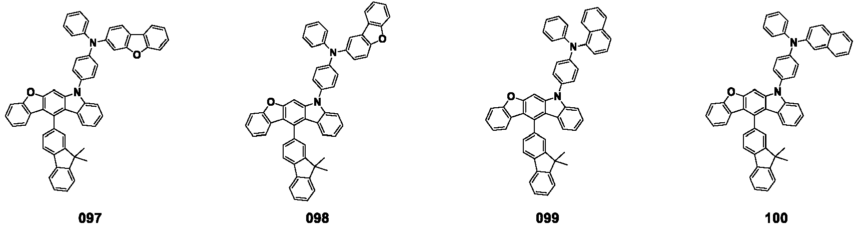

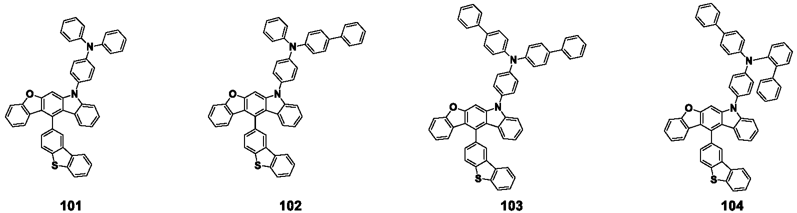

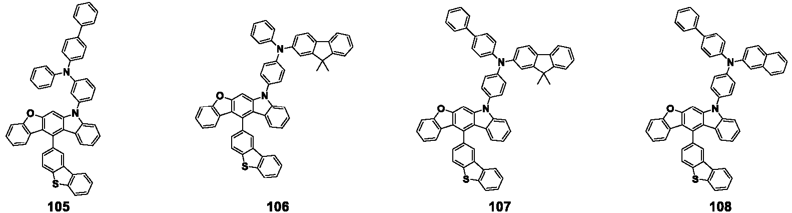

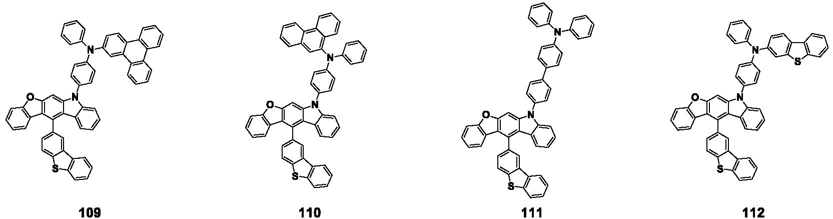

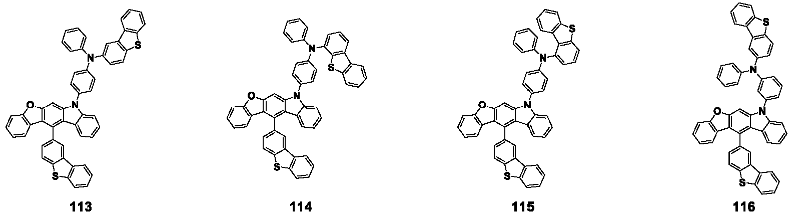

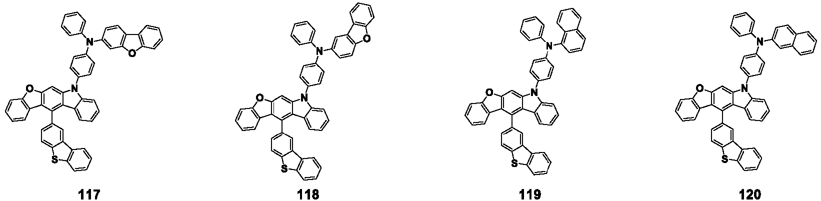

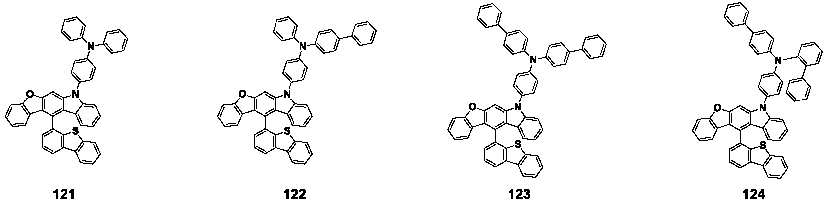

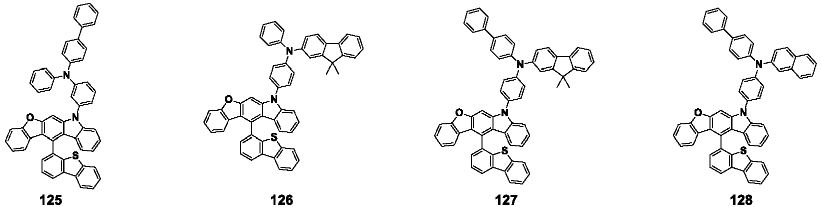

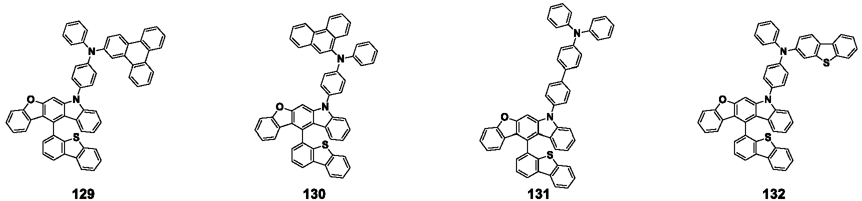

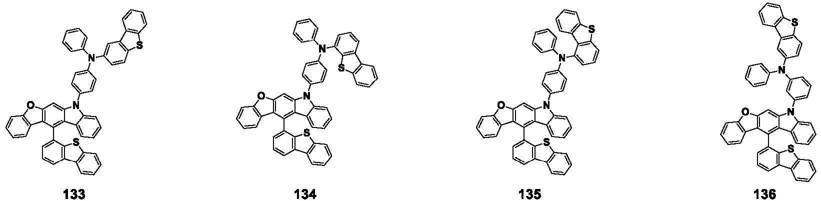

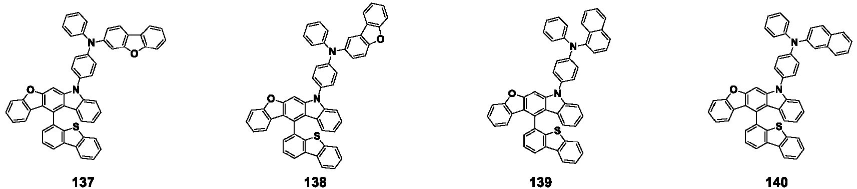

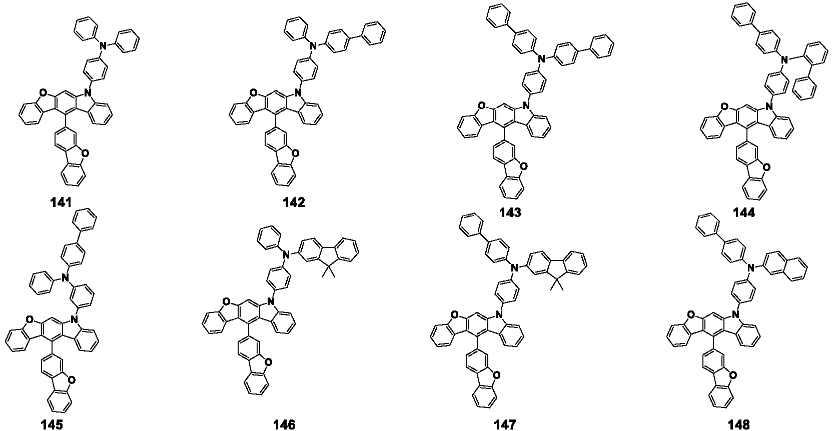

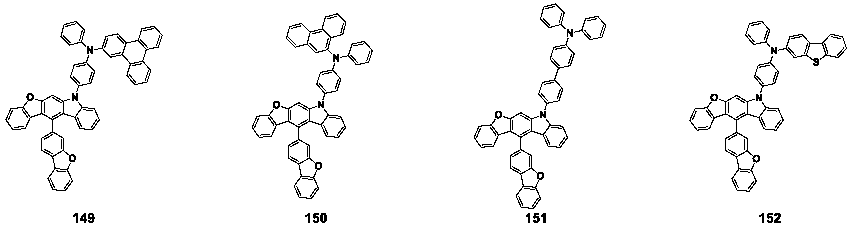

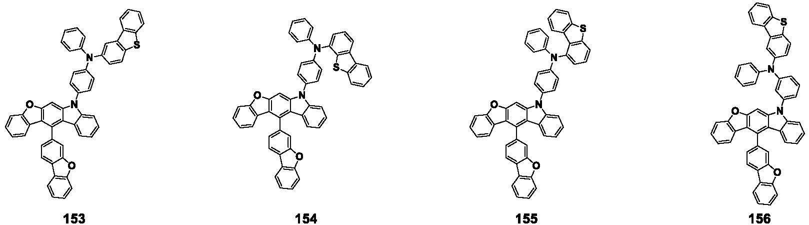

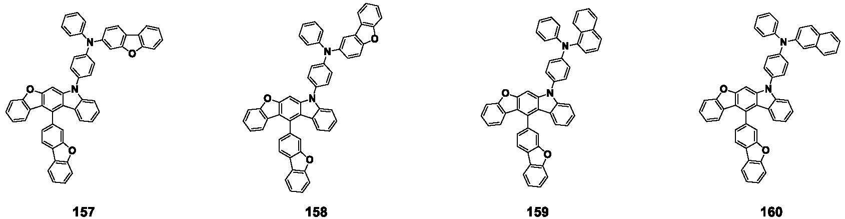

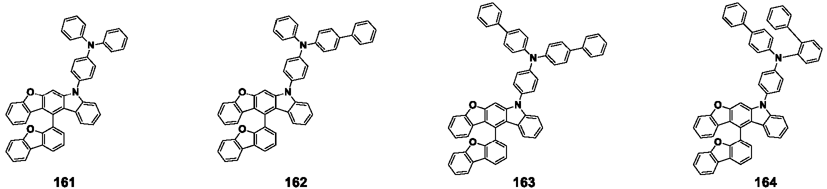

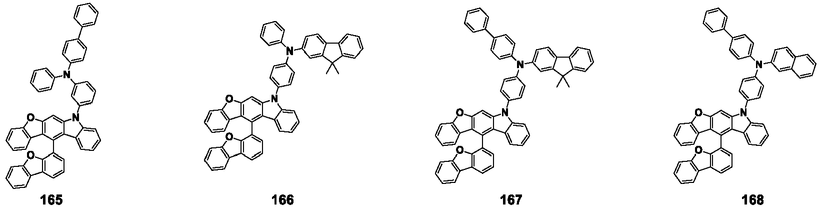

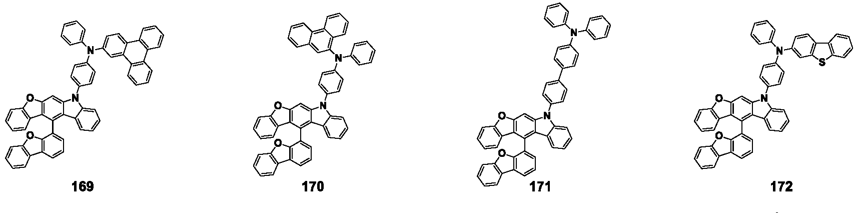

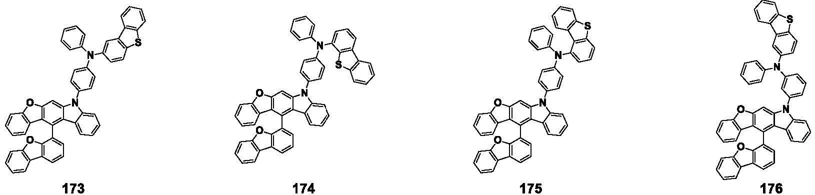

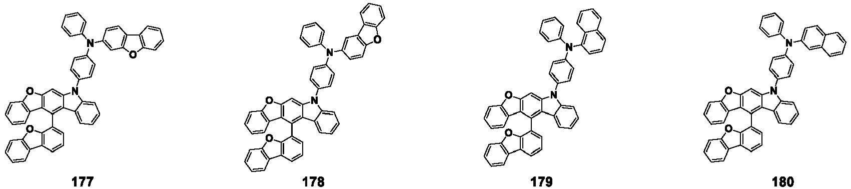

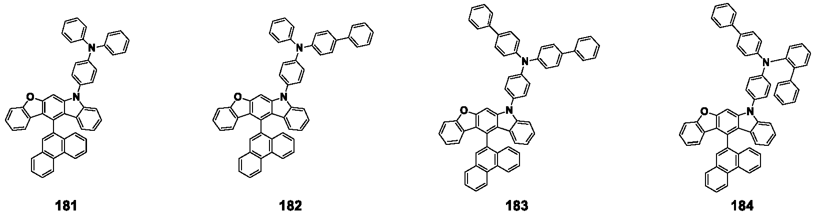

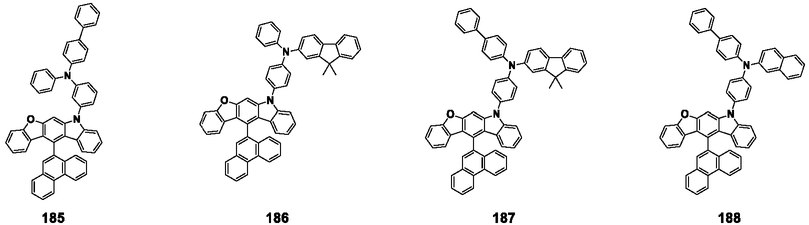

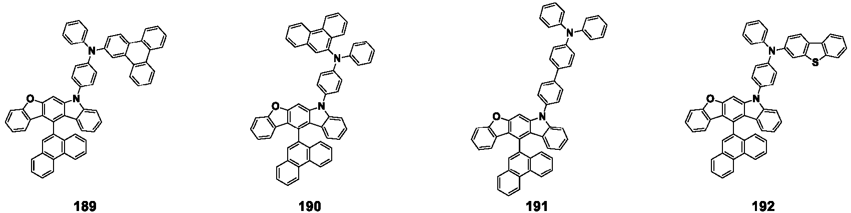

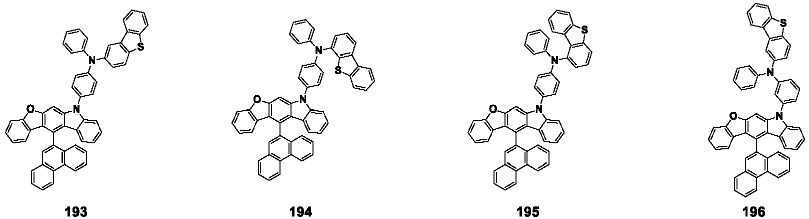

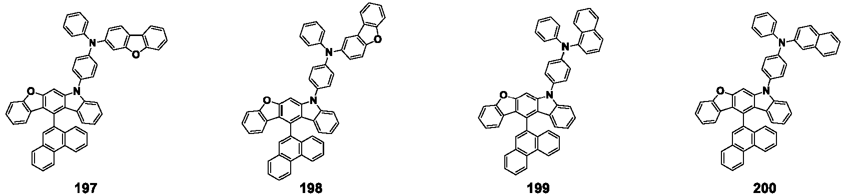

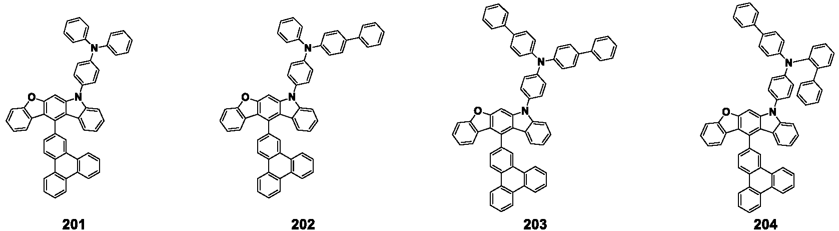

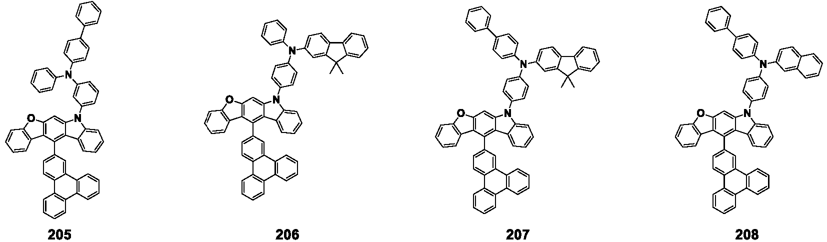

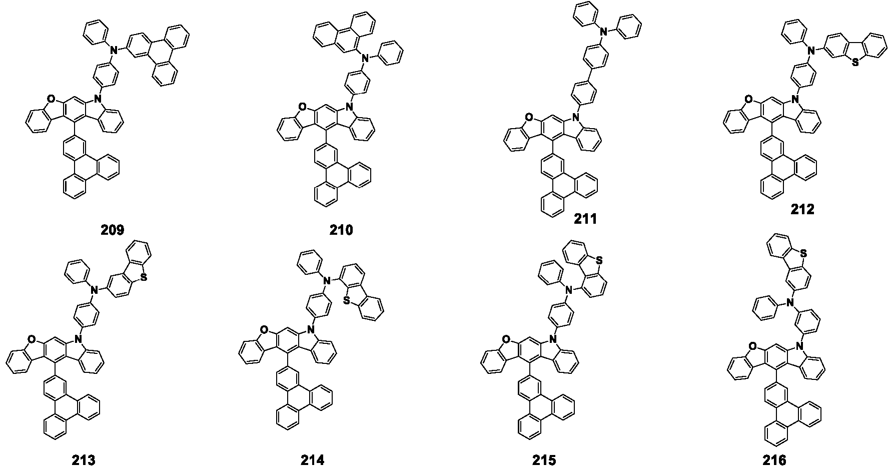

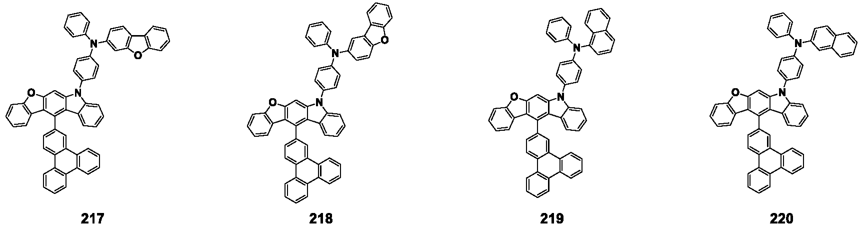

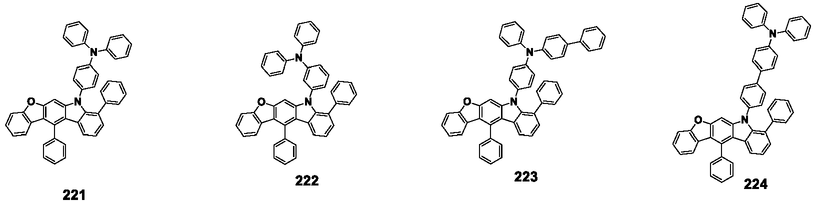

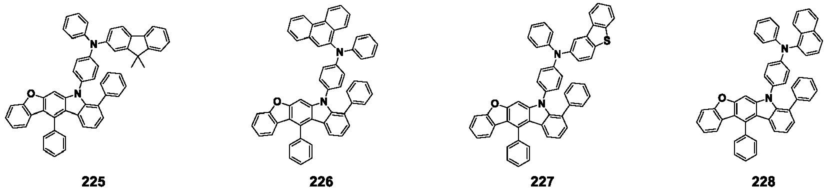

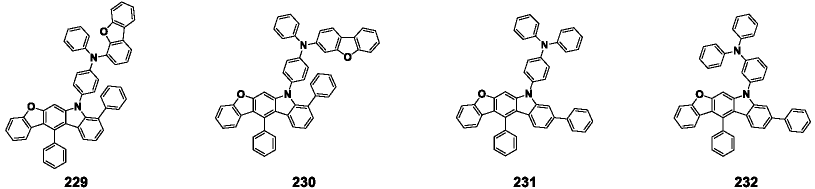

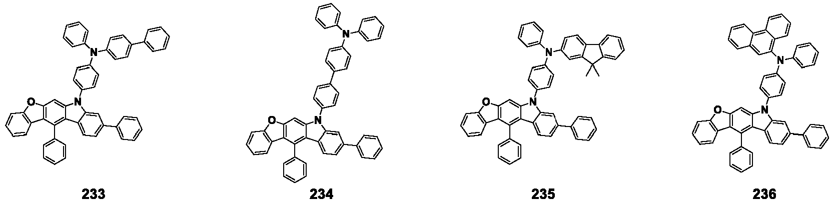

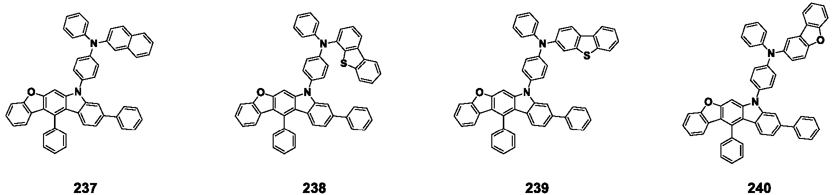

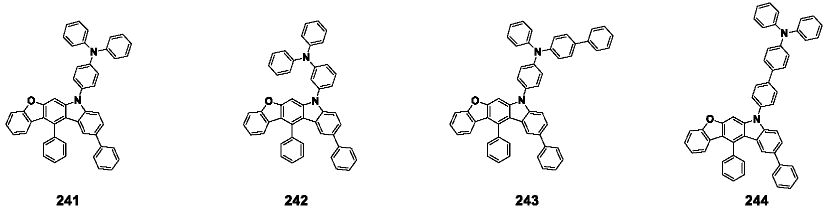

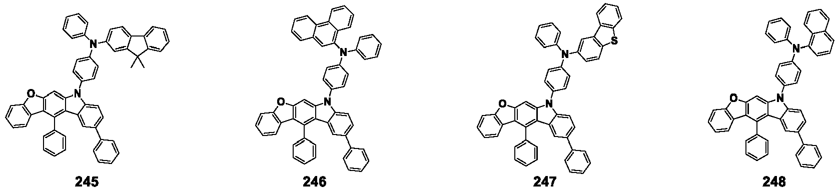

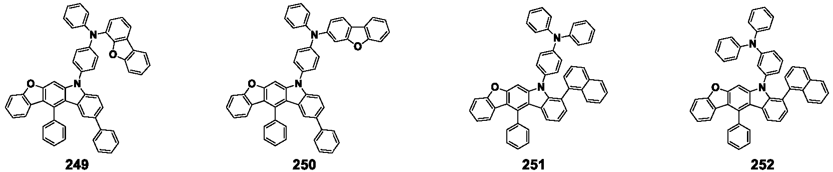

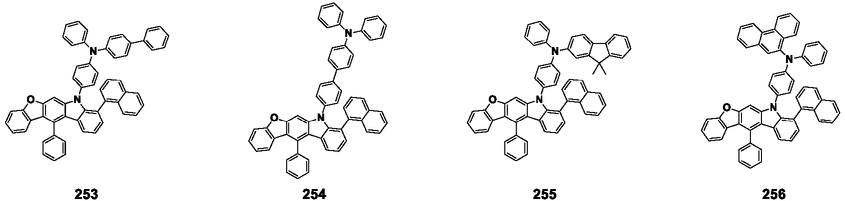

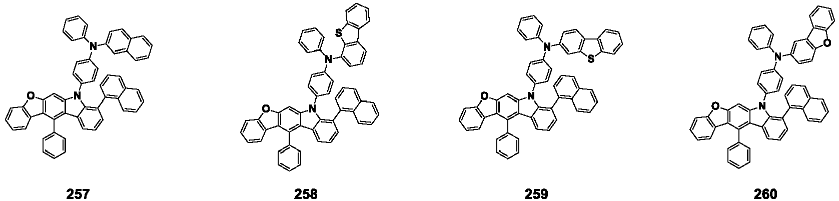

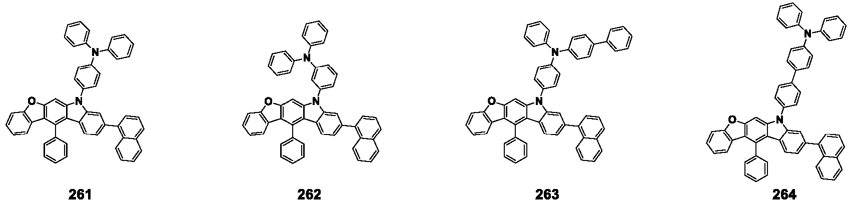

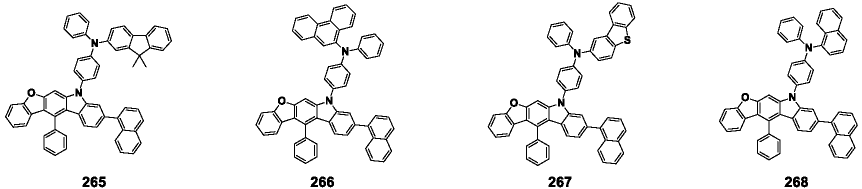

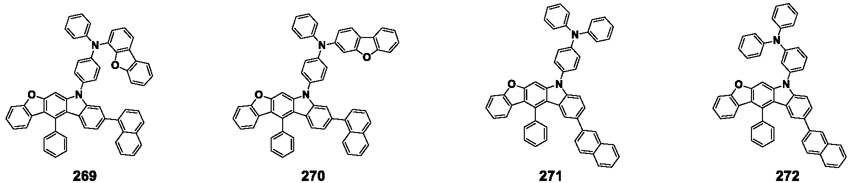

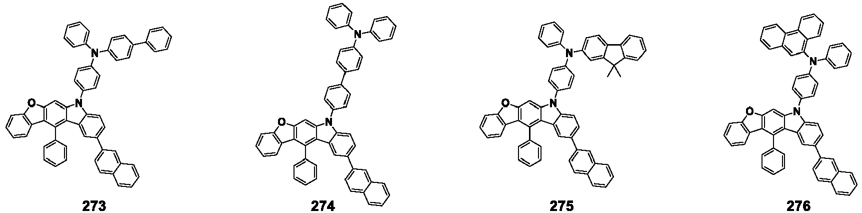

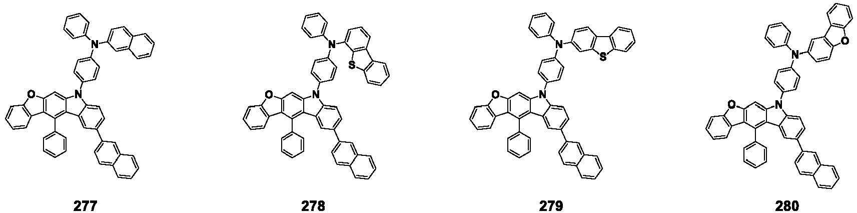

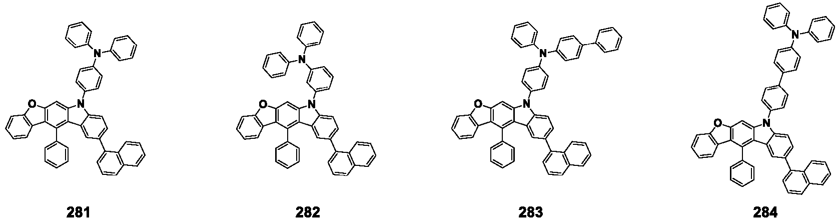

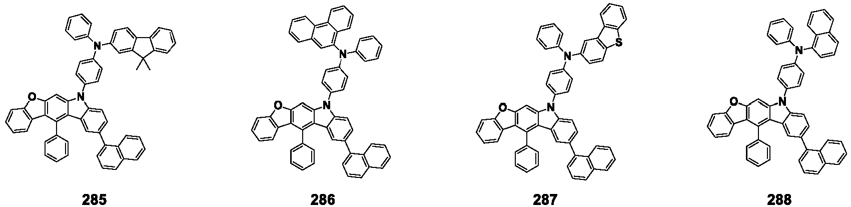

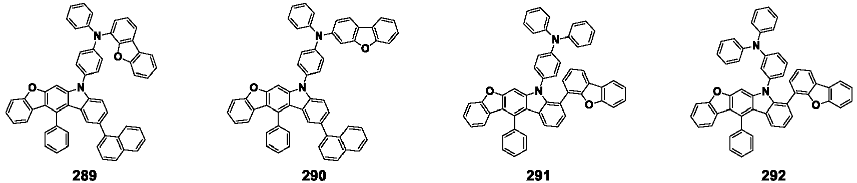

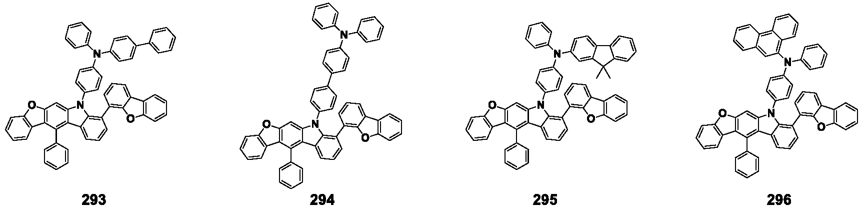

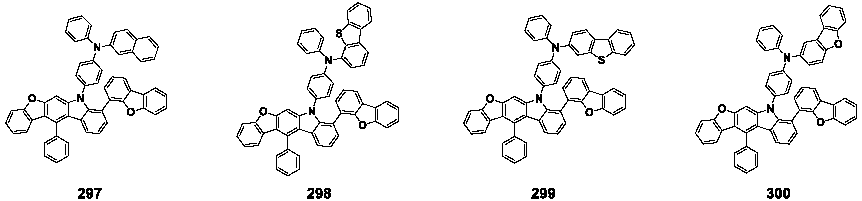

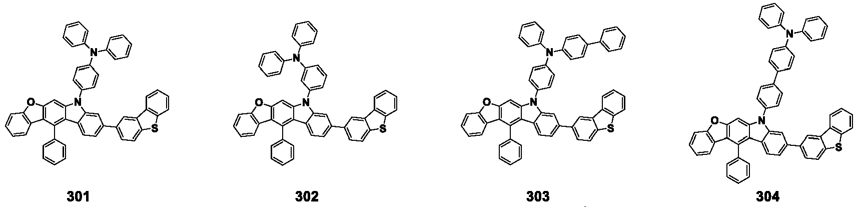

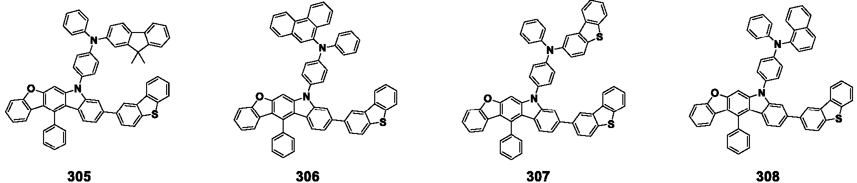

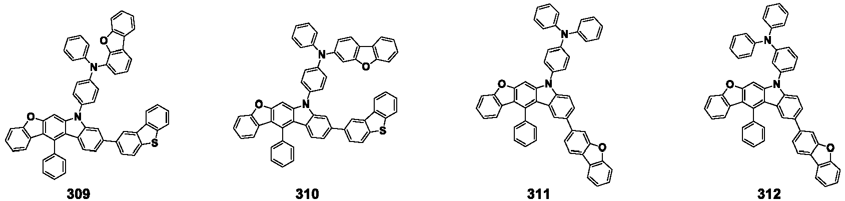

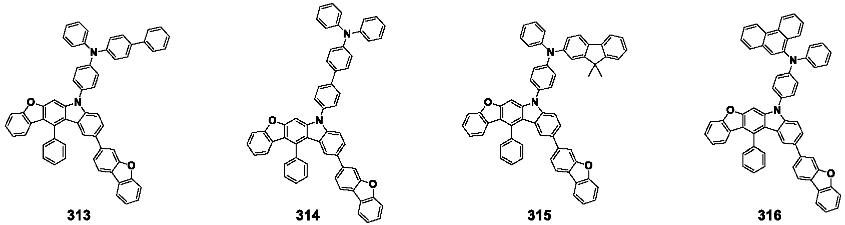

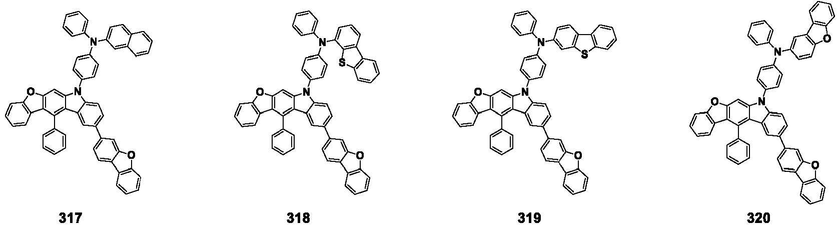

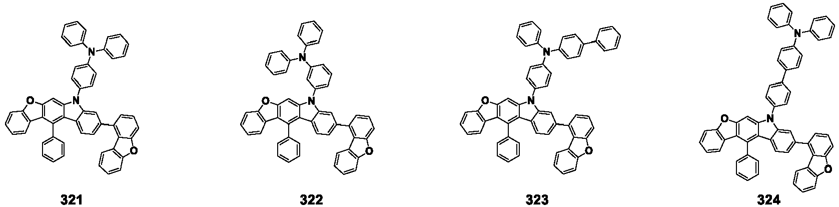

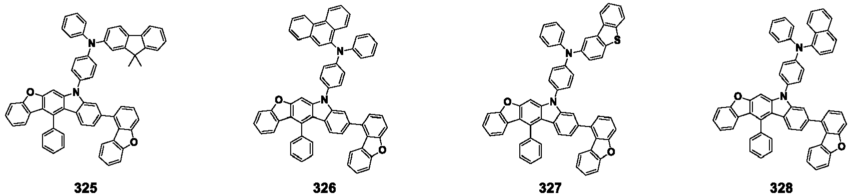

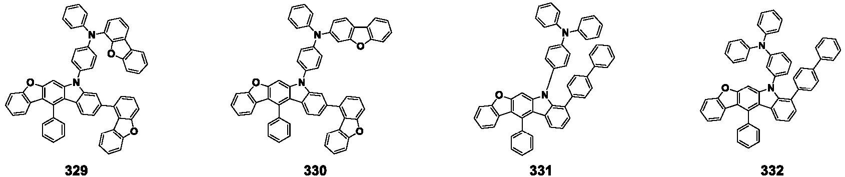

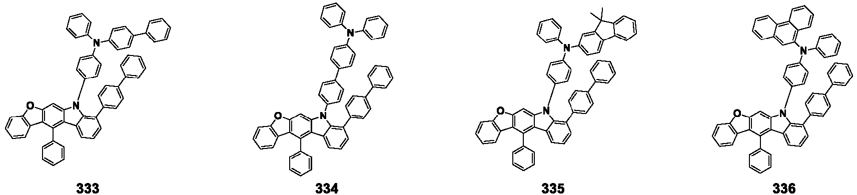

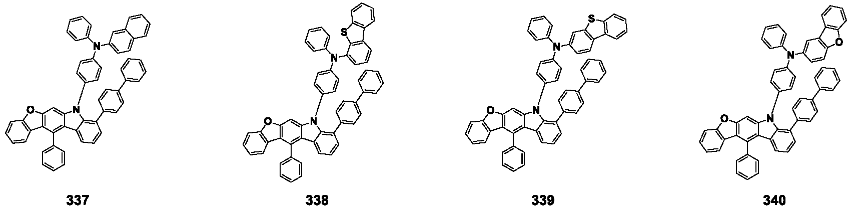

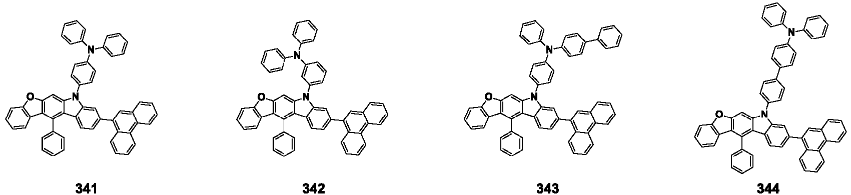

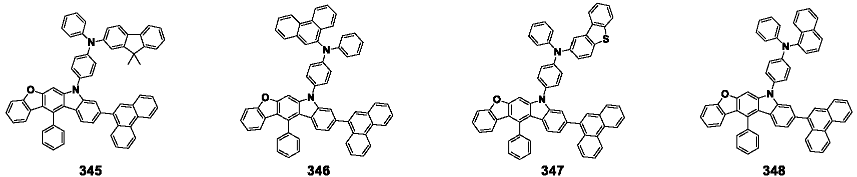

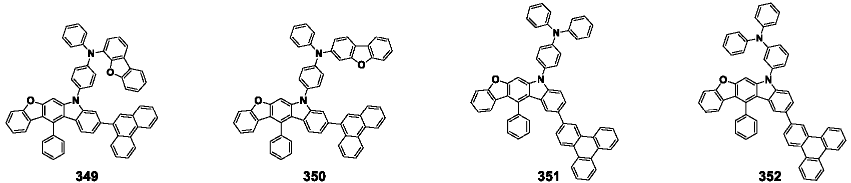

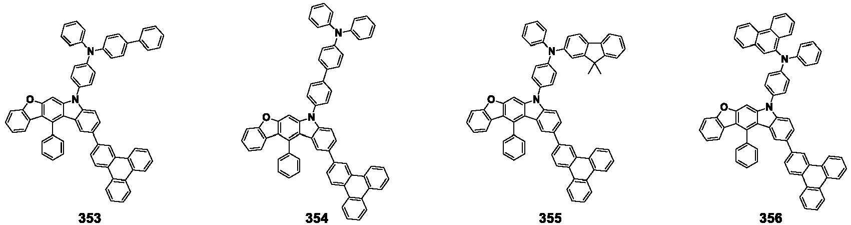

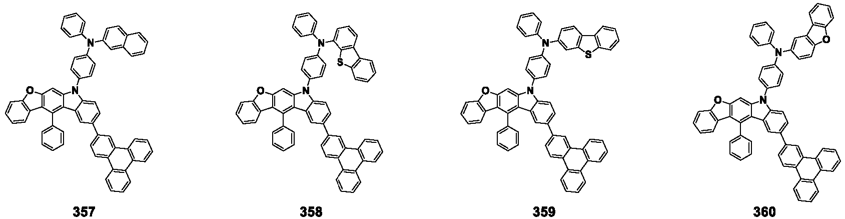

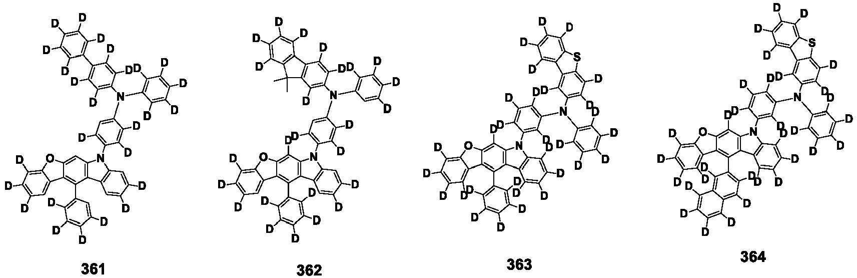

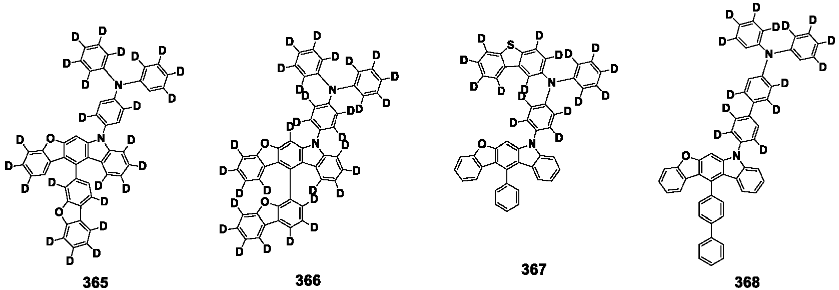

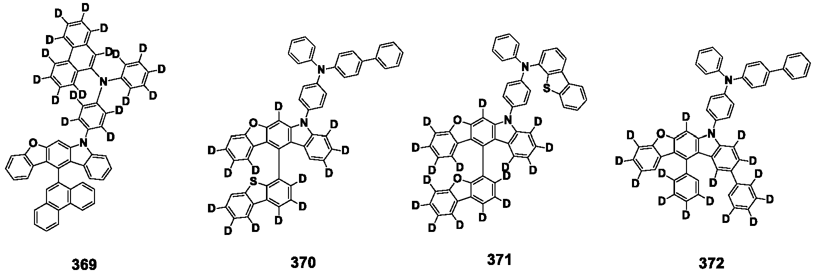

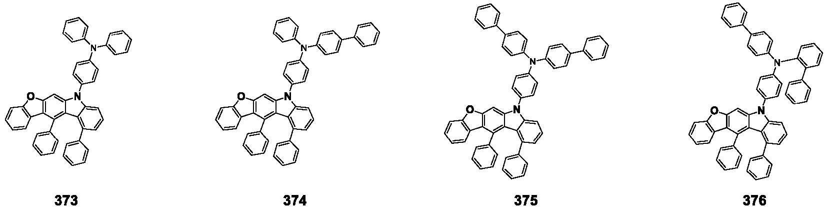

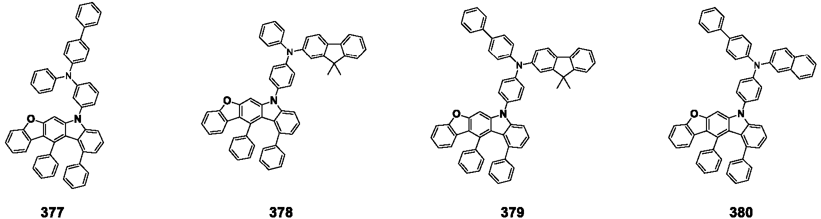

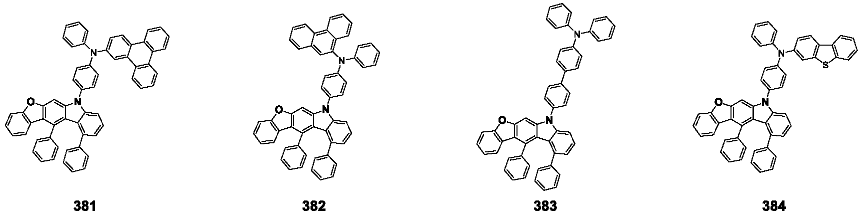

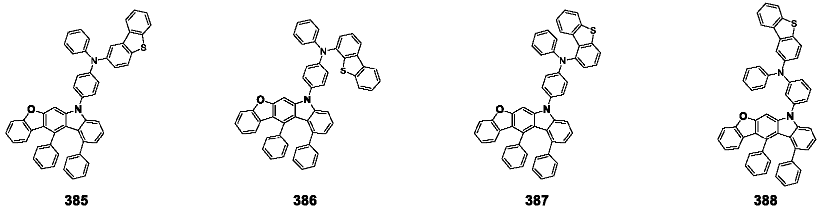

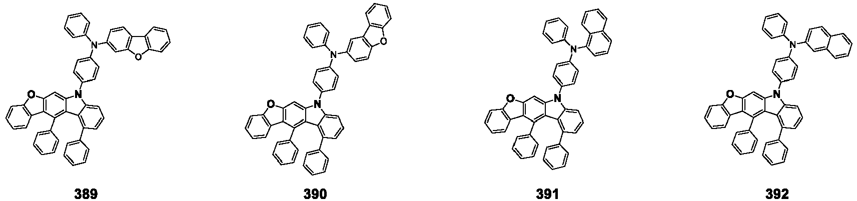

In addition, by introducing various substituents to the structure of Chemical Formula 1, compounds having unique properties of the introduced substituents may be synthesized. For example, by introducing substituents normally used for a hole injection layer material, a hole transport layer material, a light emitting layer material, an electron transport layer material, an electron blocking layer material and a charge generation layer material used for manufacturing an organic light emitting device to the core structure, materials satisfying conditions required for each organic material layer may be synthesized.

In addition, by introducing various substituents to the structure of Chemical Formula **1,** the energy bandgap may be finely controlled, and meanwhile, properties at interfaces between organic materials may be enhanced, and material applications may become diverse.

Meanwhile, the heterocyclic compound represented by Chemical Formula 1 has a high glass transition temperature (Tg), and thereby has excellent thermal stability. Such an increase in the thermal stability becomes an important factor providing driving stability to a device.

The heterocyclic compound according to one embodiment of the present disclosure may be prepared using a multi-step chemical reaction. Some intermediate compounds are prepared first, and from the intermediate compounds, the heterocyclic compound represented by Chemical Formula 1 may be prepared. More specifically, the heterocyclic compound according to one embodiment of the present disclosure may be prepared based on preparation examples to be described later.

Another embodiment of the present disclosure provides an organic light emitting device including the heterocyclic compound represented by Chemical Formula **1.** The "organic light emitting device" may be expressed in terms such as an "organic light emitting diode", an "OLED", an "OLED device" and an "organic electroluminescent device".

In addition, one embodiment of the present disclosure provides an organic light emitting device comprising:
a first electrode;
a second electrode provided opposite to the first electrode; and
one or more organic material layers provided between the first electrode and the second electrode,
wherein at least one of the one or more organic material layers comprise the heterocyclic compound represented by Chemical Formula 1.

In one embodiment of the present disclosure, the first electrode may be a positive electrode, and the second electrode may be a negative electrode.

In another embodiment, the first electrode may be a negative electrode, and the second electrode may be a positive electrode.

In one embodiment of the present disclosure, the organic material layer may include one or more types selected from the group consisting of an electron injection layer, an electron transport layer, a hole blocking layer, a light emitting layer, a light emitting auxiliary layer, an electron blocking layer, a hole transport layer and a hole injection layer, and one or more types of the layers selected from the group consisting of the electron injection layer, the electron transport layer, the hole blocking layer, the light emitting layer, the light emitting auxiliary layer, the electron blocking layer, the hole transport layer and the hole injection layer may include the heterocyclic compound represented by Chemical Formula **1.**

In another embodiment of the present disclosure, the organic material layer may include a hole transport layer, and the hole transport layer may include the heterocyclic compound represented by Chemical Formula 1. When the heterocyclic compound is used in the hole transport layer, the heterocyclic compound has high hole mobility and has a proper HOMO level, and therefore, using the heterocyclic compound in the hole transport layer facilitates hole transport to a light emitting layer, thereby lowering a driving voltage of an organic light emitting device and enhancing driving efficiency and lifetime.

In another embodiment of the present disclosure, the organic material layer may include an electron blocking layer, and the electron blocking layer may include the heterocyclic compound represented by Chemical Formula 1. The heterocyclic compound has a high LUMO level, and therefore, using the heterocyclic compound in the electron blocking layer may increase probability of holes and electrons forming excitons, and may increase possibility of being emitted as light in a light emitting layer. Accordingly, an electron blocking ability is enhanced and holes and electrons form a charge balance, which may result in excellent driving efficiency and lifetime of an organic light emitting device.

In another embodiment of the present disclosure, the organic material layer may include an electron injection layer or an electron transport layer, and the electron injection layer or the electron transport layer may include the heterocyclic compound represented by Chemical Formula **1.**

In another embodiment of the present disclosure, the organic material layer may include an electron blocking layer or a hole blocking layer, and the electron blocking layer or the hole blocking layer may include the heterocyclic compound represented by Chemical Formula **1.**

In another embodiment of the present disclosure, the organic material layer may include an electron transport layer, a light emitting layer or a hole blocking layer, and the electron transport layer, the light emitting layer or the hole blocking layer may include the heterocyclic compound represented by Chemical Formula 1.

In another embodiment of the present disclosure, the organic material layer may include a hole transport layer, an electron blocking layer or a light emitting auxiliary layer, and the hole transport layer, the electron blocking layer or the light emitting auxiliary layer may include the heterocyclic compound represented by Chemical Formula 1.

In another embodiment of the present disclosure, the organic material layer may include a light emitting layer, and the light emitting layer may include the heterocyclic compound represented by Chemical Formula 1.

In another embodiment of the present disclosure, the organic material layer includes a light emitting layer, the light emitting layer includes a host material, and the host material may include the heterocyclic compound represented by Chemical Formula 1.

In one embodiment of the present disclosure, the organic light emitting device may be a red organic light emitting device, and the heterocyclic compound represented by Chemical Formula 1 may be used as a material of the red organic light emitting device.

In another embodiment of the present disclosure, the organic light emitting device may be a blue organic light emitting device, and the heterocyclic compound represented by Chemical Formula 1 may be used as a material of the blue organic light emitting device.

In another embodiment of the present disclosure, the organic light emitting device may be a green organic light emitting device, and the heterocyclic compound represented by Chemical Formula 1 may be used as a material of the green organic light emitting device.

In one embodiment of the present disclosure, the organic light emitting device may be a red organic light emitting device, and the heterocyclic compound represented by Chemical Formula 1 may be used as a light emitting layer material of the red organic light emitting device.

In another embodiment of the present disclosure, the organic light emitting device may be a blue organic light emitting device, and the heterocyclic compound represented by Chemical Formula 1 may be used as a light emitting layer material of the blue organic light emitting device.

In another embodiment of the present disclosure, the organic light emitting device may be a green organic light emitting device, and the heterocyclic compound represented by Chemical Formula 1 may be used as a light emitting layer material of the green organic light emitting device.

In one embodiment of the present disclosure, the organic light emitting device may further include one, two or more layers selected from the group consisting of a light emitting layer, a hole injection layer, a hole transport layer, an electron injection layer, an electron transport layer, an electron blocking layer and a hole blocking layer.

Specific descriptions on the heterocyclic compound represented by Chemical Formula 1 are the same as the descriptions provided above.

FIGS. 1 to 3 illustrate a lamination order of electrodes and organic material layers of the organic light emitting device according to one embodiment of the present disclosure. However, it is not intended that the scope of the present application be limited by these drawings, and structures of organic light emitting devices known in the art may also be applied to the present application.

FIG. 1 illustrates an organic light emitting device in which a positive electrode 200, an organic material layer 300 and a negative electrode 400 are sequentially laminated on a substrate 100. However, the structure is not limited only to such a structure, and as illustrated in FIG. **2****,** an organic light emitting device in which a negative electrode (400), an organic material layer (300) and a positive electrode (200) are sequentially laminated on a substrate may also be obtained.

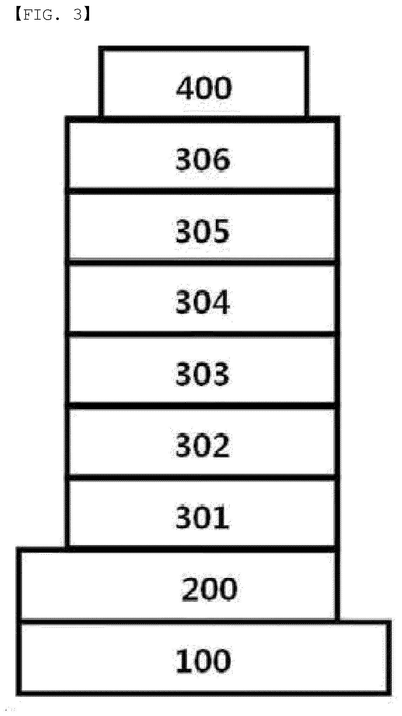

FIG. 3 illustrates a case of the organic material layer being a multilayer. An organic light emitting device according to FIG. 3 includes a hole injection layer 301, a hole transport layer 302, a light emitting layer 303, a hole blocking layer 304, an electron transport layer 305 and an electron injection layer 306. However, the scope of the present application is not limited by such a lamination structure, and as necessary, the layers other than the light emitting layer may not be included, and other necessary functional layers may be further added. For example, a light emitting auxiliary layer may be added (not shown in FIG. 3).

The organic light emitting device of the present disclosure may be manufactured using common organic light emitting device manufacturing methods and materials except that one or more organic material layers are formed using the heterocyclic compound represented by Chemical Formula 1 described above.

The heterocyclic compound may be formed into an organic material layer using a solution coating method as well as a vacuum deposition method when the organic light emitting device is manufactured. Herein, the solution coating method means spin coating, dip coating, inkjet printing, screen printing, a spray method, roll coating and the like, but is not limited thereto.

The organic material layer of the organic light emitting device of the present disclosure may be formed in a single layer structure, but may also be formed in a multilayer structure in which two or more organic material layers are laminated. For example, the organic light emitting device of the present disclosure may have a structure including an electron injection layer, an electron transport layer, a hole blocking layer, a light emitting layer, a light emitting auxiliary layer, an electron blocking layer, a hole transport layer, an electron injection layer and the like as the organic material layer. However, the structure of the organic light emitting device is not limited thereto, and may include a smaller number of organic material layers.

In addition, one embodiment of the present disclosure provides a composition for an organic material layer, the composition including the heterocyclic compound represented by Chemical Formula 1.

Specific descriptions on the heterocyclic compound represented by Chemical Formula 1 are the same as the descriptions provided above.

The composition for an organic material layer may be used when forming an organic material layer of an organic light emitting device, and particularly, may be more preferably used when forming a hole transport layer, an electron blocking layer or a light emitting auxiliary layer.

In one embodiment of the present disclosure, the organic material layer includes the heterocyclic compound represented by Chemical Formula 1, and a phosphorescent dopant may be used therewith.

As the phosphorescent dopant material, those known in the art may be used. For example, phosphorescent dopant materials represented by LL'MX', LL'L"M, LMX'X", L₂MX' and L₃M may be used, however, the scope of the present disclosure is not limited by these examples.

M may be iridium, platinum, osmium or the like.

L is an anionic bidentate ligand coordinated to M by sp² carbon and heteroatom, and X may function to trap electrons or holes. Nonlimiting examples of L may include 2-(1-naphthyl)benzoxazole, 2-phenylbenzoxazole, 2-phenylbenzothiazole, 7,8-benzoquinoline, phenylpyridine, benzothiophenylpyridine, 3-methoxy-2-phenylpyridine, thiophenylpyridine, tolylpyridine and the like. Nonlimiting examples of X' and X" may include acetylacetonate (acac), hexafluoroacetylacetonate, salicylidene, picolinate, 8-hydroxyquinolinate and the like.

Specific examples of the phosphorescent dopant are shown below, however, the phosphorescent dopant is not limited to these examples.

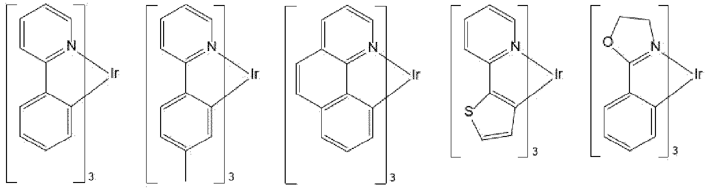

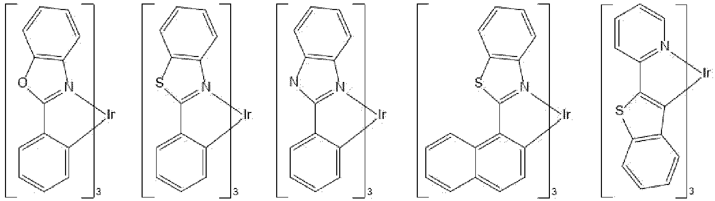

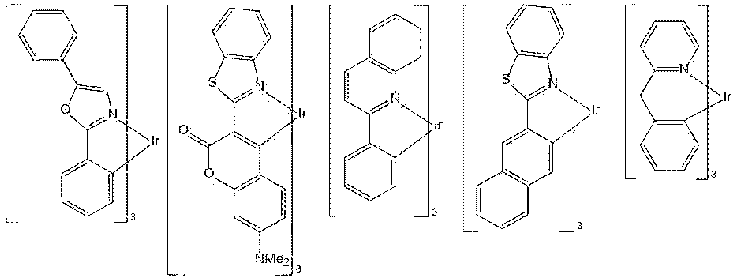

In one embodiment of the present disclosure, the organic material layer includes the heterocyclic compound represented by Chemical Formula 1, and an iridium-based dopant may be used therewith.

In one embodiment of the present disclosure, as the iridium-based dopant, (piq)₂(Ir) (acac) may be used as a red phosphorescent dopant or Ir(ppy)₃ may be used as a green phosphorescent dopant.

In one embodiment of the present disclosure, a content of the dopant may be from 1% to 15%, preferably from 2% to 10% and more preferably from 3% to 7% based on the total weight of the light emitting layer.

One embodiment of the present disclosure provides a method for manufacturing an organic light emitting device, the method including:
preparing a substrate;
forming a first electrode on the substrate;
forming one or more organic material layers on the first electrode; and
forming a second electrode on the one or more organic material layers,
wherein the forming of one or more organic material layers includes forming the one or more organic material layers using the composition for an organic material layer of an organic light emitting device according to one embodiment of the present disclosure.

In one embodiment of the present disclosure, the forming of organic material layers may be forming the heterocyclic compound represented by Chemical Formula 1 using a thermal vacuum deposition method.

The organic material layer including the heterocyclic compound represented by Chemical Formula 1 may further include other materials as necessary.

In the organic light emitting device according to one embodiment of the present disclosure, materials other than the heterocyclic compound represented by Chemical Formula 1 are illustrated below, however, these are for illustrative purposes only and not for limiting the scope of the present application, and these materials may be replaced by materials known in the art.

As the positive electrode material, materials each having a relatively large work function may be used, and transparent conductive oxides, metals, conductive polymers or the like may be used. Specific examples of the positive electrode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the negative electrode material, materials each having a relatively small work function may be used, and metals, metal oxides, conductive polymers or the like may be used. Specific examples of the negative electrode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection layer material, known hole injection layer materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tris[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], conductive polymers having solubility such as polyaniline/dodecylbenzenesulfonic acid or poly(3,4-ethylenedioxythiophene)/poly(4-styrenesulfonate), polyaniline/camphor sulfonic acid or polyaniline/poly(4-styrenesulfonate), and the like, may be used.

As the hole transport layer material, pyrazoline derivatives, arylamine-based derivatives, stilbene derivatives, triphenyldiamine derivatives and the like may be used, and low molecular or high molecular materials may also be used.

As the electron transport layer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials as well as low molecular materials may also be used.

As examples of the electron injection layer material, LiF is typically used in the art, however, the present application is not limited thereto.

As the light emitting layer material, red, green or blue light emitting materials may be used, and as necessary, two or more light emitting materials may be mixed and used. Herein, the two or more light emitting materials may be deposited as individual sources of supply or pre-mixed and deposited as one source of supply when used. In addition, fluorescent materials may also be used as the light emitting layer material, however, phosphorescent materials may also be used. As the light emitting layer material, materials emitting light alone by binding holes and electrons injected from a positive electrode and a negative electrode, respectively, may be used, however, materials having a host material and a dopant material involving together in light emission may also be used.

When hosts of the light emitting layer material are mixed and used, same series hosts may be mixed and used, or different series hosts may be mixed and used. For example, any two or more types of materials among n-type host materials and p-type host materials may be selected and used as a host material of a light emitting layer.

The organic light emitting device according to one embodiment of the present disclosure may be a top-emission type, a bottom-emission type or a dual-emission type depending on the materials used.

The heterocyclic compound according to one embodiment of the present disclosure may also be used in an organic electronic device including an organic solar cell, an organic photo conductor, an organic transistor and the like under a principle similar to that in the organic light emitting device.

Hereinafter, preferred examples are provided to help to understand the present disclosure, however, the following examples are only provided to more readily understand the present disclosure, and the present disclosure is not limited thereto.

### <Preparation Example>

### Preparation Example 1. Preparation of Compound 002

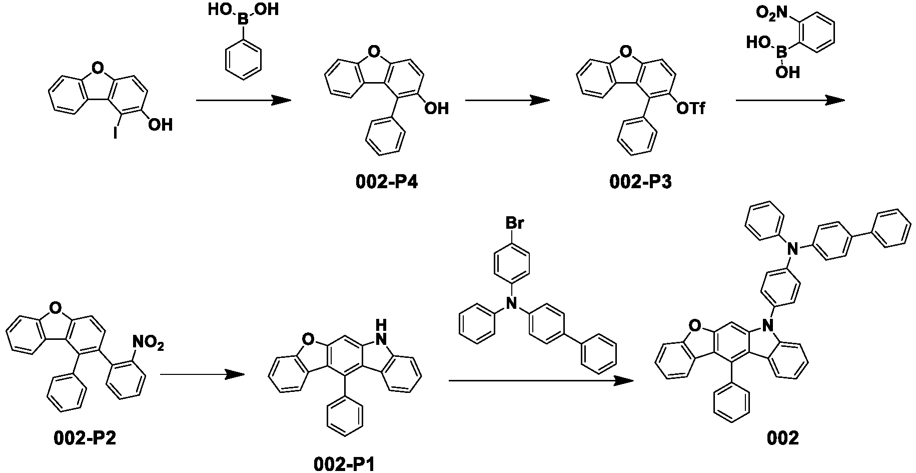

### Preparation Example 1-1. Preparation of Compound 002-P4

After dissolving 1-iododibenzo[b,d]furan-2-ol (50 g, 161.24 mmol) and phenylboronic acid (21.63 g, 177.37 mmol) in 1,4-dioxane (500 mL) and distilled water (100 mL), tetrakis(triphenylphosphine)palladium(0) (Pd(PPh₃)₄) (5.59 g, 4.84 mmol) and potassium carbonate (K₂CO₃) (55.71 g, 403.11 mmol) were introduced thereto, and the mixture was stirred under reflux for 10 hours.

After the reaction was completed, the reaction solution was extracted with dichloromethane and distilled water. The organic layer was dried with anhydrous MgSO₄, and then the solvent was removed using a rotary evaporator. After that, the result was purified by column chromatography using dichloromethane and hexane as a developing solvent to obtain Compound 002-P4 (35 g, yield 83%).

### Preparation Example 1-2. Preparation of Compound 002-P3

After dissolving Compound 002-P4 (35 g, 134.47 mmol) in dichloromethane (500 mL), triethylamine (16.33 g, 161.36 mmol) was added thereto, and after slowly adding triflic anhydride (45.53 g, 161.36 mmol) thereto at 0°C, the mixture was stirred for 1 hour.

After the reaction was completed, distilled water was slowly added to the reaction solution to terminate the reaction, and then the result was extracted with dichloromethane and distilled water. The organic layer was dried with anhydrous MgSO₄, and then the solvent was removed using a rotary evaporator. After that, the result was purified by column chromatography using dichloromethane and hexane as a developing solvent to obtain Compound 002-P3 (47 g, yield 89%).

### Preparation Example 1-3. Preparation of Compound 002-P2

After dissolving Compound 002-P3 (47 g, 119.79 mmol) and (2-nitrophenyl)boronic acid (22 g, 131.77 mmol) in 1,4-dioxane (500 mL) and distilled water (100 mL), tetrakis(triphenylphosphine)palladium(0) (Pd(PPh₃)₄) (6.92 g, 5.99 mmol) and potassium carbonate (K₂CO₃) (41.39 g, 299.48 mmol) were introduced thereto, and the mixture was stirred under reflux for 10 hours.

After the reaction was completed, the reaction solution was extracted with dichloromethane and distilled water. The organic layer was dried with anhydrous MgSO₄, and then the solvent was removed using a rotary evaporator. After that, the result was purified by column chromatography using dichloromethane and hexane as a developing solvent to obtain Compound 002-P2 (36 g, yield 82%).

### Preparation Example 1-4. Preparation of Compound 002-P1

Compound 002-P2 (36 g, 98.53 mmol) and triphenylphosphine (64.61 g, 246.32 mmol) were introduced to 1,2-dichlorobenzene (400 mL), and the mixture was stirred under reflux for 7 hours.

After the reaction was completed, the reaction solution was extracted with dichloromethane and distilled water. The organic layer was dried with anhydrous MgSO₄, and then the solvent was removed using a rotary evaporator. After that, the result was purified by column chromatography using dichloromethane and hexane as a developing solvent to obtain Compound 002-P1 (28 g, yield 85%).

### Preparation Example 1-5. Preparation of Compound 002

After dissolving Compound 002-P1 (10 g, 30 mmol) and N-(4-bromophenyl)-N-phenyl-[1,1'-biphenyl]-4-amine (12.61 g, 31.5 mmol) in toluene (100 mL), tris(dibenzylideneacetone)dipalladium(0) (Pd₂(dba)₃) (1.37 g, 1.5 mmol), Xphos (1.43 g, 3 mmol) and sodium tert-butoxide (NaOtBu) (5.77 g, 59.99 mmol) were introduced thereto, and the mixture was stirred under reflux for 2 hours.

After the reaction was completed, the reaction solution was extracted with dichloromethane and distilled water. The organic layer was dried with anhydrous MgSO₄, and then the solvent was removed using a rotary evaporator. After that, the result was purified by column chromatography using dichloromethane and hexane as a developing solvent to obtain Compound 002 (15 g, yield 77%).

Target compounds were prepared as in the following Table 1 and in the same manner as in Preparation Example 1 except that, Compound A of the following Table 1 was used instead of phenylboronic acid, Compound B of the following Table 1 was used instead of (2-nitrophenyl)boronic acid, and Compound C of the following Table 1 was used instead of N-(4-bromophenyl)-N-phenyl-[1,1'-biphenyl]-4-amine.

**[Table 1]**

| Comp ound No. | Compound A | Compound B | Compound C | Target Compound (Yield %) | Yie ld |
|---|---|---|---|---|---|
| 003 | 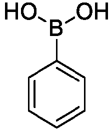 | 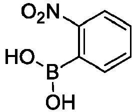 | 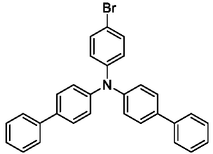 | 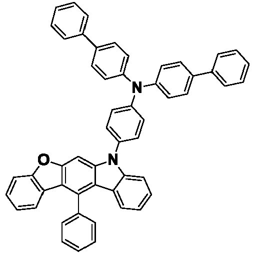 | 81% |
| 006 | 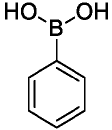 | 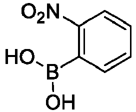 | 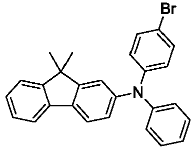 | 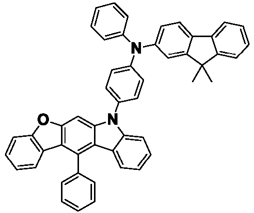 | 78% |
| 008 | 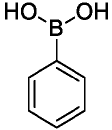 | 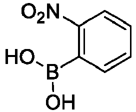 | 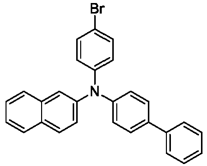 | 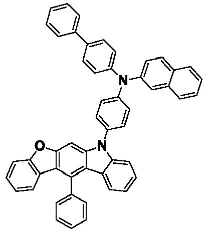 | 75% |
| 009 | 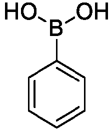 | 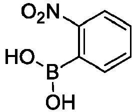 | 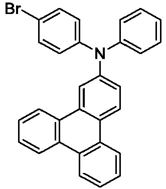 | 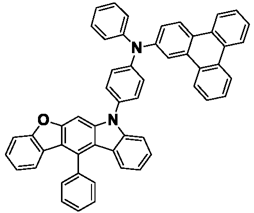 | 68% |
| 011 | 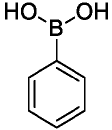 | 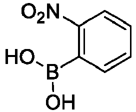 | 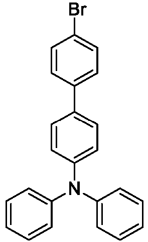 | 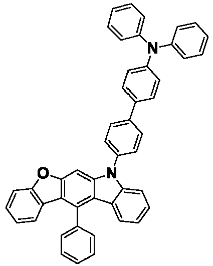 | 73% |
| 013 | 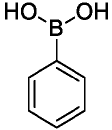 | 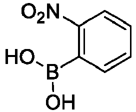 | 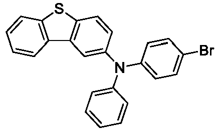 | 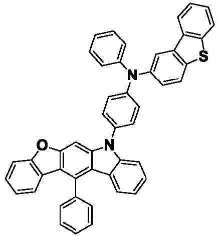 | 75% |
| 016 | 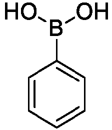 | 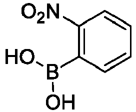 | 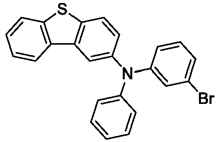 | 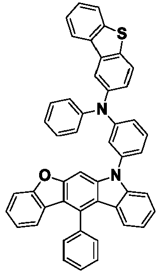 | 74% |
| 018 | 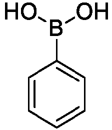 | 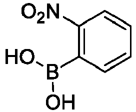 | 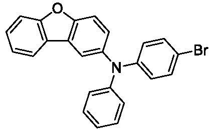 | 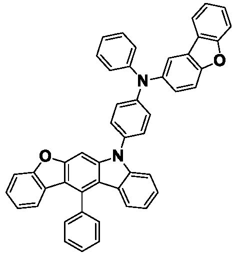 | 79% |
| 022 | 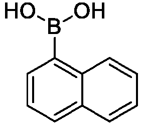 | 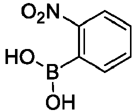 | 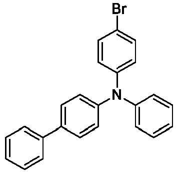 | 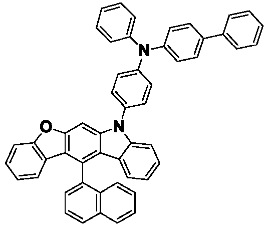 | 80% |
| 032 | 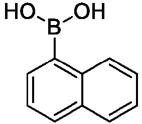 | 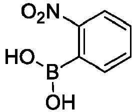 | 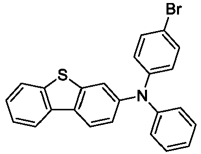 | 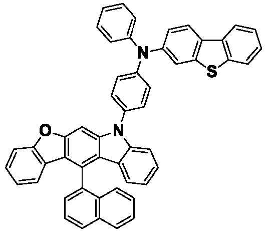 | 72% |
| 040 | 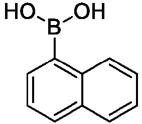 | 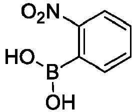 | 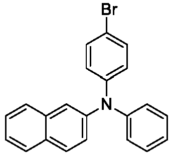 | 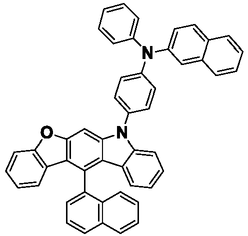 | 73% |
| 053 | 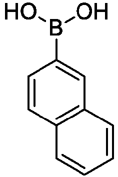 | 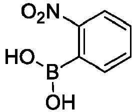 | 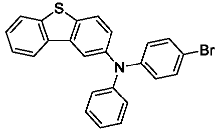 | 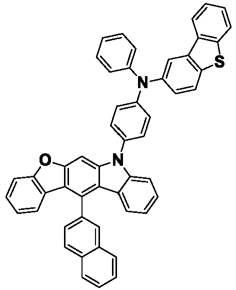 | 78% |
| 056 | 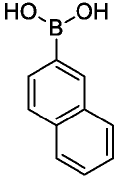 | 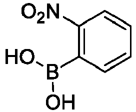 | 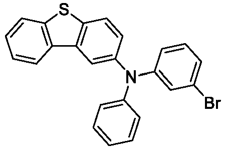 | 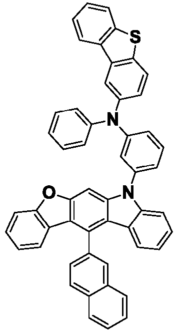 | 79% |
| 066 | 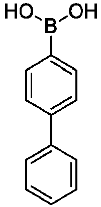 | 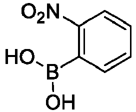 | 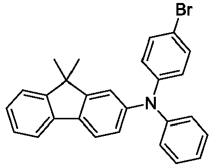 | 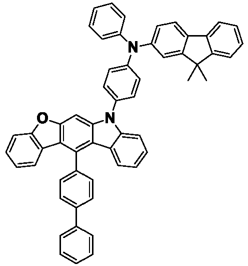 | 76% |
| 071 | 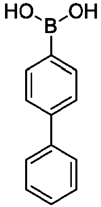 | 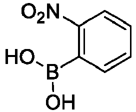 | 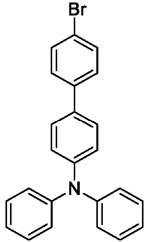 | 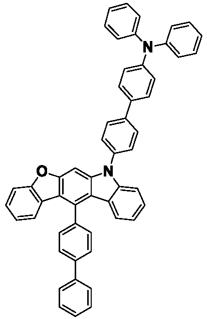 | 80% |
| 082 | 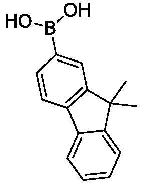 | 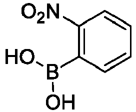 | 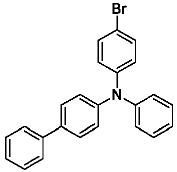 | 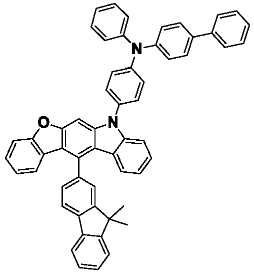 | 72% |
| 086 | 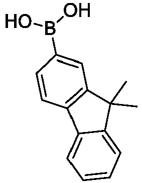 | 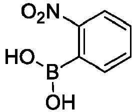 | 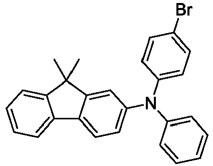 | 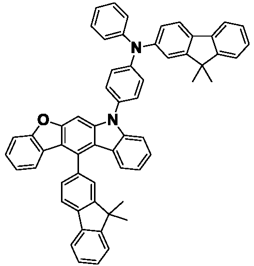 | 74% |
| 092 | 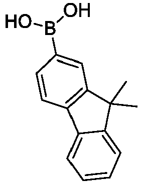 | 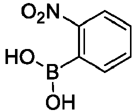 | 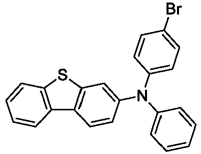 | 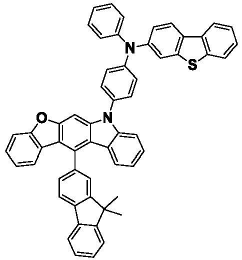 | 72% |
| 110 | 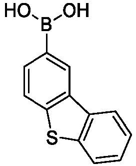 | 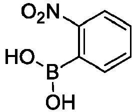 | 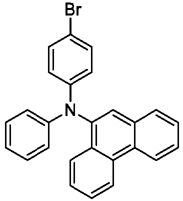 | 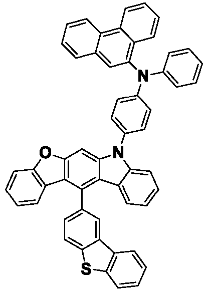 | 77% |
| 122 | 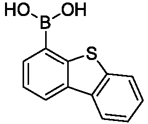 | 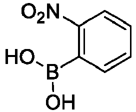 | 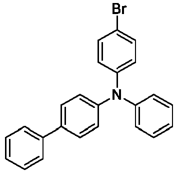 | 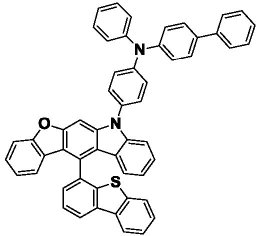 | 77% |
| 130 | 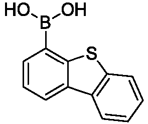 | 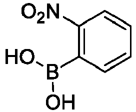 | 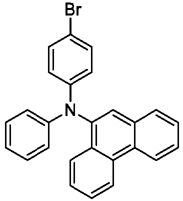 | 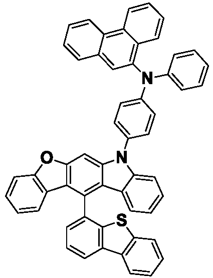 | 74% |
| 141 | 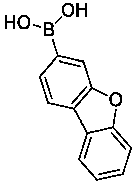 | 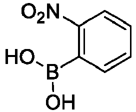 | 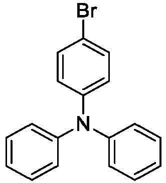 | 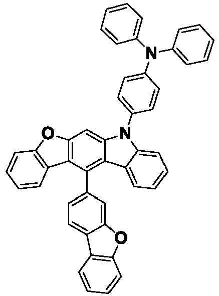 | 78% |
| 161 | 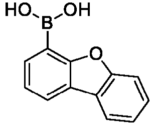 | 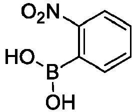 | 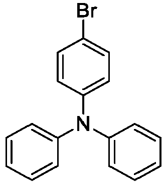 | 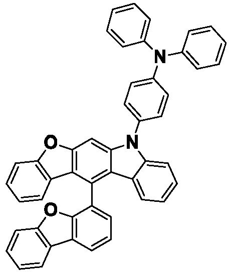 | 75% |
| 174 | 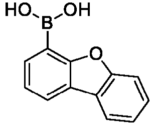 | 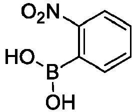 | 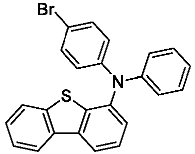 | 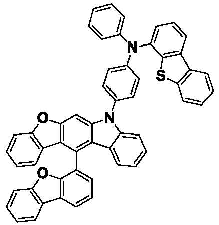 | 73% |
| 190 | 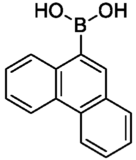 | 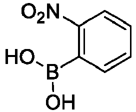 | 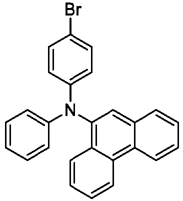 | 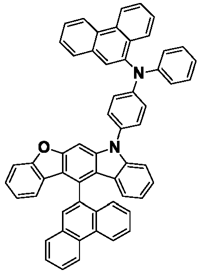 | 71% |
| 201 | 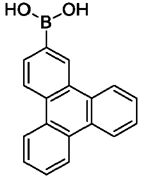 | 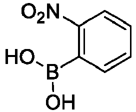 | 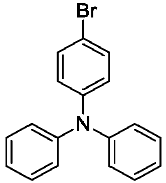 | 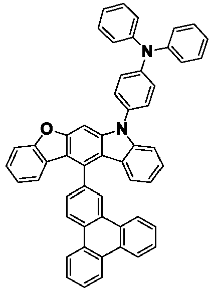 | 77% |
| 202 | 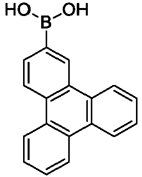 | 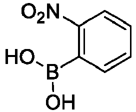 | 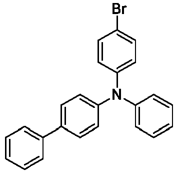 | 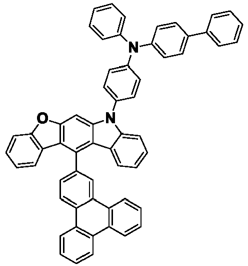 | 75% |
| 367 | 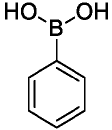 | 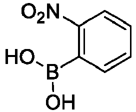 | 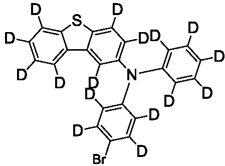 | 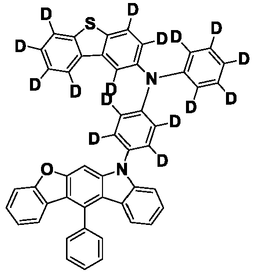 | 74% |

### Preparation Example 2. Preparation of Compound 231

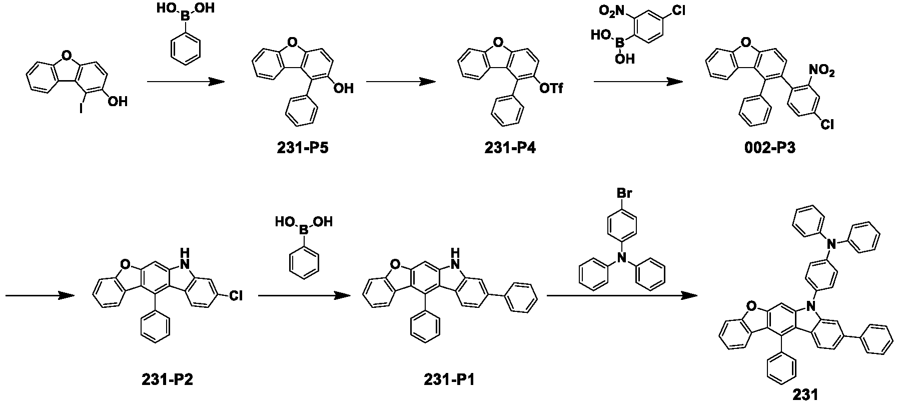

### Preparation Example 2-1. Preparation of Compound 231-P5

After dissolving 1-iododibenzo[b,d]furan-2-ol (50 g, 161.24 mmol) and phenylboronic acid (21.63 g, 177.37 mmol) in 1,4-dioxane (500 mL) and distilled water (100 mL), tetrakis(triphenylphosphine)palladium(0) (Pd(PPh₃)₄) (5.59 g, 4.84 mmol) and potassium carbonate (K₂CO₃) (55.71 g, 403.11 mmol) were introduced thereto, and the mixture was stirred under reflux for 10 hours.

After the reaction was completed, the reaction solution was extracted with dichloromethane and distilled water. The organic layer was dried with anhydrous MgSO₄, and then the solvent was removed using a rotary evaporator. After that, the result was purified by column chromatography using dichloromethane and hexane as a developing solvent to obtain Compound 231-P5 (35 g, yield 83%).

### Preparation Example 2-2. Preparation of Compound 231-P4

After dissolving Compound 231-P5 (35 g, 134.47 mmol) in dichloromethane (500 mL), triethylamine (16.33 g, 161.36 mmol) was added thereto, and after slowly adding triflic anhydride (45.53 g, 161.36 mmol) thereto at 0°C, the mixture was stirred for 1 hour.

After the reaction was completed, distilled water was slowly added to the reaction solution to terminate the reaction, and then the result was extracted with dichloromethane and distilled water. The organic layer was dried with anhydrous MgSO₄, and then the solvent was removed using a rotary evaporator. After that, the result was purified by column chromatography using dichloromethane and hexane as a developing solvent to obtain Compound 231-P4 (47 g, yield 89%).

### Preparation Example 2-3. Preparation of Compound 231-P3

After dissolving Compound 231-P4 (47 g, 119.79 mmol) and (4-chloro-2-nitrophenyl)boronic acid (26.53 g, 131.77 mmol) in 1,4-dioxane (500 mL) and distilled water (100 mL), tetrakis(triphenylphosphine)palladium(0) (Pd(PPh₃)₄) (6.92 g, 5.99 mmol) and potassium carbonate (K₂CO₃) (41.39 g, 299.48 mmol) were introduced thereto, and the mixture was stirred under reflux for 10 hours.

After the reaction was completed, the reaction solution was extracted with dichloromethane and distilled water. The organic layer was dried with anhydrous MgSO₄, and then the solvent was removed using a rotary evaporator. After that, the result was purified by column chromatography using dichloromethane and hexane as a developing solvent to obtain Compound 231-P3 (37 g, yield 77%).

### Preparation Example 2-4. Preparation of Compound 231-P2

Compound 231-P3 (37 g, 92.54 mmol) and triphenylphosphine (60.68 g, 231.35 mmol) were introduced to 1,2-dichlorobenzene (500 mL), and the mixture was stirred under reflux for 7 hours.

After the reaction was completed, the reaction solution was extracted with dichloromethane and distilled water. The organic layer was dried with anhydrous MgSO₄, and then the solvent was removed using a rotary evaporator. After that, the result was purified by column chromatography using dichloromethane and hexane as a developing solvent to obtain Compound 231-P2 (30 g, yield 88%).

### Preparation Example 2-5. Preparation of Compound 231-P1

After dissolving Compound 231-P2 (30 g, 81.56 mmol) and phenylboronic acid (10.94 g, 89.72 mmol) in 1,4-dioxane (300 mL) and distilled water (60 mL), tris(dibenzylideneacetone)dipalladium(0) (Pd₂(dba)₃) (0.94 g, 1.63 mmol), Xphos (1.94 g, 4.08 mmol) and potassium carbonate (K₂CO₃) (28.18 g, 203.9 mmol) were introduced thereto, and the mixture was stirred under reflux for 4 hours.

After the reaction was completed, the reaction solution was extracted with dichloromethane and distilled water. The organic layer was dried with anhydrous MgSO₄, and then the solvent was removed using a rotary evaporator. After that, the result was purified by column chromatography using dichloromethane and hexane as a developing solvent to obtain Compound 231-P1 (25 g, yield 75%).

### Preparation Example 2-6. Preparation of Compound 231

After dissolving Compound 231-P1 (8 g, 19.54 mmol) and 4-bromo-N,N-diphenylaniline (6.65 g, 20.51 mmol) in toluene (100 mL), tris(dibenzylideneacetone)dipalladium(0) (Pd₂(dba)₃) (0.89 g, 0.98 mmol), Xphos (0.93 g, 1.95 mmol) and sodium tert-butoxide (NaOtBu) (3.76 g, 39.07 mmol) were introduced thereto, and the mixture was stirred under reflux for 2 hours.

After the reaction was completed, the reaction solution was extracted with dichloromethane and distilled water. The organic layer was dried with anhydrous MgSO₄, and then the solvent was removed using a rotary evaporator. After that, the result was purified by column chromatography using dichloromethane and hexane as a developing solvent to obtain Compound 231 (9 g, yield 71%).

Target compounds were prepared as in the following Table 2 and in the same manner as in Preparation Example 2 except that, Compound D of the following Table 2 was used instead of phenylboronic acid of Preparation Example 2-1, Compound E of the following Table 2 was used instead of (4-chloro-2-nitrophenyl)boronic acid, Compound F of the following Table 2 was used instead of phenylboronic acid of Preparation Example 2-5, and Compound G of the following Table 2 was used instead of 4-bromo-N,N-diphenylaniline.

**[Table 2]**

| Com pou nd No. | Compound D | Compound E | Compound F | Compound G | Target Compound | Yie ld |
|---|---|---|---|---|---|---|
| 23 3 | 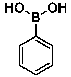 | 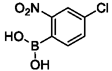 | 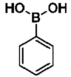 | 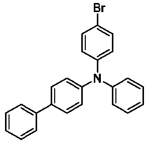 | 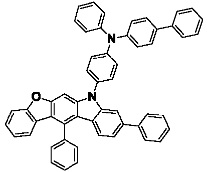 | 73% |
| 24 3 | 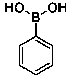 | 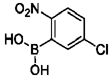 | 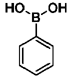 | 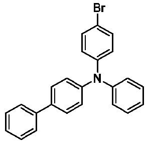 | 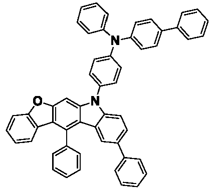 | 72% |
| 25 0 | 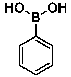 | 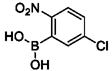 | 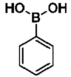 | 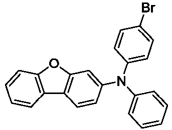 | 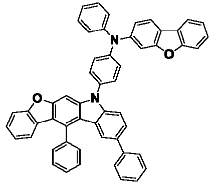 | 74% |
| 26 5 | 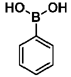 | 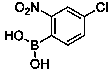 | 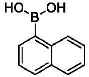 | 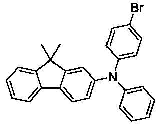 | 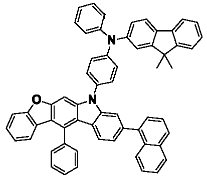 | 77% |
| 27 3 | 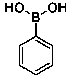 | 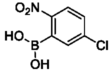 | 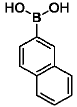 | 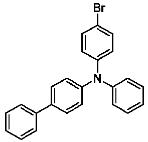 | 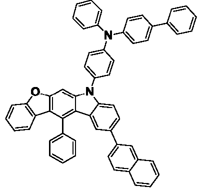 | 71% |
| 28 2 | 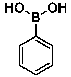 | 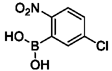 | 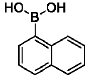 | 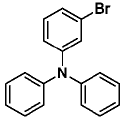 | 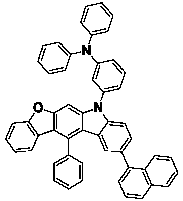 | 78% |
| 30 1 | 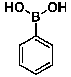 | 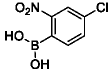 | 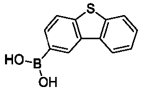 | 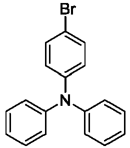 | 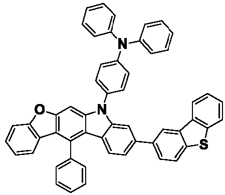 | 82% |
| 31 2 | 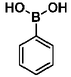 | 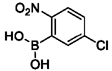 | 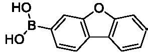 | 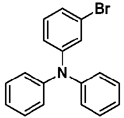 | 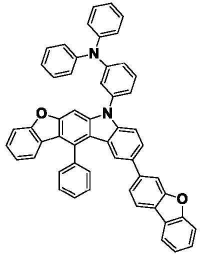 | 79% |
| 34 6 | 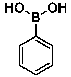 | 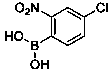 | 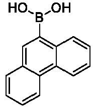 | 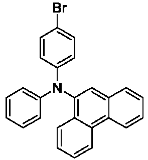 | 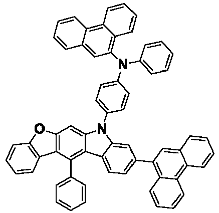 | 81% |
| 35 1 | 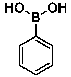 | 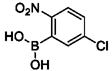 | 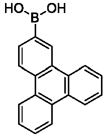 | 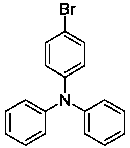 | 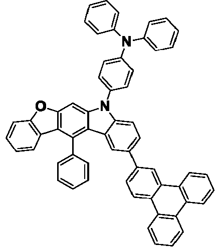 | 76% |
| 37 4 | 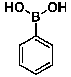 | 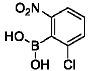 | 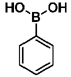 | 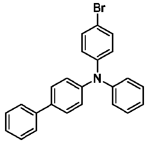 | 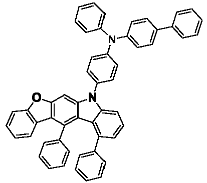 | 67% |

### Preparation Example 3. Preparation of Compound 361

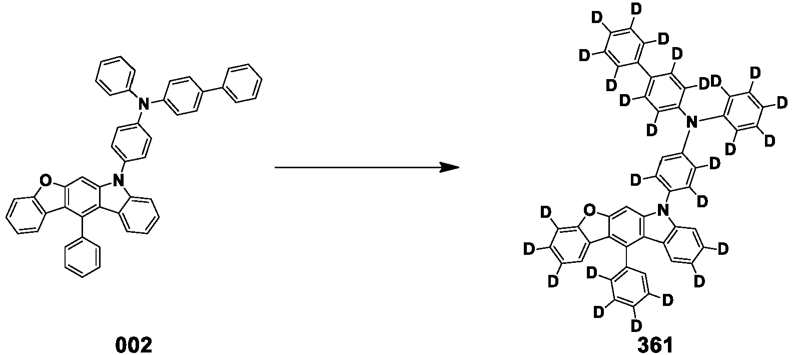

Compound 002 (9 g, 13.79 mmol), trifluoromethanesulfonic acid (3.10 g, 20.68 mmol) and D₆-benzene (100 mL) were introduced to a reaction flask, and then the mixture was stirred under reflux for 5 hours.
After the reaction was completed, distilled water was slowly added to the reaction solution to terminate the reaction, and then the result was extracted with dichloromethane and distilled water. The organic layer was dried with anhydrous MgSO₄, and then the solvent was removed using a rotary evaporator. After that, the result was purified by column chromatography using dichloromethane and hexane as a developing solvent to obtain Compound 361 (8 g, yield 85%).

Synthesis results for the compounds described in Preparation Examples 1 to 3 and Tables 1 and 2 are shown in the following Tables 3 and 4.

The following Table 3 shows measurement values of ¹H NMR (CDCl₃, 300 MHz), and the following Table 4 shows measurement values of FD-mass spectrometry (FD-MS: field desorption mass spectrometry).

**[Table 3]**

| Compound | ¹H NMR (CDCl₃, 300 MHz) |
|---|---|
| 002 | δ=8.55 (1H, d), 7.94 (1H, d), 7.89 (1H, d), 7.66 (1H, d), 7.54-7.20 (21H, m), 6.81 (1H, t), 6.69 (2H, d), 6.63 (2H, d) |
| 003 | δ=8.55 (1H, d), 7.94 (1H, d), 7.89 (1H, d), 7.66 (1H, d), 7.54-7.25 (26H, m), 6.69 (4H, d), 6.63 (2H, d) |
| 006 | δ=8.55 (1H, d), 7.94 (1H, d), 7.89 (1H, d), 7.87 (1H, d), 7.66-7.20 (19H, m), 6.81 (1H, t), 6.75 (1H, s), 6.63 (4H, d), 6.58 (1H, d), 1.72 (6H, s) |
| 008 | δ=8.55 (1H, d), 7.94 (1H, d), 7.89-7.74 (6H, m), 7.66 (1H, d), 7.54-7.25 (22H, m), 6.69 (2H, d), 6.63 (2H, d) |
| 009 | δ=8.93 (2H, d), 8.55 (1H, d), 8.13 (1H, s), 8.12 (2H, d), 7.94-7.82 (7H, m), 7.66 (1H, d), 7.51-7.20 (14H, m), 7.02 (1H, d), 6.81 (1H, t), 6.63 (4H, d) |
| 011 | δ=8.55 (1H, d), 7.94 (1H, d), 7.89 (1H, d), 7.79 (2H, d), 7.68 (2H, d), 7.66 (1H, d), 7.54-7.20 (16H, m), 6.81 (2H, t), 6.69 (2H, d), 6.63 (4H, d) |
| 013 | δ=8.55 (1H, d), 8.45 (1H, d), 7.98 (1H, d), 7.94 (1H, d), 7.89 (1H, d), 7.73 (1H, d), 7.66 (1H, d), 7.52-7.20 (17H, m), 6.86 (1H, d), 6.81 (1H, t), 6.63 (4H, d) |
| 016 | δ=8.55 (1H, d), 8.45 (1H, d), 7.98 (1H, d), 7.94 (1H, d), 7.89 (1H, d), 7.73 (1H, d), 7.66 (1H, d), 7.52-7.20 (16H, m), 6.86 (2H, d), 6.81 (1H, t), 6.64-6.63 (4H, m) |
| 018 | δ=8.55 (1H, d), 7.94 (1H, d), 7.89 (2H, d), 7.66 (2H, d), 7.65 (1H, s), 7.52-7.20 (17H, m), 6.81 (1H, t), 6.63 (4H, d), 6.39 (1H, d) |
| 022 | δ=8.55 (2H, d), 8.42 (1H, d), 8.08 (1H, d), 8.04 (1H, d), 7.94 (1H, d), 7.89 (1H, d), 7.66-7.20 (20H, m), 6.81 (1H, t), 6.69 (2H, d), 6.63 (4H, d) |
| 032 | δ=8.55 (2H, d), 8.45 (1H, d), 8.42 (1H, d), 8.08 (1H, d), 8.04 (1H, d), 7.98 (1H, d), 7.94 (1H, d), 7.89 (1H, d), 7.80 (1H, d), 7.66-7.50 (6H, m), 7.38-7.20 (9H, m), 7.06 (1H, s), 6.88 (1H, d), 6.81 (1H, t), 6.63 (4H, d) |
| 040 | δ=8.55 (2H, d), 8.42 (1H, d), 8.08 (1H, d), 8.04 (1H, d), 7.94-7.74 (6H, m), 7.66-7.49 (6H, m), 7.38-7.20 (10H, m), 6.81 (1H, t), 6.63 (4H, d) |
| 053 | δ=8.55 (1H, d), 8.45 (1H, d), 8.00-7.89 (6H, m), 7.73-7.50 (8H, m), 7.40-7.20 (10H, m), 6.86 (1H, d), 6.81 (1H, t), 6.63 (4H, d) |
| 056 | δ=8.55 (1H, d), 8.45 (1H, d), 8.00-7.89 (6H, m), 7.73-7.50 (8H, m), 7.40-7.20 (9H, m), 6.86 (2H, d), 6.81 (1H, t), 6.64-6.63 (4H, m) |
| 066 | δ=8.55 (1H, d), 7.94 (1H, d), 7.89 (1H, d), 7.87 (1H, d), 7.66-7.20 (23H, m), 6.81 (1H, t), 6.75 (1H, s), 6.63 (4H, d), 6.58 (1H, d), 1.72 (6H, s) |
| 071 | δ=8.55 (1H, d), 7.94 (1H, d), 7.89 (1H, d), 7.79 (2H, d), 7.68 (2H, d), 7.66 (1H, d), 7.54-7.20 (20H, m), 6.81 (2H, t), 6.69 (2H, d), 6.63 (4H, d) |
| 082 | δ=8.55 (1H, d), 7.94 (1H, d), 7.93 (1H, d), 7.89 (1H, d), 7.87 (1H, d), 7.77 (1H, s), 7.66 (1H, d), 7.63 (1H, d), 7.55-7.20 (19H, m), 6.81 (1H, t), 6.69 (2H, d), 6.63 (4H, d), 1.72 (6H, s) |
| 086 | δ=8.55 (1H, d), 7.94-7.87 (5H, m), 7.77 (1H, s), 7.66 (1H, d), 7.63 (1H, d), 7.62 (1H, d), 7.55 (1H, d), 7.38-7.20 (13H, m), 6.81 (1H, t), 6.75 (1H, s), 6.63 (4H, d), 6.58 (1H, d), 1.72 (12H, s) |
| 092 | δ=8.55 (1H, d), 8.45 (1H, d), 7.98-7.77 (7H, m), 7.66 (1H, d), 7.63 (1H, d), 7.55 (1H, d), 7.52 (1H, t), 7.50 (1H, t), 7.38-7.20 (11H, m), 7.06 (1H, s), 6.88 (1H, d), 6.81 (1H, t), 6.63 (4H, d), 1.72 (6H, s) |
| 110 | δ=8.93 (2H, d), 8.55 (1H, d), 8.45 (1H, d), 8.12 (2H, d), 8.00-7.82 (10H, m), 7.66 (1H, d), 7.52 (1H, t), 7.50 (1H, t), 7.38-7.20 (9H, m), 6.91 (1H, s), 6.81 (1H, t), 6.63 (4H, d) |
| 122 | δ=8.55 (1H, d), 8.45 (1H, d), 8.41 (1H, d), 8.20 (1H, d), 7.98 (1H, d), 7.94 (1H, d), 7.89 (1H, d), 7.66-7.20 (20H, m), 6.81 (1H, t), 6.69 (2H, d), 6.63 (4H, d) |
| 130 | δ=8.93 (2H, d), 8.55 (1H, d), 8.45 (1H, d), 8.41 (1H, d), 8.20 (1H, d), 8.12 (2H, d), 7.98-7.82 (7H, m), 7.66 (1H, d), 7.58 (1H, t), 7.52 (1H, t), 7.50 (1H, t), 7.37-7.20 (9H, m), 6.91 (1H, s), 6.81 (1H, t), 6.63 (4H, d) |
| 141 | δ=8.55 (1H, d), 7.95 (1H, d), 7.94 (1H, d), 7.89 (2H, d), 7.75 (1H, d), 7.66 (2H, d), 7.64 (1H, s), 7.38-7.20 (13H, m), 6.81 (2H, t), 6.63 (6H, d) |
| 161 | δ=8.55 (1H, d), 7.94 (1H, d), 7.89 (2H, d), 7.85 (1H, d), 7.81 (1H, d), 7.66 (2H, d), 7.38-7.20 (14H, m), 6.81 (2H, t), 6.63 (6H, d) |
| 174 | δ=8.55 (1H, d), 8.45 (1H, d), 7.98-7.81 (7H, m), 7.66 (2H, d), 7.52 (1H, t), 7.50 (1H, t), 7.38-7.20 (13H, m), 6.86 (1H, d), 6.81 (1H, t), 6.63 (4H, d) |
| 190 | δ=8.93 (4H, d), 8.55 (1H, d), 8.12 (2H, d), 7.94-7.82 (11H, m), 7.66 (1H, d), 7.38-7.20 (9H, m), 6.91 (1H, s), 6.81 (1H, t), 6.63 (4H, d) |
| 201 | δ=9.15 (1H, s), 8.93 (2H, d), 8.55 (1H, d), 8.18 (1H, d), 8.12 (2H, d), 8.04 (1H, d), 7.94-7.82 (6H, m), 7.66 (1H, d), 7.38-7.20 (11H, m), 6.81 (2H, t), 6.63 (6H, d) |
| 202 | δ=9.15 (1H, s), 8.93 (2H, d), 8.55 (1H, d), 8.18 (1H, d), 8.12 (2H, d), 8.04 (1H, d), 7.94-7.82 (6H, m), 7.66 (1H, d), 7.54-7.20 (16H, m), 6.81 (1H, t), 6.69 (2H, d), 6.63 (4H, d) |
| 231 | δ=8.49 (1H, d), 8.10 (1H, d), 7.89 (1H, d), 7.66 (1H, d), 7.62 (1H, s), 7.52-7.32 (15H, m), 7.20 (4H, t), 6.81 (2H, t), 6.63 (6H, d) |
| 233 | δ=8.49 (1H, d), 8.10 (1H, d), 7.89 (1H, d), 7.66 (1H, d), 7.62 (1H, s), 7.54-7.32 (22H, m), 7.20 (2H, t), 6.81 (1H, t), 6.69 (2H, d), 6.63 (4H, d) |
| 243 | δ=8.18 (1H, d), 8.00 (1H, d), 7.89 (1H, d), 7.77 (1H, s), 7.66 (1H, d), 7.54-7.32 (22H, m), 7.20 (2H, t), 6.81 (1H, t), 6.69 (2H, d), 6.63 (4H, d) |
| 250 | δ=8.18 (1H, d), 8.00 (1H, d), 7.89 (1H, d), 7.77 (1H, s), 7.66 (2H, d), 7.64 (1H, d), 7.52-7.32 (18H, m), 7.20 (2H, t), 6.81 (1H, t), 6.63 (4H, d), 6.33 (1H, d) |
| 265 | δ=8.55 (1H, d), 8.49 (1H, d), 8.42 (1H, d), 8.10 (1H, d), 8.08 (1H, d), 8.04 (1H, d), 7.89 (1H, d), 7.87 (1H, d), 7.66-7.20 (21H, m), 6.81 (1H, t), 6.75 (1H, s), 6.63 (4H, d), 6.58 (1H, d), 1.72 (6H, s) |
| 273 | δ=8.18 (1H, d), 8.00 (3H, d), 7.92 (1H, d), 7.89 (1H, d), 7.77 (1H, s), 7.73 (1H, d), 7.66-7.32 (21H, m), 7.20 (2H, t), 6.81 (1H, t), 6.69 (2H, d), 6.63 (4H, d) |
| 282 | δ=8.55 (1H, d), 8.42 (1H, d), 8.18 (1H, d), 8.08 (1H, d), 8.04 (1H, d), 8.00 (1H, d), 7.89 (1H, d), 7.77 (1H, s), 7.66-7.32 (12H, m), 7.20 (5H, t), 6.86 (1H, d), 6.81 (2H, t), 6.64 (1H, s), 6.63 (5H, d) |
| 301 | δ=8.49 (1H, d), 8.45 (1H, d), 8.10 (1H, d), 8.00 (2H, d), 7.98 (1H, d), 7.89 (1H, d), 7.86 (1H, d), 7.66 (1H, d), 7.62 (1H, s), 7.52-7.32 (12H, m), 7.20 (4H, t), 6.81 (2H, t), 6.63 (6H, d) |
| 312 | δ=8.18 (1H, d), 8.00 (1H, d), 7.95 (1H, d), 7.89 (2H, d), 7.77 (1H, s), 7.75 (1H, d), 7.66 (2H, d), 7.64 (1H, s), 7.52-7.32 (10H, m), 7.20 (5H, t), 6.86 (1H, d), 6.81 (2H, t), 6.64 (1H, s), 6.63 (5H, d) |
| 346 | δ=8.93 (4H, d), 8.49 (1H, d), 8.12 (4H, d), 8.10 (1H, d), 7.93-7.82 (10H, m), 7.66 (1H, d), 7.62 (1H, s), 7.52-7.32 (10H, m), 7.20 (2H, t), 6.91 (1H, s), 6.81 (1H, t), 6.63 (4H, d) |
| 351 | δ=9.15 (1H, s), 8.93 (2H, d), 8.18 (2H, d), 8.12 (2H, d), 8.04 (1H, d), 8.00 (1H, d), 7.89-7.77 (6H, m), 7.66 (1H, d), 7.51-7.32 (10H, m), 7.20 (4H, t), 6.81 (2H, t), 6.63 (6H, d) |
| 361 | δ=7.98 (1H, s), 7.83 (1H, s), 7.80 (1H, s), 7.66 (1H, s), 7.38 (1H, s), 6.64 (1H, s) |
| 367 | δ=8.55 (1H, d), 7.94 (1H, d), 7.89 (1H, d), 7.66 (1H, d), 7.52-7.25 (10H, m) |
| 374 | δ=8.10 (1H, d), 7.90 (1H, d), 7.89 (1H, d), 7.79 (2H, d), 7.66 (1H, d), 7.52-7.32 (21H, m), 7.20 (2H, t), 6.81 (1H, t), 6.69 (2H, d), 6.63 (4H, d) |

**[Table 4]**

| Comp ound | FD-MS | Comp ound | FD-MS |
|---|---|---|---|
| 002 | m/z=652.78 (C₄₈H₃₂N₂O=652.25) | 130 | m/z=782.95 (C₅₆H₃₄N₂OS=782.24) |
| 003 | m/z=728.88 (C₅₄H₃₆N₂O=728.28) | 141 | m/z=666.76 (C₄₈H₃₀N₂O₂=666.23) |
| 006 | m/z=692.84 (C₅₁H₃₆N₂O=692.28) | 161 | m/z=666.76 (C₄₈H₃₀N₂O₂=666.23) |
| 008 | m/z=702.84 (C₅₂H₃₄N₂O=702.27) | 174 | m/z=818.96 (C₆₀H₃₈N₂O₂=818.29) |
| 009 | m/z=726.86 (C₅₄H₃₄N₂O=726.27) | 190 | m/z=776.92 (C₅₈H₃₆N_{2O}=776.28) |
| 011 | m/z=652.78 (C₄₈H₃₂N₂O=652.25 | 201 | m/z=726.86 (C₅₄H₃₄N₂O=726.27) |
| 013 | m/z=682.83 (C₄₈H₃₀N₂OS=682.21) | 202 | m/z=802.96 (C₆₀H₃₈N₂O=802.30) |
| 016 | m/z=682.83 (C₄₈H₃₀N₂OS=682.21) | 231 | m/z=652.78 (C₄₈H₃₂N₂O=652.25) |
| 018 | m/z=666.76 (C₄₈H₃₀N₂O₂=666.23) | 233 | m/z=728.88 (C₅₄H₃₆N₂O=728.28) |
| 022 | m/z=702.84 (C₅₂H₃₄N₂O=702.27) | 243 | m/z=728.88 (C₅₄H₃₆N₂O=728.28) |
| 032 | m/z=732.89 (C₅₂H₃₂N₂OS=732.22) | 250 | m/z=742.86 (C₅₄H₃₄N₂O₂=742.26) |
| 040 | m/z=676.80 (C₅₀H₃₂N₂O=676.25) | 265 | m/z=819.00 (C₆₁H₄₂N₂O=818.33) |
| 053 | m/z=732.89 (C₅₂H₃₂N₂OS=732.22) | 273 | m/z=778.94 (C₅₈H₃₈N₂₀=778.30) |
| 056 | m/z=732.89 (C₅₂H₃₂N₂OS=732.22) | 282 | m/z=702.84 (C₅₂H₃₄N₂O=702.27) |
| 066 | m/z=768.94 (C₅₇H₄₀N₂O=768.31) | 301 | m/z=758.93 (C₅₄H₃₄N₂OS=758.24) |
| 071 | m/z=728.88 (C₅₄H₃₆N₂O=728.28) | 312 | m/z=742.86 (C₅₄H₃₄N₂O₂=742.26) |
| 082 | m/z=768.94 (C₅₇H₄₀N₂O=768.31) | 346 | m/z=853.02 (C₆₄H₄₀N₂₀=852.31) |
| 086 | m/z=809.00 (C₆₀H₄₄N₂O=808.35) | 351 | m/z=802.96 (C₆₀H₃₈N₂O=802.30) |
| 092 | m/z=798.99 (C₆₇H₃₈N₂OS=798.27) | 361 | m/z=678.94 (C₄₈H₆D₂₆N₂O=678.41) |
| 110 | m/z=782.95 (C₅₆H₃₄N₂OS=782.24) | 367 | m/z=698.93 (C₄₈H₁₄D₁₆N₂OS=698.31) |
| 122 | m/z=758.93 (C₅₄H₃₄N₂OS=758.24) | 374 | m/z=728.88 (C₅₄H₃₆N₂O=728.28) |

### <Experimental Example>

### Experimental Example 1.

### Experimental Example 1-1. Manufacture of Organic Light Emitting Device

A glass substrate coated with ITO as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and then subjected to UVO (ultraviolet ozone) treatment for 5 minutes using UV (ultraviolet) in a UV cleaner. After that, the substrate was transferred to a plasma cleaner (PT), and after subjected to plasma treatment under vacuum for ITO work function increase and residual film removal, the substrate was transferred to a thermal deposition apparatus for organic deposition.

To a cell in the vacuum deposition apparatus, 4,4',4"-tris(N,N-(2-naphthyl)-phenylamino)triphenylamine (2-TNATA) was introduced.

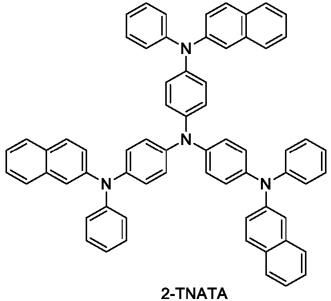

Subsequently, the chamber was evacuated until the degree of vacuum therein reached 10⁻⁶ torr, and then the 2-TNATA was evaporated by applying a current to the cell to deposit a hole injection layer having a thickness of 600 Å on the ITO substrate. To another cell in the vacuum deposition apparatus, a compound represented by Chemical Formula 1 or a comparative compound described in the following Table 5 was introduced, and evaporated by applying a current to the cell to deposit a hole transport layer having a thickness of 1000 Å on the hole injection layer.

After forming the hole injection layer and the hole transport layer as above, a light emitting layer was thermal vacuum deposited thereon as follows. As the light emitting layer, 9-[4-(4,6-diphenyl-1,3,5-triazin-2-yl)phenyl]-9'-phenyl-3,3'-bi-9H-carbazole was deposited to a thickness of 400 Å as a host, and, as a green phosphorescent dopant, Ir(ppy)₃ was doped thereto by 7% and deposited. After that, BCP was deposited to 60 Å as a hole blocking layer, and E1 was deposited to 300 Å thereon as an electron transport layer.

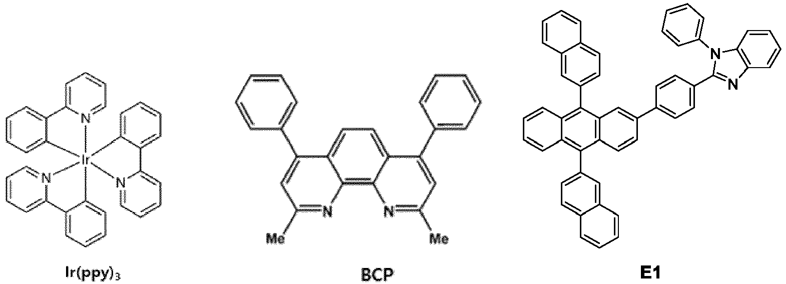

After that, lithium fluoride (LiF) was deposited to a thickness of 10 Å as an electron injection layer, and a negative electrode was formed by depositing Al to a thickness of 1200 Å, and as a result, an organic light emitting device was manufactured.

Meanwhile, all the organic compounds required to manufacture the organic light emitting device were vacuum sublimation purified under 10⁻⁸ torr to 10⁻⁶ torr for each material to be used in the organic light emitting device manufacture.

Herein, the comparative compounds used as the hole transport layer are as follows.

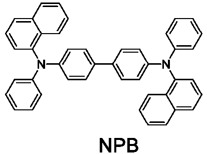

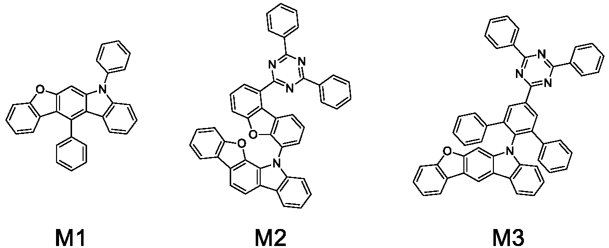

### Experimental Example 1-2. Driving Voltage and Light Emission Efficiency of Organic Light Emitting Device

For each of the organic electroluminescent devices manufactured as above, electroluminescent (EL) properties were measured using M7000 manufactured by McScience Inc., and with the measurement results, a lifetime T₉₅, a time taken for luminance to become 95% with respect to initial luminance, was measured when standard luminance was 20,000 cd/m² through a lifetime measurement system (M6000) manufactured by McScience Inc.

Results of measuring driving voltage, light emission efficiency and lifetime (T₉₅) of the green organic light emitting devices manufactured according to the manufacturing method are as shown in Table 5.

**[Table 5]**

| | Compound | Driving Voltage (V) | Light Emission Efficiency (cd/A) | Lifetime (T₉₅) |
|---|---|---|---|---|
| Example 1 | 002 | 4.33 | 116.70 | 128 |
| Example 2 | 003 | 4.39 | 115.88 | 131 |
| Example 3 | 006 | 4.42 | 118.42 | 139 |
| Example 4 | 008 | 4.36 | 112.19 | 137 |
| Example 5 | 009 | 4.12 | 109.53 | 124 |
| Example 6 | 011 | 4.29 | 112.77 | 136 |
| Example 7 | 013 | 4.46 | 117.08 | 129 |
| Example 8 | 016 | 4.36 | 115.47 | 125 |
| Example 9 | 018 | 4.42 | 120.65 | 134 |
| Example 10 | 022 | 4.15 | 113.52 | 130 |
| Example 11 | 032 | 4.23 | 114.77 | 126 |
| Example 12 | 040 | 4.30 | 119.25 | 131 |
| Example 13 | 053 | 4.37 | 116.49 | 134 |
| Example 14 | 056 | 4.37 | 115.56 | 128 |
| Example 15 | 066 | 4.26 | 114.85 | 123 |
| Example 16 | 071 | 4.30 | 118.73 | 134 |
| Example 17 | 082 | 4.07 | 116.42 | 128 |
| Example 18 | 086 | 4.11 | 111.44 | 131 |
| Example 19 | 092 | 4.34 | 115.91 | 135 |
| Example 20 | 110 | 4.18 | 114.78 | 135 |
| Example 21 | 122 | 4.29 | 117.16 | 121 |
| Example 22 | 130 | 4.37 | 115.09 | 132 |
| Example 23 | 141 | 4.36 | 116.15 | 127 |
| Example 24 | 161 | 4.29 | 119.66 | 131 |
| Example 25 | 174 | 4.20 | 115.76 | 132 |
| Example 26 | 190 | 4.33 | 116.29 | 127 |
| Example 27 | 201 | 4.24 | 117.69 | 135 |
| Example 28 | 202 | 4.25 | 114.57 | 127 |
| Example 29 | 231 | 4.21 | 115.61 | 138 |
| Example 30 | 233 | 4.19 | 113.70 | 134 |
| Example 31 | 243 | 4.11 | 112.54 | 135 |
| Example 32 | 250 | 4.30 | 115.92 | 137 |
| Example 33 | 265 | 4.21 | 118.47 | 131 |
| Example 34 | 273 | 4.34 | 118.08 | 126 |
| Example 35 | 282 | 4.36 | 113.78 | 125 |
| Example 36 | 301 | 4.28 | 117.54 | 131 |
| Example 37 | 312 | 4.17 | 113.21 | 137 |
| Example 38 | 346 | 4.31 | 115.24 | 130 |
| Example 39 | 351 | 4.22 | 113.37 | 133 |
| Example 40 | 361 | 4.35 | 115.98 | 164 |
| Example 41 | 367 | 4.42 | 116.87 | 157 |
| Example 42 | 374 | 4.32 | 115.09 | 124 |
| Comparative Example 1 | NPB | 5.53 | 88.42 | 95 |
| Comparative Example 2 | M1 | 6.86 | 64.72 | 76 |
| Comparative Example 3 | M2 | 8.62 | 52.75 | 68 |
| Comparative Example 4 | M3 | 8.21 | 56.29 | 61 |

From the results of Table 5, it was seen that Examples 1 to 42, organic light emitting devices using the heterocyclic compound represented by Chemical Formula 1 of the present disclosure as a hole transport layer material, exhibited results of lowered driving voltage and significantly improved light emission efficiency and lifetime compared to Comparative Examples 1 to 4, organic light emitting devices not using the heterocyclic compound represented by Chemical Formula 1 of the present disclosure as a hole transport layer material.

The compounds of M1 to M3 used in Comparative Examples 2 to 4 are similar to the compound of the present disclosure in having a benzofurocarbazole-type five-ring skeleton, but are different from the compound of the present disclosure in the introduced substituents. The heterocyclic compound of the present disclosure has properties of high hole mobility by introducing an arylamine group as a substituent and has properties of a proper HOMO level. For such a reason, it can be seen that holes are more readily transported to a light emitting layer when using the heterocyclic compound of the present disclosure as a hole transport layer compared to when using M1 to M3 of Comparative Examples 2 to 4, thereby lowering a driving voltage, and improving light emission efficiency and lifetime.

### Experimental Example 2.

### Experimental Example 2-1. Manufacture of Organic Light Emitting Device

A transparent electrode ITO thin film obtained from glass for an OLED (manufactured by Samsung-Corning) was ultrasonic cleaned using trichloroethylene, acetone, ethanol and distilled water sequentially for 5 minutes each, and then stored in isopropanol before use.

To a cell in the vacuum deposition apparatus, 4,4',4"-tris(N,N-(2-naphthyl)-phenylamino)triphenylamine (2-TNATA) was introduced.

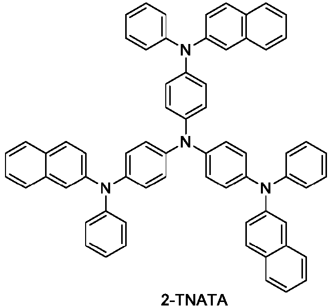

Subsequently, the chamber was evacuated until the degree of vacuum therein reached 10⁻⁶ torr, and then the 2-TNATA was evaporated by applying a current to the cell to deposit a hole injection layer having a thickness of 600 Å on the ITO substrate. To another cell in the vacuum deposition apparatus, N,N'-bis(α-naphthyl)-N,N'-diphenyl-4,4'-diamine (NPB) was introduced, and evaporated by applying a current to the cell to deposit a hole transport layer having a thickness of 1000 Å on the hole injection layer.

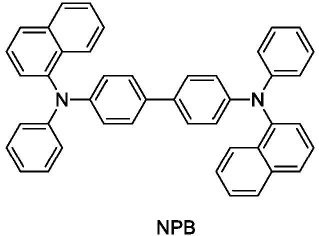

Subsequently, a compound represented by Chemical Formula 1 or a comparative compound described in Table 6 was deposited to a thickness of 1000 Å as an electron blocking layer.

A blue light emitting material with the following structure was deposited thereon as a light emitting layer. Specifically, on one cell in the vacuum deposition apparatus, H1, a blue light emitting host material, was vacuum deposited to a thickness of 300 Å, and D1, a blue light emitting dopant material, was vacuum deposited thereon by 5% with respect to the host material.

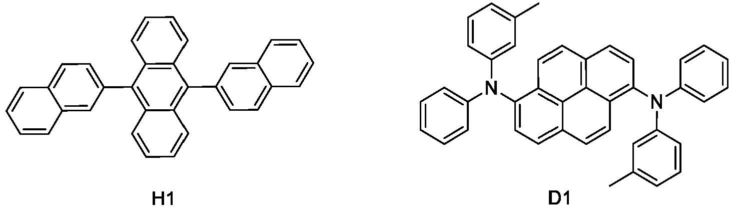

After that, a compound with the following Structural Formula E1 was deposited to a thickness of 300 Å as an electron transport layer.

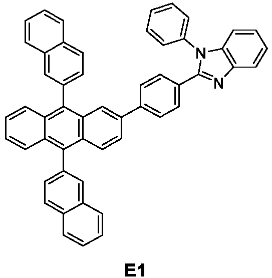

After that, lithium fluoride (LiF) was deposited to a thickness of 10 Å as an electron injection layer, and a negative electrode was formed by depositing Al to a thickness of 1000 Å, and as a result, an organic light emitting device was manufactured.

Meanwhile, all the organic compounds required to manufacture the organic light emitting device were vacuum sublimation purified under 10⁻⁸ torr to 10⁻⁶ torr for each material to be used in the organic light emitting device manufacture.

Herein, the comparative compounds used as the electron blocking layer are as follows.

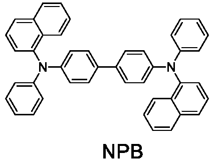

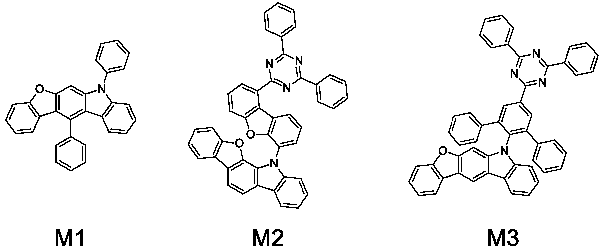

### Experimental Example 2-2. Driving Voltage and Light Emission Efficiency of Organic Light Emitting Device

For each of the organic electroluminescent devices manufactured as above, electroluminescent (EL) properties were measured using M7000 manufactured by McScience Inc., and with the measurement results, a lifetime T₉₅, a time taken for luminance to become 95% with respect to initial luminance, was measured when standard luminance was 20,000 cd/m² through a lifetime measurement system (M6000) manufactured by McScience Inc.

Results of measuring driving voltage, light emission efficiency and lifetime (T₉₅) of the blue organic light emitting devices manufactured according to the manufacturing method are as shown in Table 6.

**[Table 6]**

| | Compound | Driving Voltage (V) | Light Emission Efficiency (cd/A) | Lifetime (T₉₅) |
|---|---|---|---|---|
| Example 43 | 002 | 5.34 | 6.86 | 66 |
| Example 44 | 006 | 5.42 | 7.28 | 64 |
| Example 45 | 011 | 5.51 | 6.91 | 70 |
| Example 46 | 013 | 5.29 | 7.21 | 62 |
| Example 47 | 018 | 5.33 | 6.93 | 68 |
| Example 48 | 071 | 5.28 | 6.74 | 64 |
| Example 49 | 110 | 5.27 | 7.05 | 65 |
| Example 50 | 130 | 5.45 | 6.90 | 74 |
| Example 51 | 141 | 5.31 | 6.69 | 71 |
| Example 52 | 190 | 5.25 | 6.80 | 69 |
| Example 53 | 233 | 5.28 | 6.96 | 67 |
| Example 54 | 243 | 5.40 | 6.83 | 68 |
| Example 55 | 273 | 5.32 | 7.15 | 72 |
| Example 56 | 346 | 5.24 | 7.17 | 66 |
| Example 57 | 361 | 5.37 | 6.76 | 97 |
| Example 58 | 367 | 5.31 | 7.09 | 88 |
| Comparative Example 5 | M1 | 6.81 | 4.74 | 32 |
| Comparative Example 6 | M2 | 8.14 | 3.96 | 36 |
| Comparative Example 7 | M3 | 8.28 | 4.29 | 34 |
| Comparative Example 8 | NPB | 6.32 | 5.99 | 46 |

From the results of Table 6, it was seen that Examples 43 to 58, organic light emitting devices using the heterocyclic compound represented by Chemical Formula 1 of the present disclosure as an electron blocking layer material, exhibited results of lowered driving voltage and significantly improved light emission efficiency and lifetime compared to Comparative Examples 5 to 8, organic light emitting devices not using the heterocyclic compound represented by Chemical Formula 1 of the present disclosure as an electron blocking layer material.

Generally, a phenomenon of reducing efficiency and lifetime of an organic light emitting device occurs when electrons migrate to a positive electrode after passing through a hole transport layer without binding in a light emitting layer. Herein, when a compound having a high LUMO (lowest unoccupied molecular orbital) level is used as an electron blocking layer, electrons to migrate to the positive electrode after passing through the light emitting layer are blocked by an energy barrier of the electron blocking layer, which may prevent the phenomenon of reducing efficiency and lifetime of an organic light emitting device. In other words, when a compound having a high LUMO (lowest unoccupied molecular orbital) level is used as an electron blocking layer, probability of holes and electrons forming excitons increases and probability of being emitted as light in the light emitting layer increases.

Accordingly, it can be seen that, by the heterocyclic compound of the present disclosure having a higher LUMO level compared to the compounds of Comparative Examples 5 to **8,** driving voltage, light emission efficiency and lifetime properties are all excellent when the heterocyclic compound of the present disclosure is used as an electron blocking layer of an organic light emitting device since the electron blocking ability is more superior and holes and electrons form a charge balance.

### [Reference Numeral]

- 100:: Substrate
- 200:: Positive Electrode
- 300:: Organic Material Layer
- 301:: Hole Injection Layer
- 302:: Hole Transport Layer
- 303:: Light Emitting Layer
- 304:: Hole Blocking Layer
- 305:: Electron Transport Layer
- 306:: Electron Injection Layer
- 400:: Negative Electrode

## Claims

1. A heterocyclic compound represented by the following Chemical Formula 1: 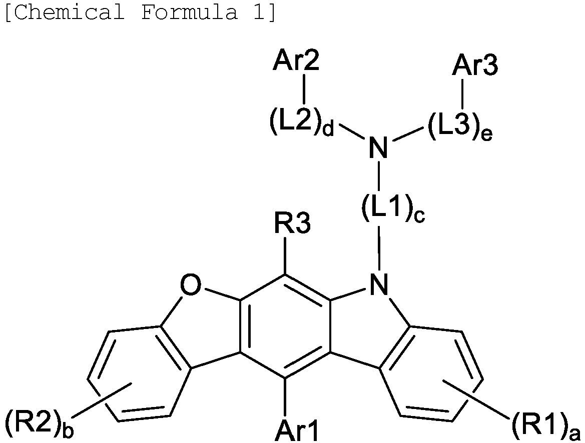 wherein, in Chemical Formula 1,
R1 to R3 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; -SiR101R102R103; and -NR101R102, or two or more groups adjacent to each other bond to each other to form a substituted or unsubstituted C6 to C60 aromatic hydrocarbon ring; or a substituted or unsubstituted C2 to C60 heteroring, and R101, R102 and R103 are the same as or different from each other and each independently a substituted or unsubstituted C1 to C60 alkyl group; a substituted or unsubstituted C6 to C60 aryl group; or a substituted or unsubstituted C2 to C60 heteroaryl group;
a is an integer of 0 to **4,** and when a is 2 or greater, R1s are the same as or different from each other;
b is an integer of 0 to **4,** and when b is 2 or greater, R2s are the same as or different from each other;
Ar1 to Ar3 are the same as or different from each other, and each independently a substituted or unsubstituted C6 to C60 aryl group; or a substituted or unsubstituted C2 to C60 heteroaryl group;
L1 is a substituted or unsubstituted C6 to C60 arylene group; or a substituted or unsubstituted C2 to C60 heteroarylene group;
c is an integer of 1 to 5, and when c is 2 or greater, L1s are the same as or different from each other;
L2 and L3 are the same as or different from each other, and each independently a direct bond; a substituted or unsubstituted C6 to C60 arylene group; or a substituted or unsubstituted C2 to C60 heteroarylene group;
d is an integer of 0 to 5, and when d is 2 or greater, L2s are the same as or different from each other; and
e is an integer of 0 to 5, and when e is 2 or greater, L3s are the same as or different from each other.

2. The heterocyclic compound of Claim 1, wherein R1 is hydrogen; deuterium; a substituted or unsubstituted C6 to C30 aryl group; or a substituted or unsubstituted C2 to C30 heteroaryl group.

3. The heterocyclic compound of Claim 1, wherein Ar1 to Ar3 are the same as or different from each other, and each independently a substituted or unsubstituted C6 to C30 aryl group; or a substituted or unsubstituted C2 to C30 heteroaryl group.

4. The heterocyclic compound of Claim 1, wherein L1 is a substituted or unsubstituted C6 to C30 arylene group; or a substituted or unsubstituted C2 to C30 heteroarylene group; and
L2 and L3 are the same as or different from each other, and each independently a direct bond; a substituted or unsubstituted C6 to C30 arylene group; or a substituted or unsubstituted C2 to C30 heteroarylene group.

5. The heterocyclic compound of Claim 1, wherein the heterocyclic compound represented by Chemical Formula 1 does not include deuterium as a substituent, or has a deuterium content of 1% to 100% with respect to a total number of hydrogen atoms and deuterium atoms.

6. The heterocyclic compound of Claim 1, wherein the heterocyclic compound represented by Chemical Formula 1 is represented by any one of the following compounds:

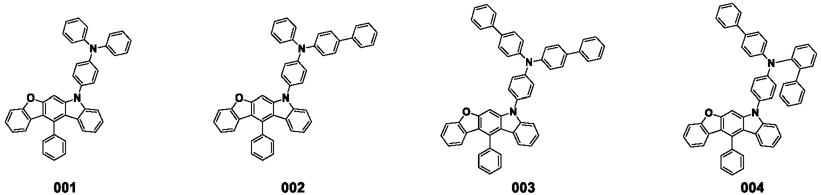

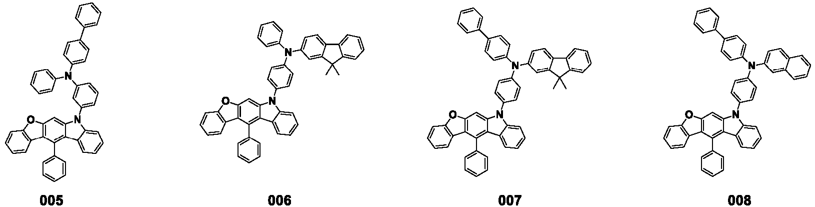

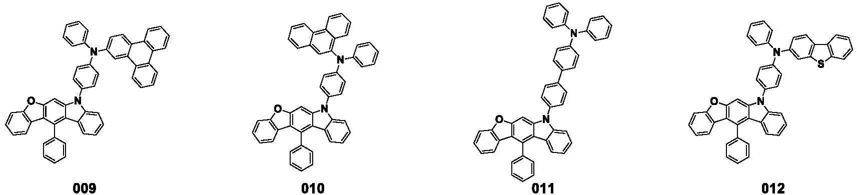

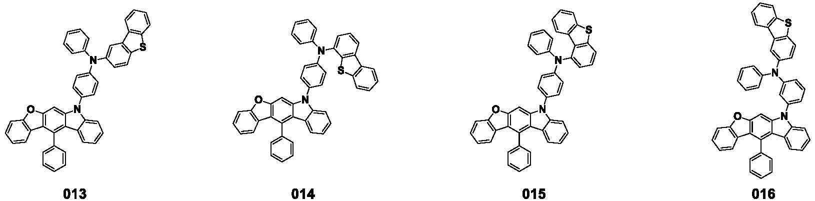

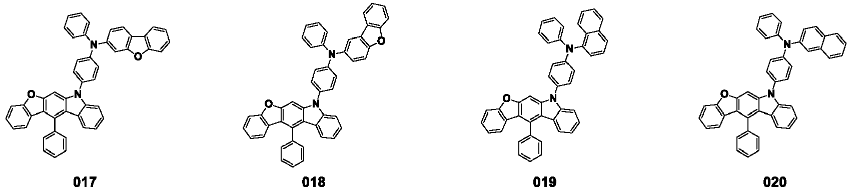

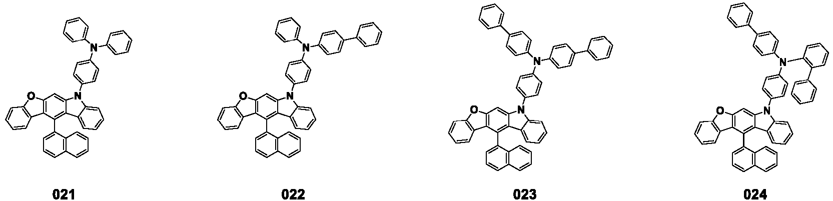

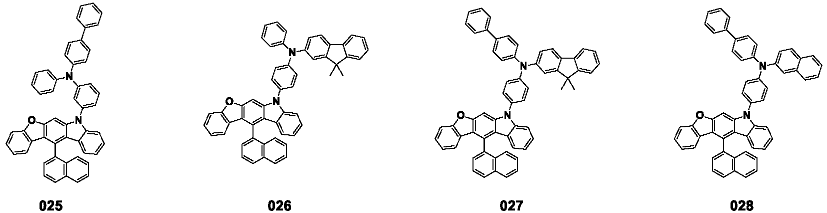

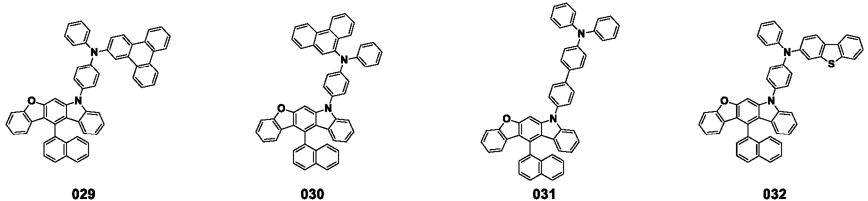

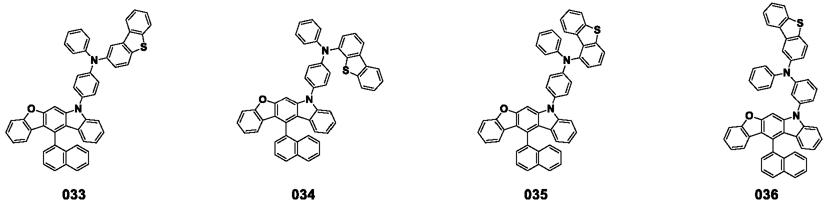

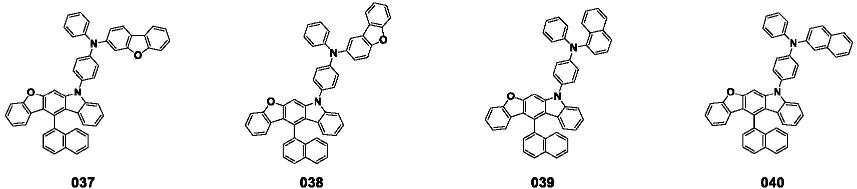

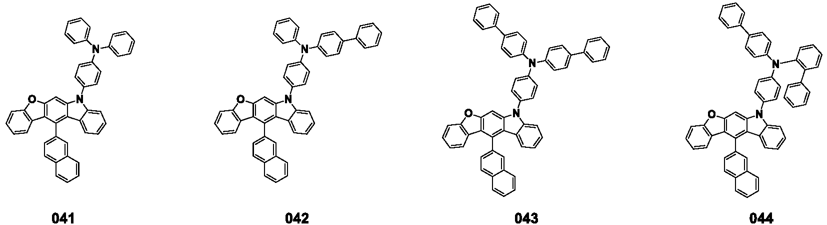

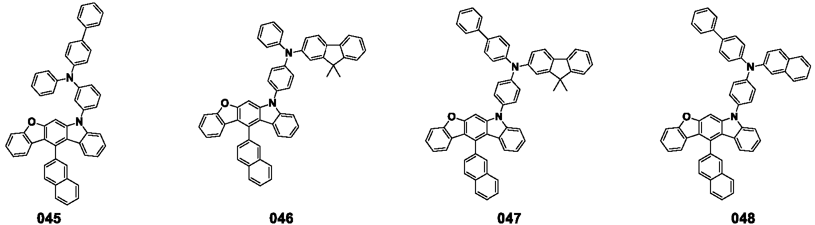

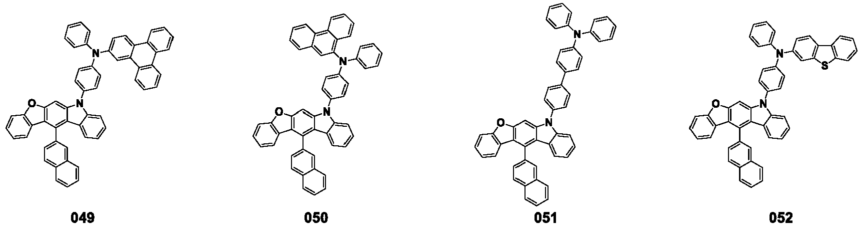

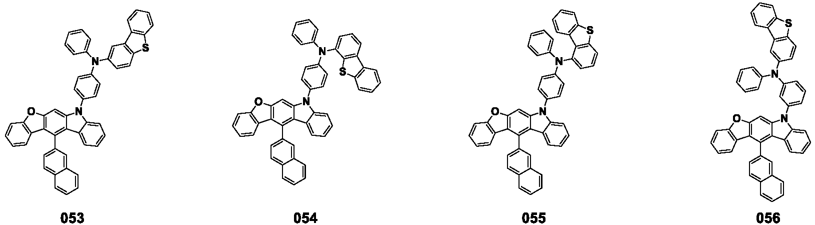

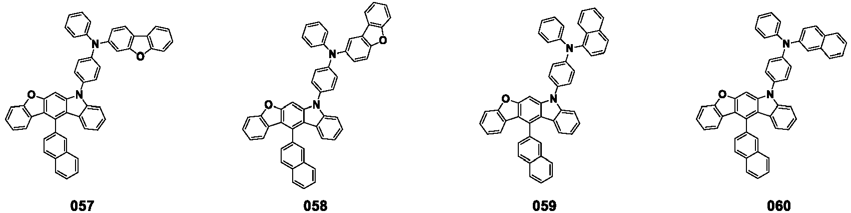

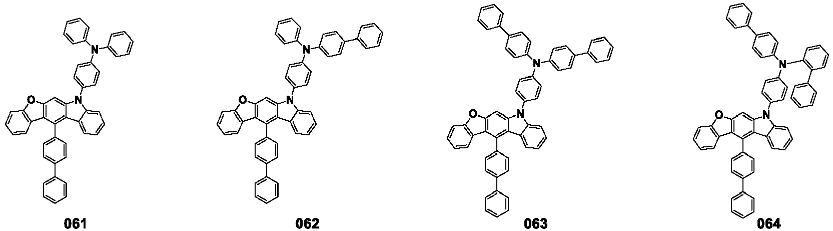

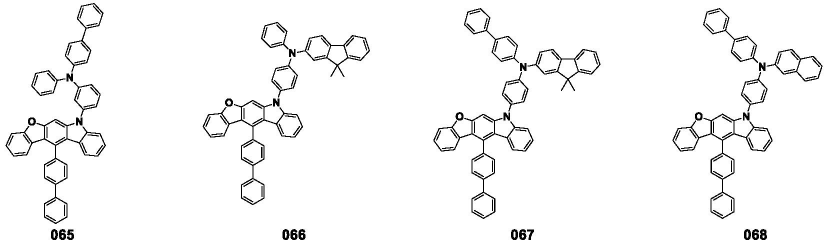

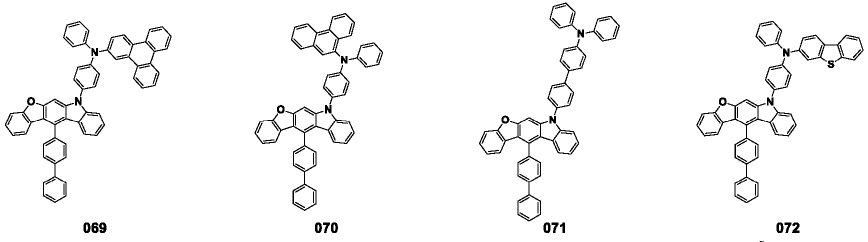

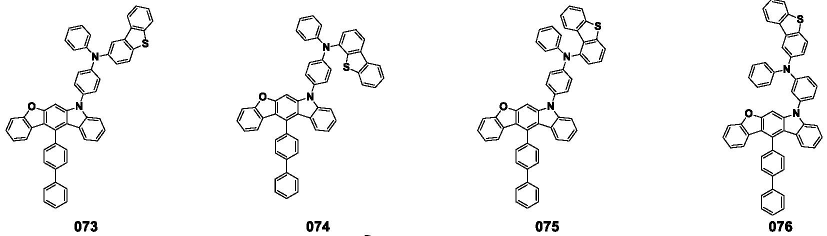

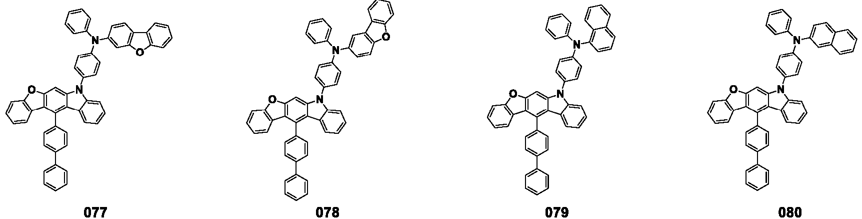

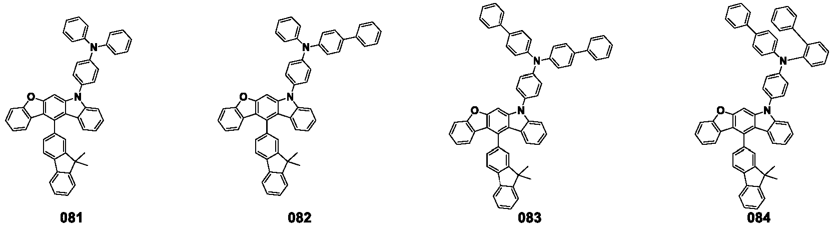

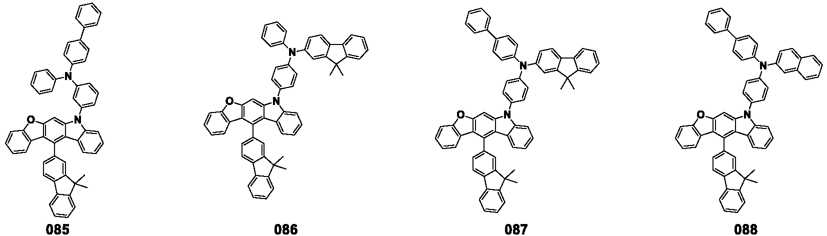

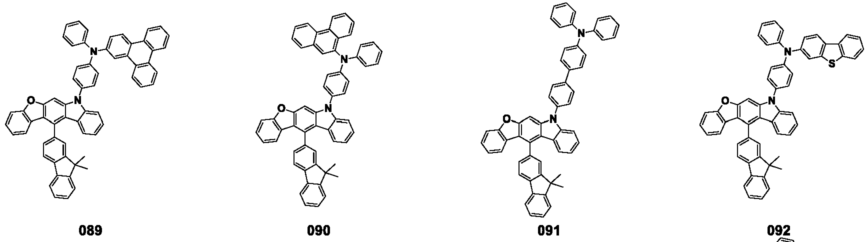

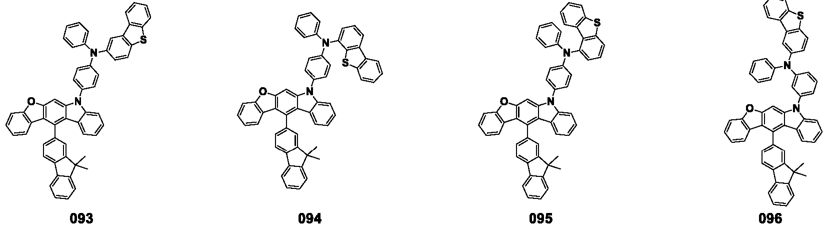

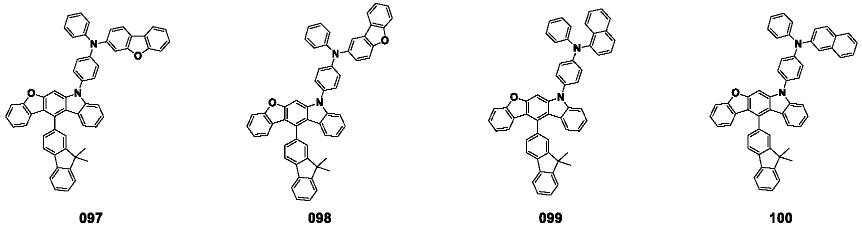

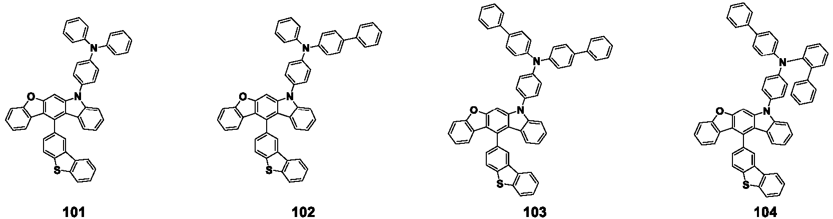

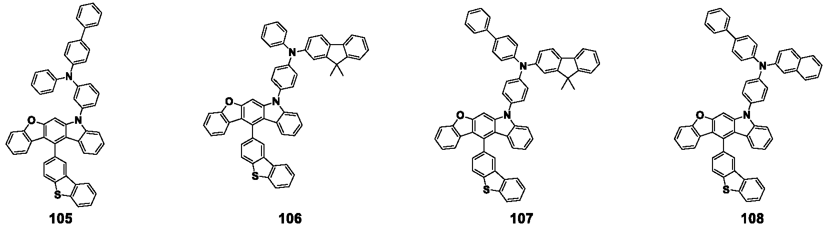

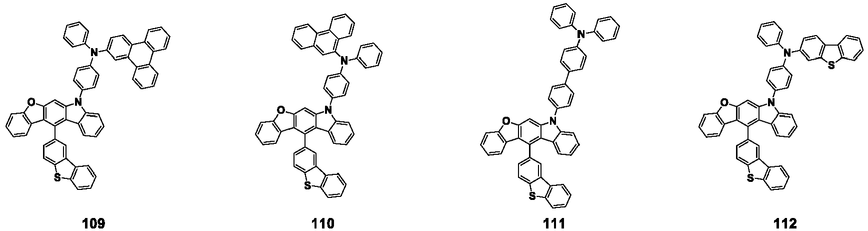

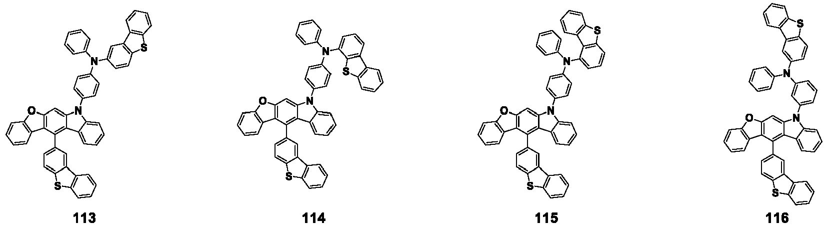

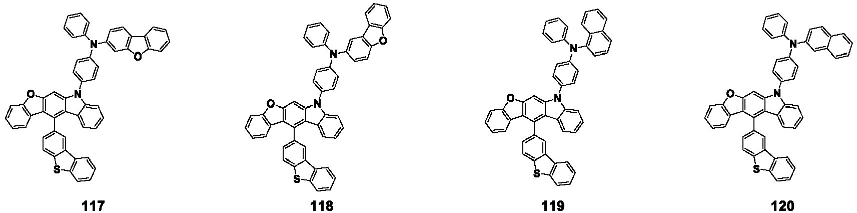

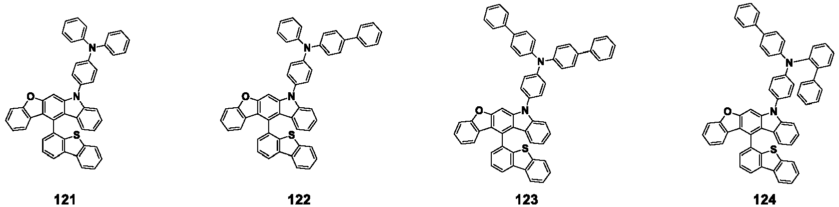

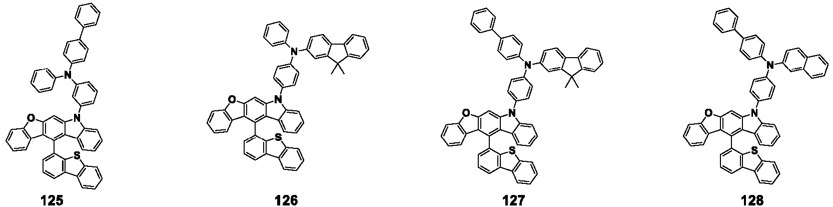

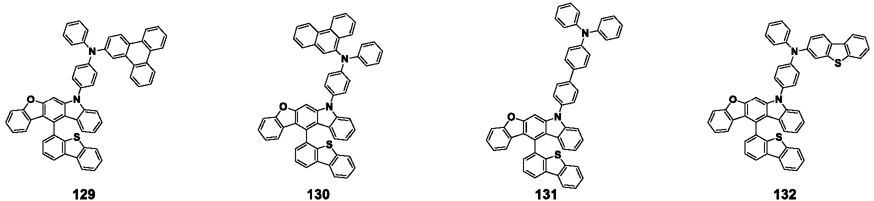

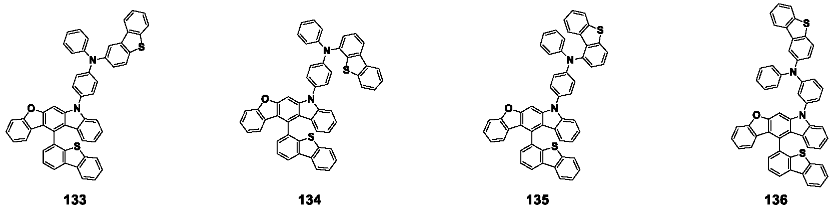

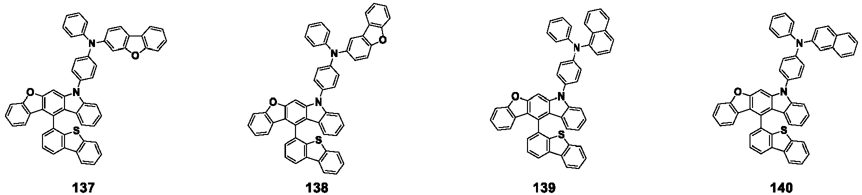

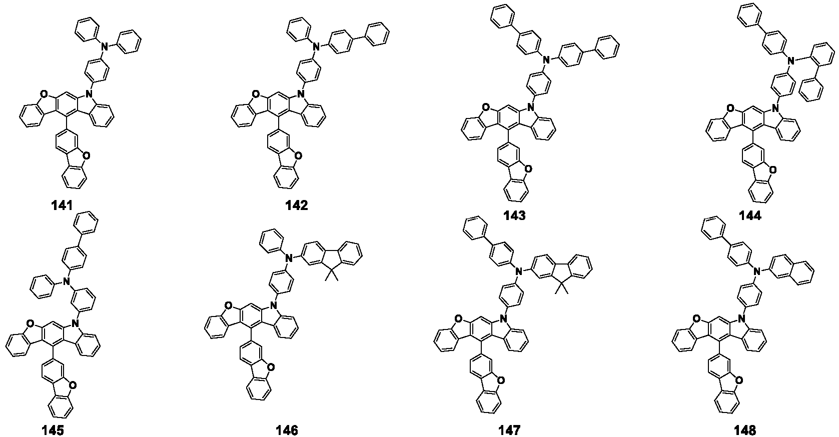

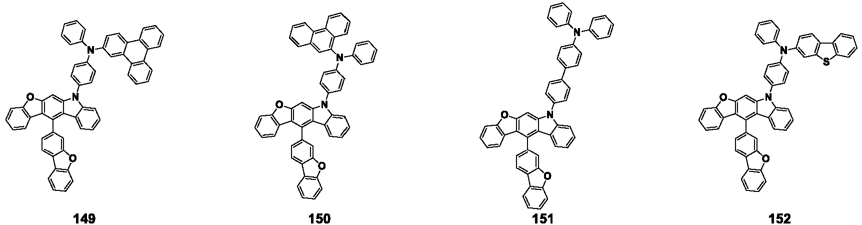

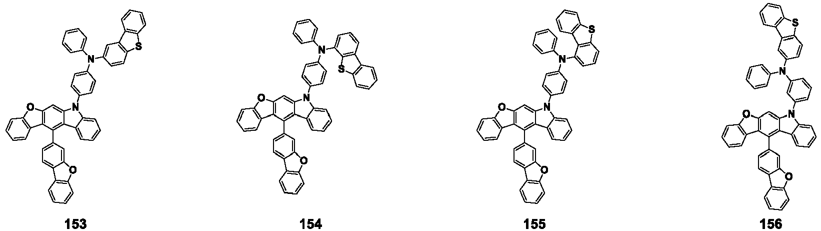

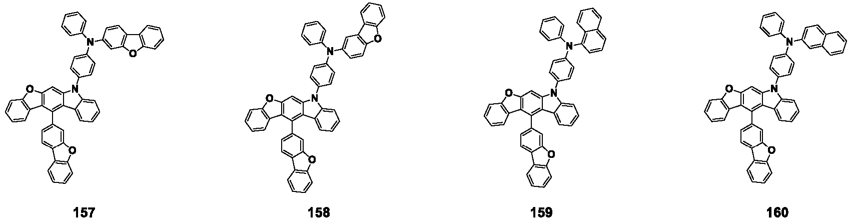

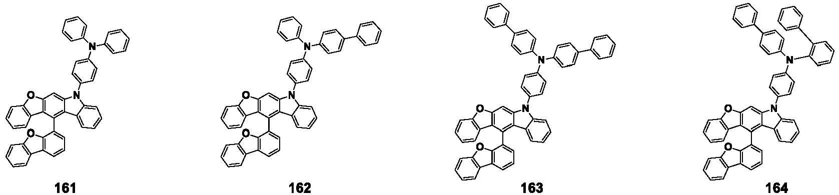

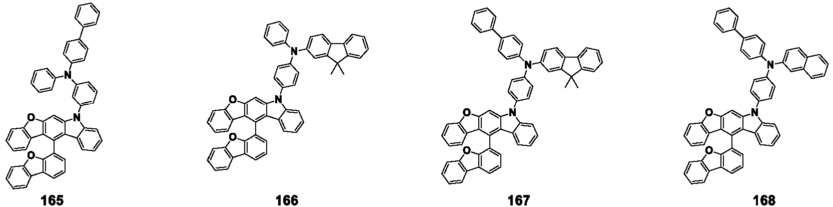

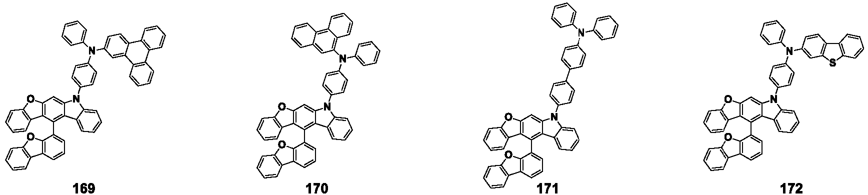

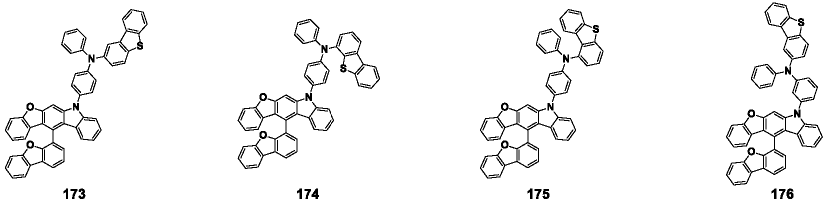

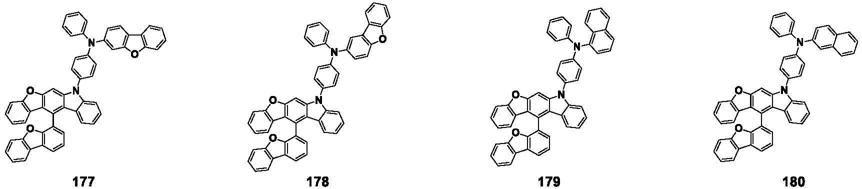

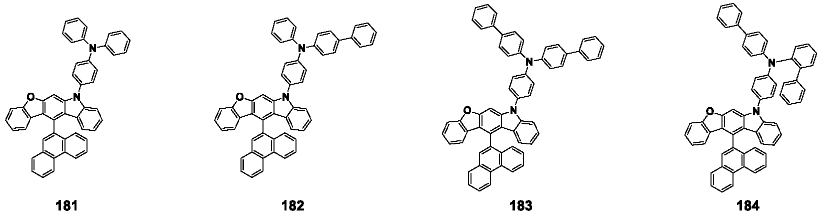

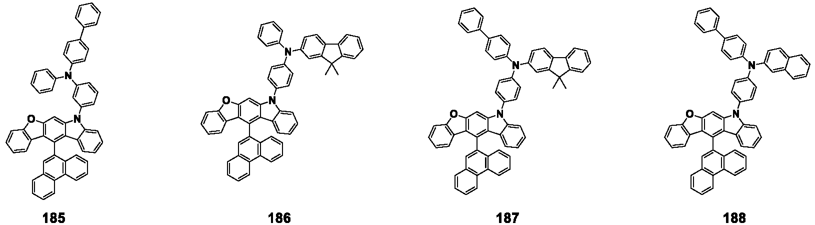

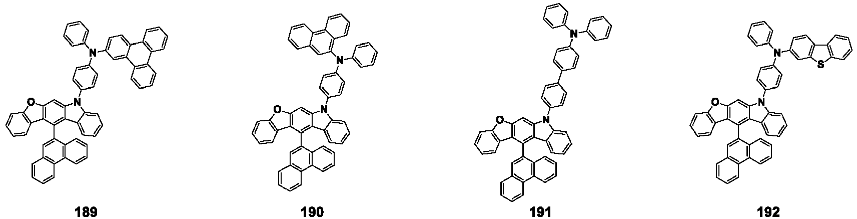

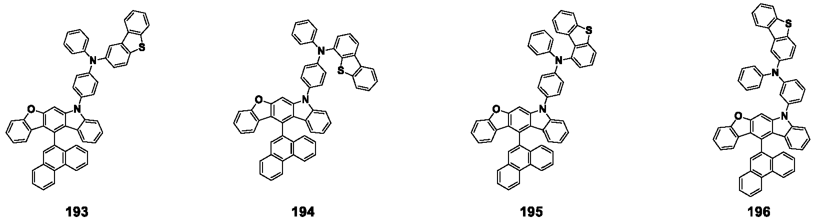

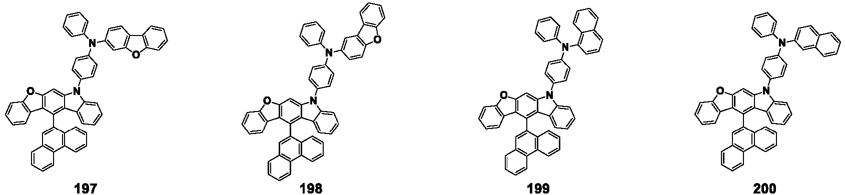

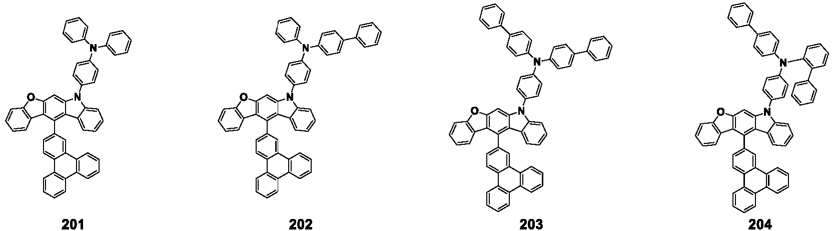

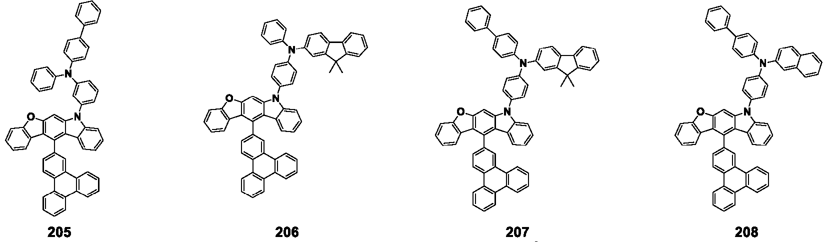

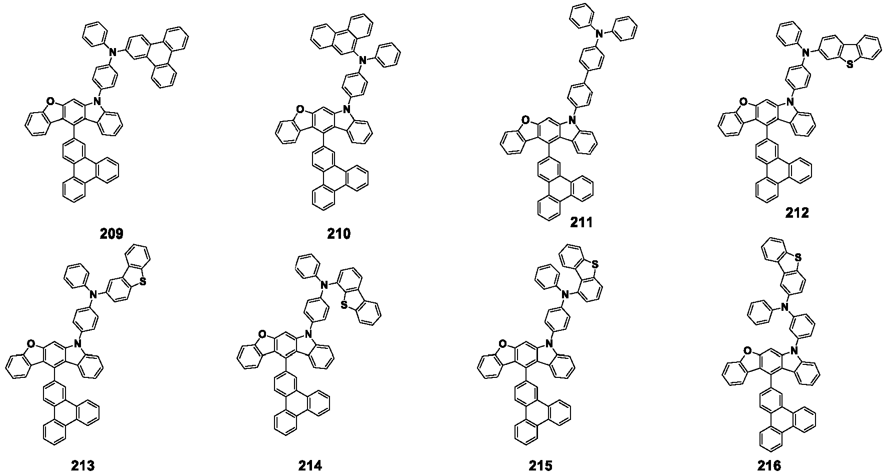

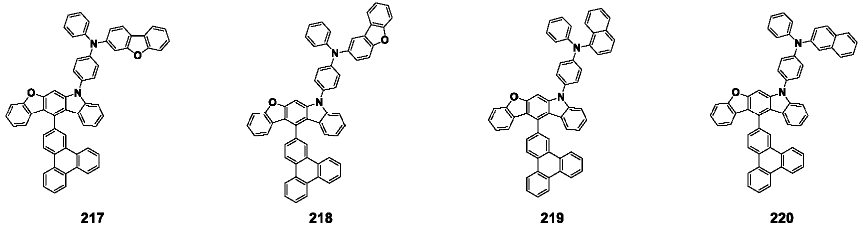

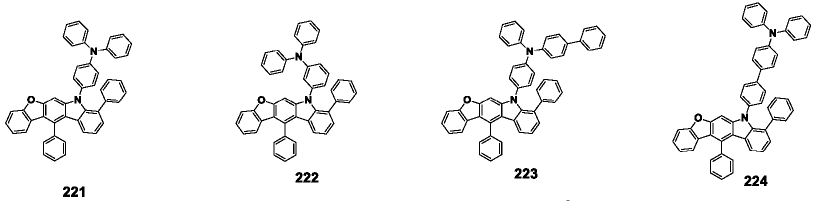

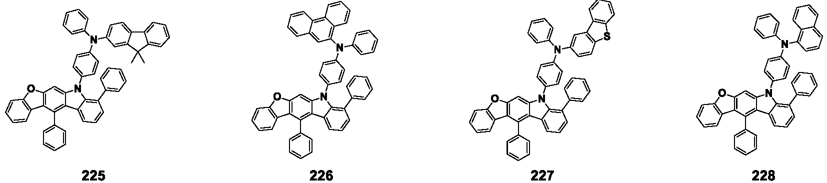

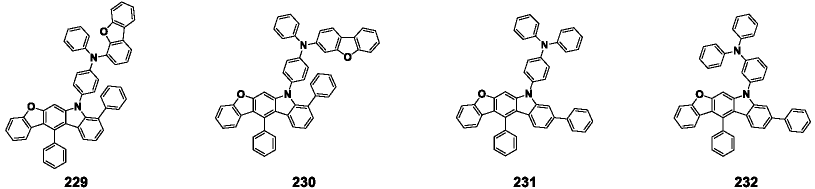

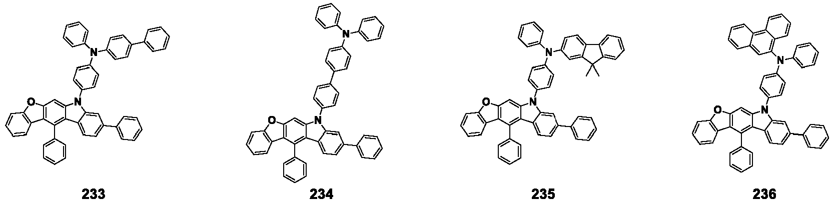

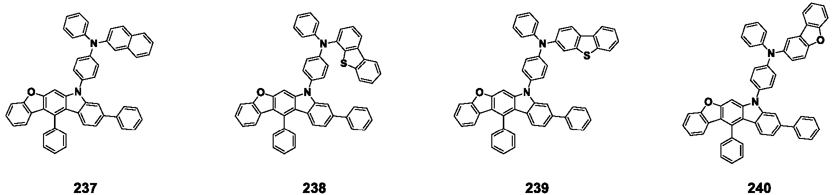

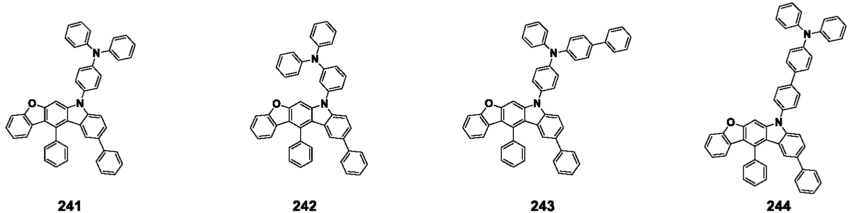

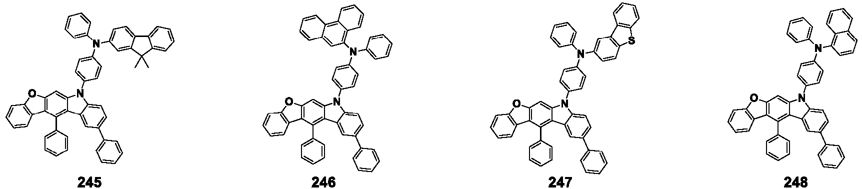

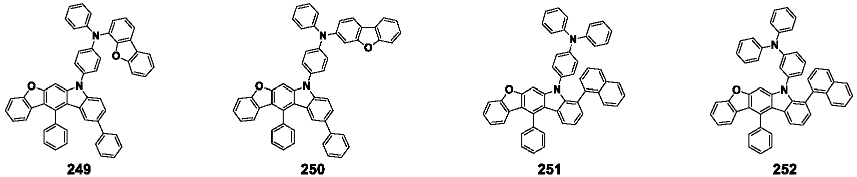

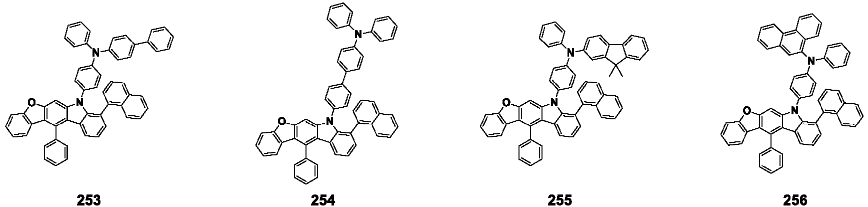

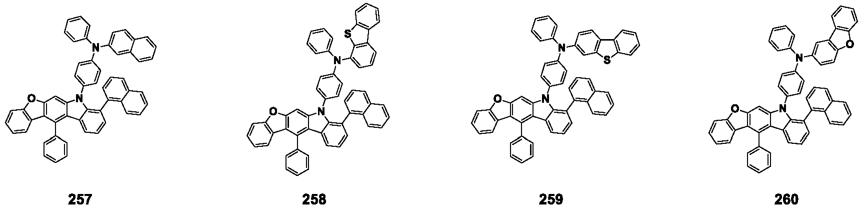

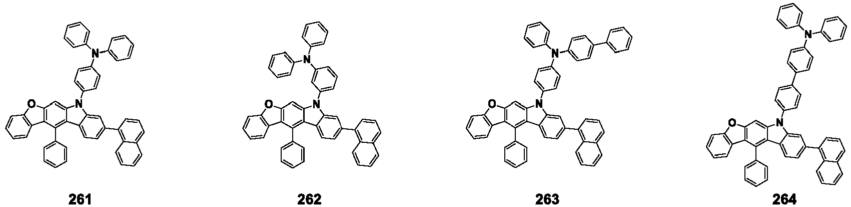

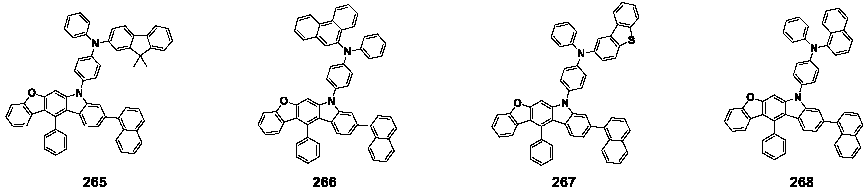

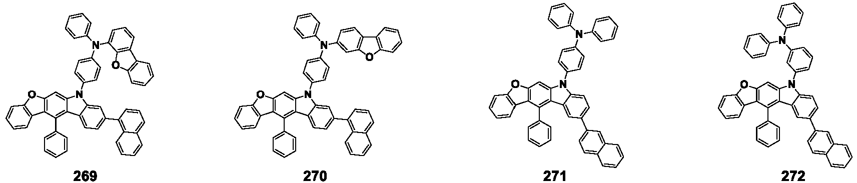

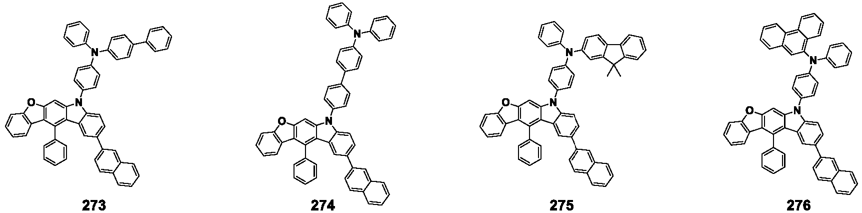

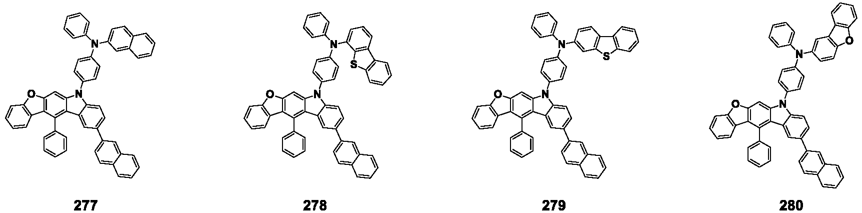

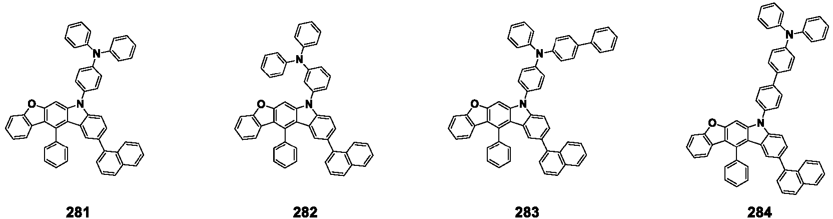

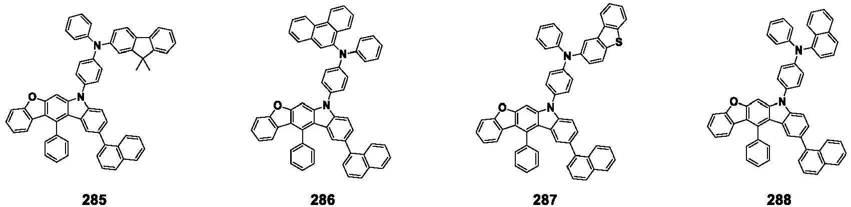

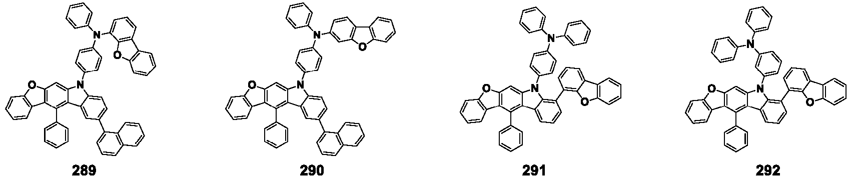

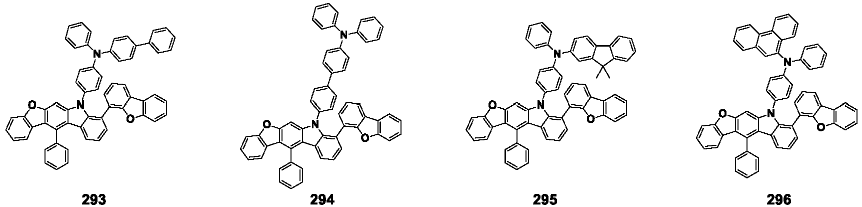

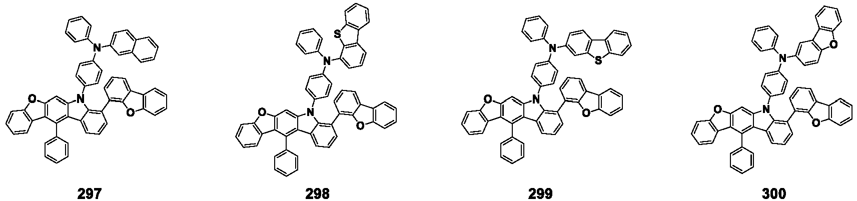

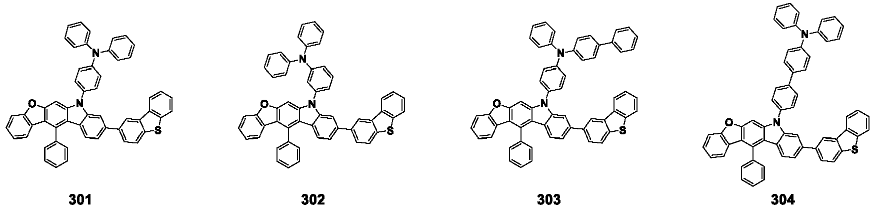

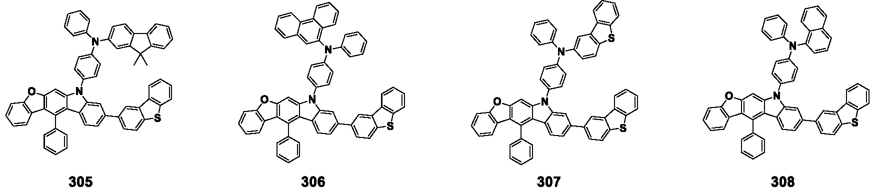

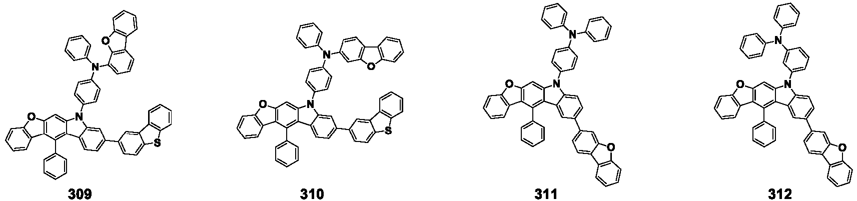

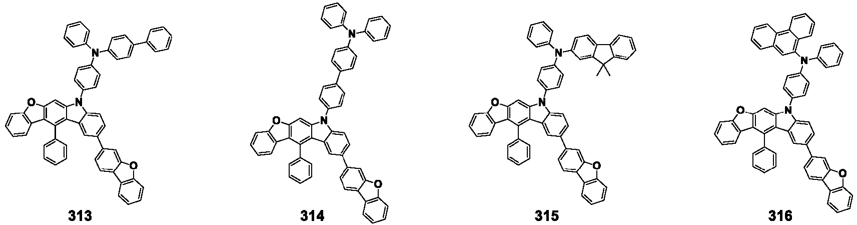

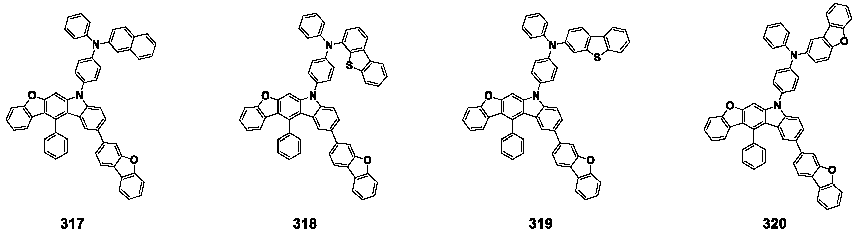

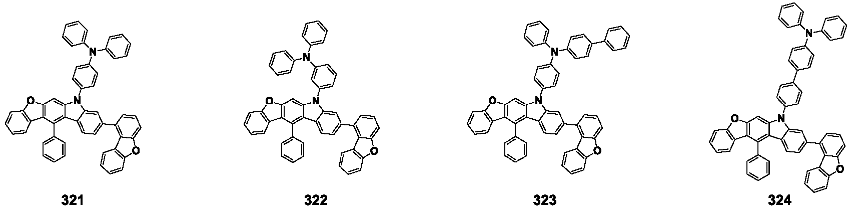

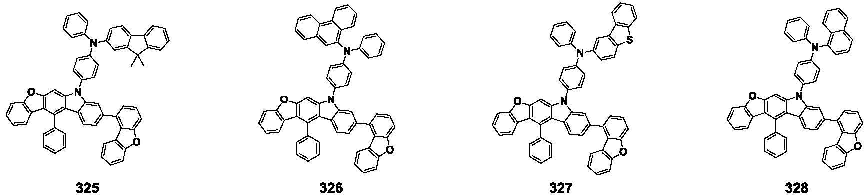

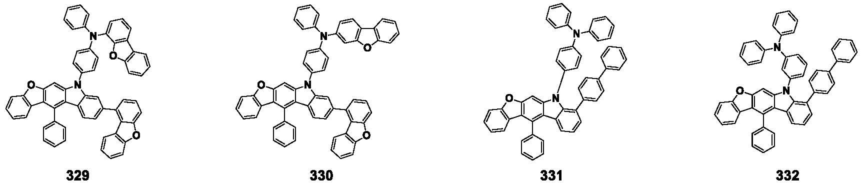

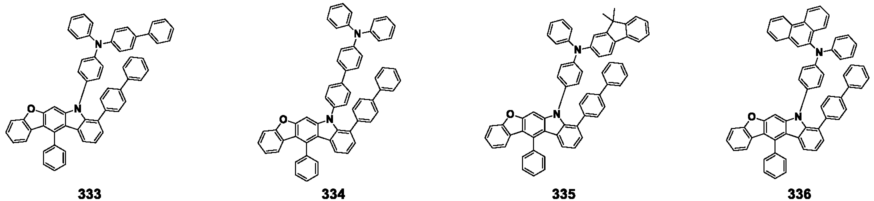

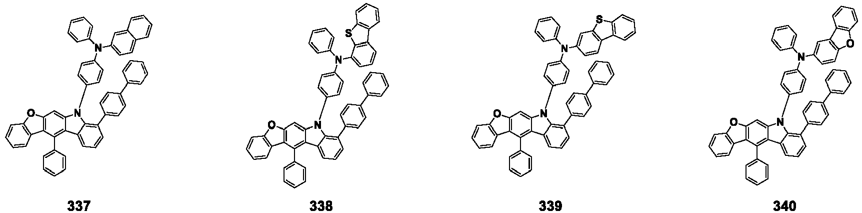

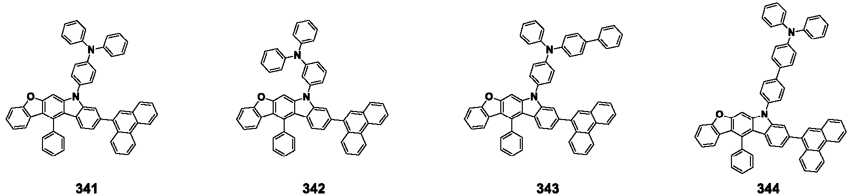

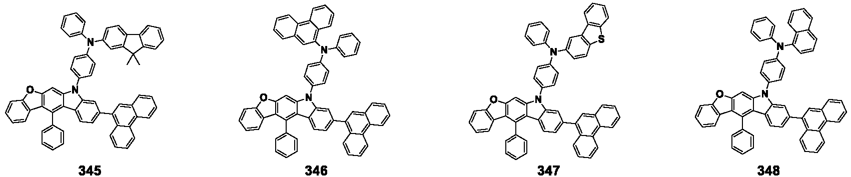

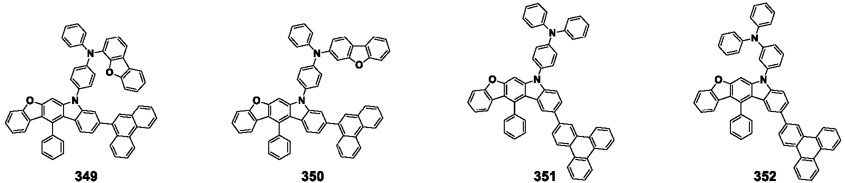

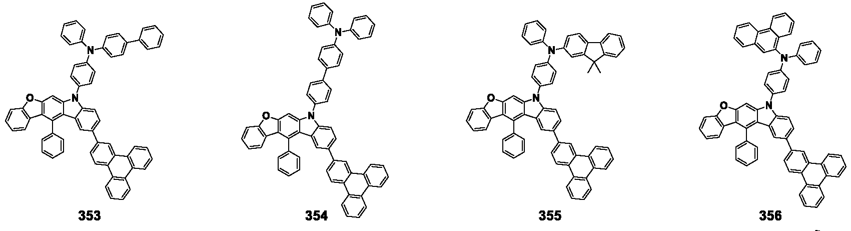

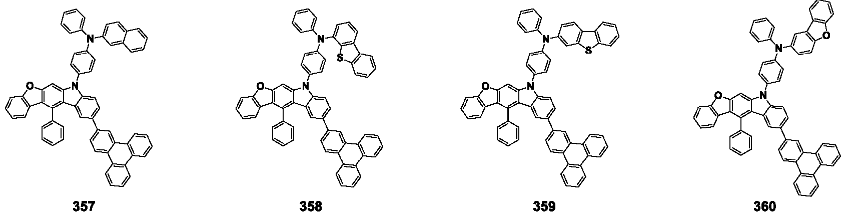

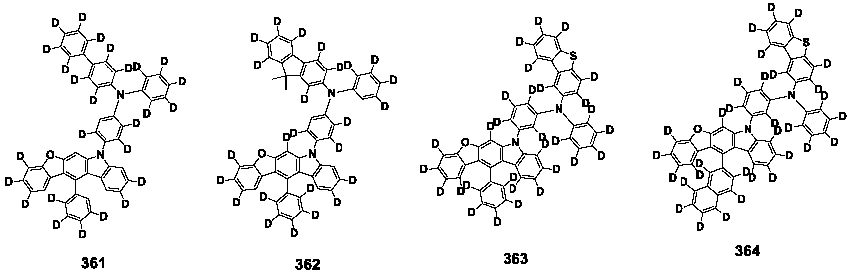

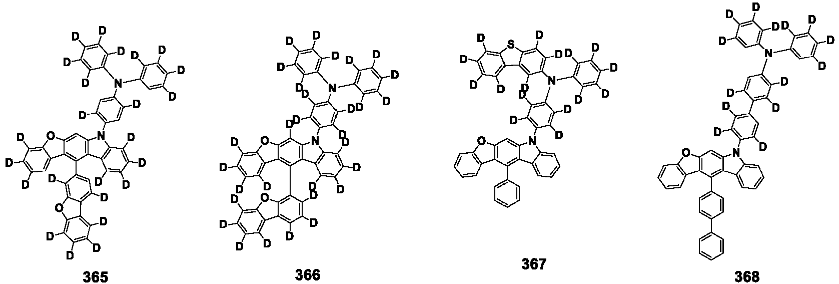

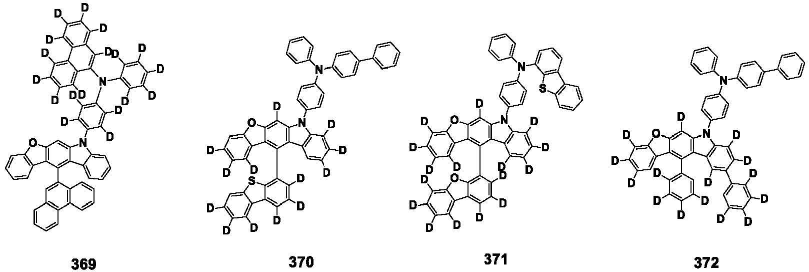

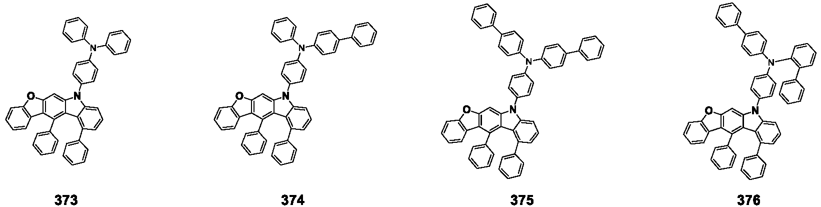

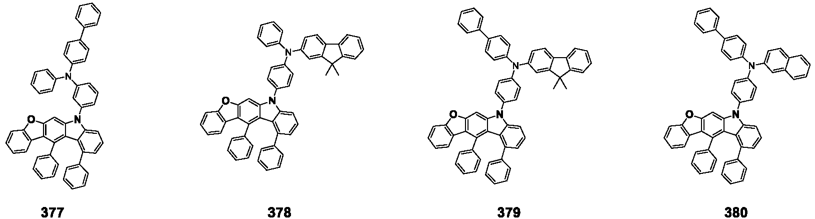

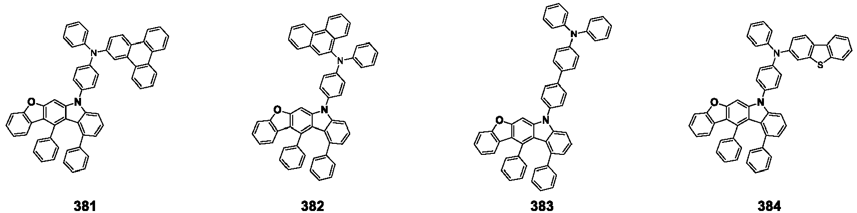

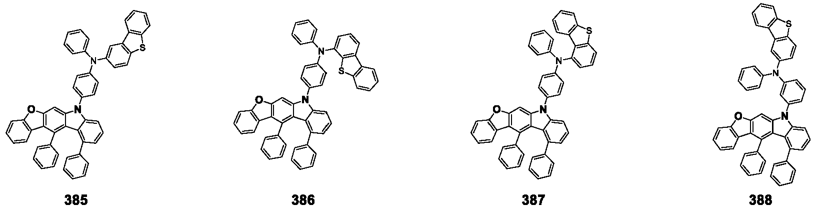

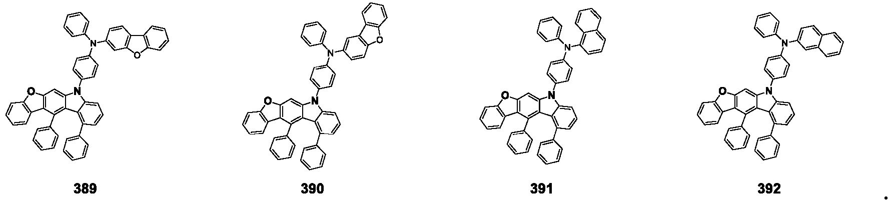

7. An organic light emitting device comprising:
a first electrode;
a second electrode provided opposite to the first electrode; and
one or more organic material layers provided between the first electrode and the second electrode,
wherein one or more layers of the one or more organic material layers comprise the heterocyclic compound of any one of Claims 1 to 6.

8. The organic light emitting device of Claim 7, wherein the one or more organic material layer comprise a hole transport layer, and the hole transport layer includes the heterocyclic compound.

9. The organic light emitting device of Claim 7, wherein the one or more organic material layer comprise an electron blocking layer, and the electron blocking layer includes the heterocyclic compound.

10. The organic light emitting device of Claim 7, wherein the one or more organic material layer comprise a light emitting layer, and the light emitting layer comprise the heterocyclic compound.

11. The organic light emitting device of Claim 7, wherein the one or more organic material layer comprise a light emitting layer, the light emitting layer comprises a host material, and the host material includes the heterocyclic compound.

12. The organic light emitting device of Claim 7, further comprising one, two or more layers selected from the group consisting of a light emitting layer, a light emitting auxiliary layer, a hole injection layer, a hole transport layer, an electron injection layer, an electron transport layer, an electron blocking layer and a hole blocking layer.
